# EUROPEAN PATENT APPLICATION

(11) **EP 1 825 802 A1**
(43) Date of publication of application: **29.08.2007**
(21) Application number: 05814604.4
(22) Date of filing: 08.12.2005
(51) Int. Cl.: A61B 1/00, A61B 19/00

(54) **GAS SUPPLYING APPARATUS, CONTROL METHOD OF THE SAME, GAS SUPPLYING SYSTEM, AND ENDOSCOPE SYSTEM**

(30) Priority: 15.12.2004 JP 2004363368; 13.04.2005 JP 2005115962; 13.04.2005 JP 2005115963
(71) Applicant: OLYMPUS MEDICAL SYSTEMS CORP., Shibuya-ku Tokyo 151-0072 (JP); Olympus Corporation, Tokyo 151-0072 (JP)
(72) Inventor: SANO, Daisuke, c/o Olympus Medical System Corp., Shibuya-ku Tokyo 151-0072 (JP); UMEMURA, Masashi, c/o Olympus Medical System Corp., Shibuya-ku Tokyo 151-0072 (JP); NODA, Kenji, c/o Olympus Medical System Corp., Shibuya-ku Tokyo 151-0072 (JP); KASAHI, Atsuhiko, c/o Olympus Medical System Corp., Shibuya-ku Tokyo 151-0072 (JP); UESUGI, Takefumi, c/o Olympus Medical System Corp., Shibuya-ku Tokyo 151-0072 (JP)
(74) Representative: von Hellfeld, Axel
(86) International application number: PCT/JP2005/022563
(87) International publication number: WO 2006/064713

(57) **Abstract**

A gas supplying apparatus according to the present invention includes a switching unit, which is connected to a gas supplying channel of an endoscope, configured to supply gas to the body cavity of a patient via the gas supplying channel, and switches to a state of supplying gas to the gas supplying channel or a state of stopping supply of gas, a time measuring unit configured to measure gas supply time, and a control unit, which is electrically connected to the time measuring unit, configured to control the switching unit, wherein the control unit controls the switching unit to make the gas supply to the gas supplying channel, and then controls the switching unit to switch from a state of supplying the gas to the gas supplying channel to a state of stopping supply of the gas when gas supply time by the time measuring unit is inputted, and the gas supply time measured by the time measuring unit reaches predetermined setting time set beforehand.

## Description

### Technical Field

The present invention relates to a gas supplying apparatus configured to supply gas for performing observation to the inside of an abdominal cavity or lumen via a gas supplying channel of an endoscope, a control method of the gas supplying apparatus, a gas supplying system including the gas supplying apparatus, and an endoscope system including the gas supplying system.

### Background Art

In recent years, technique has been performed wherein an insertion portion of an endoscope is inserted into a lumen such as large intestine or the like, thereby treating a portion to be treated, for example. With the technique, the insertion portion of an endoscope is inserted into a lumen such as the large intestine or the like of a patient. Subsequently, gas is injected into a lumen via a gas supplying channel of the endoscope for the sake of securing an endoscope visual field, and for the sake of securing an area for operating a treatment instrument.

Gas is injected into a lumen, thereby making the lumen an expanded state. Thus, a surgeon can perform treatment or the like while observing a treated portion, and confirming a treatment instrument inserted via a treatment instrument channel of the endoscope with the endoscope inserted into the lumen.

Note that as for the above-mentioned gas, for example, CO2 gas (hereafter, described as carbon dioxide gas) which can be readily absorbed by a living body is employed instead of air which has been employed heretofore.

When performing such a technique, a flexible endoscope including a flexible insertion portion to be inserted into a lumen such as large intestine or the like (hereafter, referred to as flexible endoscope) is used. The flexible endoscope is connected with a light source device and a camera control unit. Also, a gas supplying/water supplying channel is provided within the insertion portion, operating portion, and universal cord of the flexible endoscope. Carbon dioxide gas which is supplied from the gas supplying apparatus, and carbon dioxide gas tank to the inside of the lumen as observation gas is supplied to the gas supplying/water supplying channel. An endoscope system is made up of the flexible endoscope, light source device, camera control unit, gas supplying apparatus, and carbon dioxide gas tank.

With an existing endoscope system, a high-pressure gas tube extending from a carbon dioxide gas tank is communicatively connected to the input side of a gas supplying apparatus, and one end side of a gas supplying tube for lumen wherein the proximal side is connected to the light source device is communicatively connected to the output side of the gas supplying apparatus.

The surgeon sets the insertion portion of the endoscope communicatively connected to the light source device into a state of being inserted into the large intestine from the anus of the patient for example, and turns the gas supplying apparatus into an operation state. Thus, the carbon dioxide gas within the carbon dioxide gas tank is supplied to the inside of the lumen via a gas supplying base provided in the endoscope connector connected to the light source device, the inside of the universal cord, the operation portion of the endoscope, and a gas supplying/water supplying channel provided in the insertion portion.

As for such an existing endoscope system, a great number of systems have been proposed. For example, with Japanese Unexamined Patent Application Publication No. 2000-139827, an endoscope gas supplying apparatus has been disclosed wherein a gas supplying tube communicatively connected to the gas supplying output side of the above-mentioned gas supplying apparatus is connected to a forceps hole of the endoscope, whereby gas is supplied into a lumen or body cavity via a forceps channel (treatment instrument channel) communicatively connected to the forceps hole of the endoscope.

However, the existing endoscope system employs a configuration wherein carbon dioxide gas is supplied to the inside of the lumen via the gas supplying base of the endoscope connected to the light source device, and to the gas supplying/water supplying channel. Therefore, the carbon dioxide gas from the gas supplying apparatus is continuously supplied to the light source device connected with the endoscope instead of air supplied from a gas supplying/water supplying pump to the gas supplying channel via the gas supplying base.

Subsequently, when the surgeon performs an operation for closing a hole portion provided in a gas supplying/water supplying button, the carbon dioxide gas is supplied to the inside of the lumen. In other words, in a state in which the surgeon has not closed the hole portion of the gas supplying/water supplying button, while the carbon dioxide gas is supplied to the inside of the gas supplying channel from the gas supplying apparatus, the carbon dioxide gas is continuously discharged into the atmosphere. That is to say, the carbon dioxide gas within the carbon dioxide gas tank is continuously consumed even after completion of observation or operation within the lumen, resulting in the problems of an uneconomical system.

Also, in addition to the above-mentioned technique for treating the inside of the lumen using a flexible endoscope, there is laparoscope surgery (hereafter, also described as surgery) for performing curative treatment without performing abdominal surgery for the sake of reducing invasiveness as to a patient. With this surgery, a first trocar for guiding an observation endoscope into the body cavity, and a second trocar for guiding a treatment instrument to a treated portion are inserted by puncturing the abdominal portion of a patient.

Pneumoperitoneum gas is supplied into the abdominal cavity for securing an endoscope visual field, and for securing an area for operating a treatment instrument. Thus, the abdominal cavity becomes an expanded state by the pneumoperitoneum gas. Therefore, the surgeon can perform observation of the treated portion and the treatment instrument inserted via the second trocar, treatment, and so forth using the endoscope inserted into the abdominal cavity via the first trocar. Note that the above-mentioned carbon dioxide gas which is readily absorbed by a living body for example is also employed as pneumoperitoneum gas.

With an pneumoperitoneum apparatus, a state in which carbon dioxide gas flows through a gas supplying channel, and a state in which the flow of carbon dioxide gas through the gas supplying channel is shielded are repeated. Specifically, a control unit detects the pressure within the abdominal cavity by a pressure sensor, and also monitors the difference between the abdominal cavity setting pressure of a patient set beforehand and the actual abdominal cavity pressure, and regulates the flow rate of carbon dioxide gas depending on the pressure difference.

For example, with Japanese Unexamined Patent Application Publication No. 2000-139827, a gas supplying apparatus for endoscope has been disclosed wherein a state of a patient is inspected by supplying air within a body cavity such as stomach or the like. The end portion of a connection tube extending, which is connected to a connection opening of the gas supplying apparatus for endoscope, is connected to a forceps entrance communicatively connected to a treatment-instrument channel. The gas supplying apparatus for endoscope is connected with a foot switch capable of remote operations.

Therefore, air is discharged from the connection opening by the surgeon operating the foot switch or a switch for discharge provided in the gas supplying apparatus for endoscope as appropriate. The air is fed into the body cavity via the connection tube, forceps entrance, and treatment-instrument channel.

In recent years, as a new effort, in addition to an endoscope to be inserted into an abdominal cavity via the first trocar, a technique has been performed wherein a treated portion is treated by inserting an insertion portion of the endoscope into a lumen such as large intestine or the like for example. With this technique, treatment can be performed by determining a treated portion with the endoscope at the abdominal cavity side and the endoscope at the lumen side.

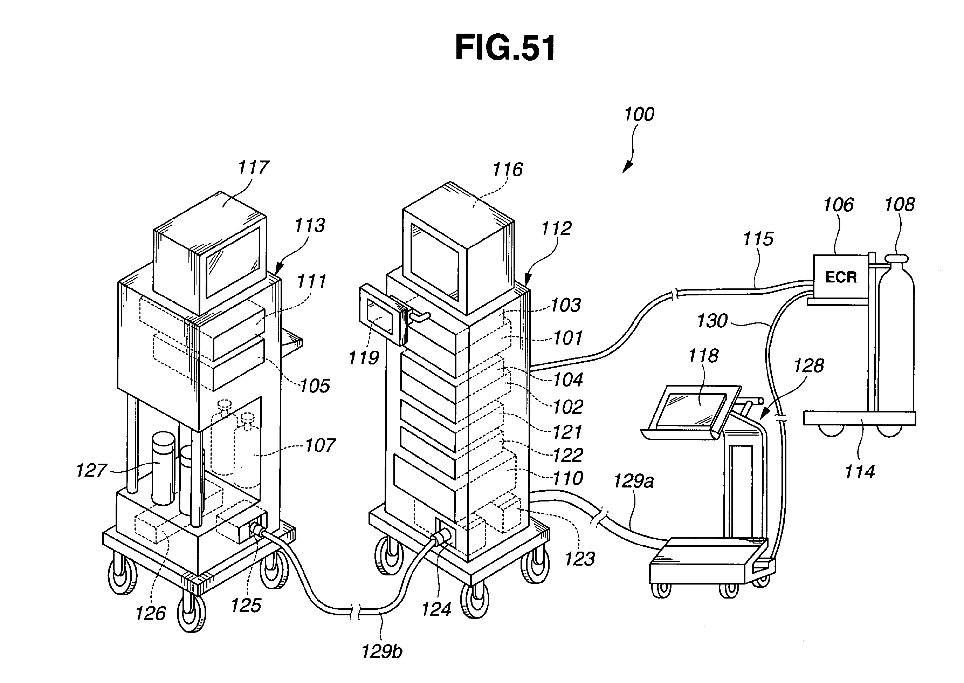

When performing the technique, for example, a laparoscope surgery system 7000 shown in Fig. 51 is configured. Description will be made below regarding the laparoscope surgery system 7000. In the drawing, a first light source device 101 and a first camera control unit 103 are connected with an endoscope (not shown) to be inserted to the abdominal cavity side via a trocar. Also, a second light source device 102 and a second camera control unit 104 are connected with an endoscope (not shown) having an insertion portion to be inserted into a lumen.

Also, a first carbon dioxide gas tank 107 is connected to an pneumoperitoneum apparatus 105. The pneumoperitoneum apparatus 105 supplies carbon dioxide gas into an abdominal cavity via a trocar. An endoscope carbon dioxide regulator (hereafter, abbreviated as ECR) 106 configured to supply carbon dioxide gas into the lumen via a gas supplying/water supplying channel provided in the insertion portion of the endoscope is connected to a second carbon dioxide gas tank 108.

Each of devices 101, 102, 103, 104, 105, and 106 is electrically connected with, for example, a treatment apparatus such as a cauterization device (also called electric scalpel) 111 or the like in addition to a system controller 110 configured to perform operational control. Such a laparoscope surgery system 7000 is configured, whereby carbon dioxide gas can be supplied into the abdominal cavity by the pneumoperitoneum apparatus 105, and also carbon dioxide gas is supplied to the lumen by the ECR 106 to perform treatment. Note that the respective devices are disposed on a first cart 112, a second cart 113, an ECR cart 114, and so forth. A lumen tube 115 extending from the ECR 106 is connected to the second light source device 102. The carbon dioxide gas supplied from the ECR 106 is supplied into the lumen from the second light source device 102 via a gas supplying base, and a gas supplying/water supplying channel provided in a light source connector (not shown) of an endoscope (not shown).

Also, the laparoscope surgery system 7000 includes an observation monitor 117 on which an endoscope image or the like is displayed, a central operating panel 118, a central display panel 119, image recording apparatuses 121 and 122, a distributor 123, a communication connector 124, a communication connector 125, a distributor 126, a suction bottle 127, a peripheral apparatus controller 128, communication cables 129a and 129b, and a connection cable 130.

As for such laparoscope surgery, a paper entitled "Anaesthesiological Problem of Thoracoscope and Laparoscope" described on pp 36-43 of a text of the 46th convention refresher course by Japan Society of Anesthesiology describes that, in the case of extremely increasing abdominal cavity pressure, hemodynamics may be affected or gas embolism may be caused. Therefore, in addition to checking of parameters such as blood pressure, an electrocardiogram, a pulse oximeter (hereafter, also referred to as vital signs), and so forth, it is necessary to set the upper limit value of pneumoperitoneum pressure correctly, and observe abdominal internal pressure.

Also, a paper entitled "Anesthesia of Child Operation under Laparoscopy" of Child Surgery VOL. 26, No. 8, and 1994-8, describes that, end-expiratory carbon dioxide gas concentration is monitored, and the number of times of ventilation (breathing) is increased so as to prevent the concentration from increasing, as preventative treatment for hypercapnia during pneumoperitoneum. Also, in the event that abdominal internal pressure is high, there is a case wherein even if the number of times of ventilation is increased, the end-expiratory carbon dioxide gas concentration cannot be prevented from increase, and in this case, an operator (surgeon) is asked for cooperation, pneumoperitoneum is interrupted temporarily, and improvement of a patient's condition is awaited.

Further, with regard to the detection and treatment of carbon dioxide gas embolism at the time of selecting carbon dioxide gas as pneumoperitoneum gas, description has been made wherein pneumoperitoneum gas may invade a blood vessel from a catheter, and if a lot of carbon dioxide gas enters a blood vessel, a carbon dioxide gas embolism will occur. In this case, end-expiratory carbon dioxide gas partial pressure falls quickly. If carbon dioxide gas embolism is caused, an pneumoperitoneum apparatus is to be immediately stopped, and as much carbon dioxide gas as possible is to be discharged from an pneumoperitoneum circuit.

Heretofore, monitoring of an apparatus (such as an anesthesia apparatus, a respirator, a patient monitoring apparatus, or the like) managed by an anesthetist has been performed by the anesthetist. In the event of any abnormal display value regarding living body information, the abnormality has been informed to the surgeon as necessary based on the determination by the anesthetist, and various types of corresponding treatment as to a patient has been performed. On the other hand, display of an operation apparatus (such as an pneumoperitoneum apparatus, an electric scalpel, or the like) managed by a surgeon has been monitored by the surgeon or a nurse, the information thereof has been informed to an anesthetist, and various types of corresponding treatment as to a patient has been performed.

Thus, in the event that the management and monitoring of various types of devices are shared by a plurality of doctors, it has been necessary to take care that there is no oversight of abnormalities from the display of each device, or it has been necessary to perform communication smoothly between an anesthetist and a surgeon, and take care that there is no delay as to various types of corresponding treatment.

Consequently, for example, with Japanese Unexamined Patent Application Publication No. 2001-170008, a surgery system has been proposed wherein in the event that there is abnormality in the parameters for informing the living body information of a patient, such as end-expiratory carbon dioxide gas partial pressure and the like, a surgeon can readily confirm the living body information of the patient using a patient monitoring apparatus.

However, with the surgery system shown in Fig. 51, an arrangement has been made wherein gas is supplied into the body cavity of a patient, and also a rigid endoscope is inserted into the abdominal cavity, and a flexible endoscope is inserted into the lumen, thereby determining a treated portion to perform treatment. However, with the surgery system, countermeasures in the event that there is abnormality in the parameters informing the living body information of a patient is not taken into consideration.

The present invention provides a gas supplying apparatus, a control method of a gas supplying apparatus, a gas supplying system, and an endoscope system, with further improvements in performance, in light of the above-mentioned situations.

### Disclosure of Invention

### Means for Solving the Problem

A gas supplying apparatus according to the present invention, which is connected to a gas supplying channel of an endoscope, configured to supply gas to the body cavity of a patient via the gas supplying channel, comprises: a switching unit configured to switch the gas supplying apparatus to a state of supplying the gas to the gas supplying channel, or a state of stopping supply of gas; a time measuring unit configured to measure the gas supply time of the gas; and a control unit, which is electrically connected to the time measuring unit, configured to control the switching unit; wherein the control unit controls the switching unit to supply the gas to the gas supplying channel, and then controls the switching unit so as to switch from a state of supplying the gas to the gas supplying channel to a state of stopping supply of gas in the event that the gas supply time by the time measuring unit is inputted, and the gas supply time measured at the time measuring unit reaches the setting time set beforehand.

A control method of a gas supplying apparatus according to the present invention comprises: a gas supplying step of supplying gas via a gas supplying channel of an endoscope; a measuring step of measuring a flow rate of the gas supplied in the gas supplying channel; a detecting step of detecting whether or not a gas supply operation by the endoscope is being performed based on the measurement result in the measuring step; and a control step of adjusting the flow rate of supply of the gas by controlling the flow rate of supply of the gas so as to be decreased in the gas supplying step in the event that a gas supply operation by the endoscope is not being performed is detected in the detecting step.

A gas supplying system according to the present invention, which is connected to a gas supplying channel of an endoscope, configured to supply gas to the abdominal cavity and lumen of a patient via the gas supplying channel, comprises: a gas supplying unit configured to supply predetermined gas to an abdominal cavity and lumen; a pressure regulating unit configured to regulate the internal pressure of each of the abdominal cavity and the lumen; and a control unit electrically connected to an external apparatus configured to output living body information; wherein the control unit regulates the internal pressure of each of the abdominal cavity and the lumen based on variations of the living body information inputted from the external apparatus.

An endoscope system according to the present invention comprises: an endoscope having a gas supplying channel capable of supplying gas to the body cavity of a patient; and a gas supplying apparatus including a switching unit configured to switch the gas supplying apparatus to a state of supplying the gas to the gas supplying channel, or a state of stopping supply of gas, a time measuring unit configured to measure the gas supply time of the gas, and a control unit, which is electrically connected to the time measuring unit, configured to control the switching unit; wherein the control unit controls the switching unit to supply the gas to the gas supplying channel, and then controls the switching unit so as to switch from a state of supplying the gas to the gas supplying channel to a state of stopping supply of gas in the event that the gas supply time by the time measuring unit is inputted, and the gas supply time measured at the time measuring unit reaches the setting time set beforehand.

### Brief Description of the Drawings

Fig. 1 is a diagram illustrating a configuration example of an endoscope system having a gas supplying apparatus according to a first embodiment of the present invention.
Fig. 2 is a front view of the gas supplying apparatus according to the first embodiment of the present invention.
Fig. 3 is a rear view of the gas supplying apparatus according to the first embodiment of the present invention.
Fig. 4 is a block diagram describing a configuration example of the inside of the gas supplying apparatus according to the first embodiment of the present invention.
Fig. 5 is a timing chart describing an operation of the gas supplying apparatus according to the first embodiment of the present invention.
Fig. 6 is a flowchart illustrating a control example of the gas supplying apparatus according to the first embodiment of the present invention.
Fig. 7 is a cross-sectional view describing a leaked state in which carbon dioxide gas is ejected from a hole portion provided in a gas supplying/water supplying button according to the first embodiment of the present invention.
Fig. 8 is a cross-sectional view describing a state in which the hole portion provided in the gas supplying/water supplying button is closed, and carbon dioxide gas is supplied to an insertion portion side, according to the first embodiment of the present invention.
Fig. 9 is a block diagram describing a configuration example of a gas supplying apparatus according to a second embodiment of the present invention.
Fig. 10 is a timing chart describing an operation of the gas supplying apparatus according to the second embodiment of the present invention.
Fig. 11 is a flowchart illustrating a control example of the gas supplying apparatus according to the second embodiment of the present invention.
Fig. 12 is a rear view of a gas supplying apparatus of a first modification according to the second embodiment of the present invention.
Fig. 13 is a block diagram describing a configuration example of the inside of the gas supplying apparatus of the first modification according to the second embodiment of the present invention.
Fig. 14 is a configuration diagram illustrating a configuration example of an endoscope system having the gas supplying apparatus according to the second embodiment of the present invention.
Fig. 15 is a rear view of a gas supplying apparatus of a second modification according to the second embodiment of the present invention.
Fig. 16 is a block diagram describing configuration examples of the inside of the gas supplying apparatus and a light source device of the second modification according to the second embodiment of the present invention.
Fig. 17 is a block diagram describing a configuration example of a gas supplying apparatus according to a third embodiment of the present invention.
Fig. 18 is a timing chart describing an operation of the gas supplying apparatus according to the third embodiment of the present invention.
Fig. 19 is a flowchart illustrating a control example of the gas supplying apparatus according to the third embodiment of the present invention.
Fig. 20 is a block diagram describing a configuration example of a gas supplying apparatus according to a fourth embodiment of the present invention.
Fig. 21 is a flowchart illustrating a control example of the gas supplying apparatus according to the fourth embodiment of the present invention.
Fig. 22 is a timing chart describing an operation of the gas supplying apparatus according to the fourth embodiment of the present invention.
Fig. 23 is a block diagram describing a configuration example of a gas supplying apparatus according to a fifth embodiment of the present invention.
Fig. 24 is a flowchart illustrating a control example of the gas supplying apparatus according to the fifth embodiment of the present invention.
Fig. 25 is a timing chart describing an operation of the gas supplying apparatus according to the fifth embodiment of the present invention.
Fig. 26 is a block diagram describing a configuration example of a gas supplying apparatus according to a sixth embodiment of the present invention.
Fig. 27 is a flowchart illustrating a control example of the gas supplying apparatus according to the sixth embodiment of the present invention.
Fig. 28 is a graph illustrating voltage-to-flow rate properties of a flow-rate throttle valve according to the sixth embodiment of the present invention.
Fig. 29 is a timing chart describing an operation of the gas supplying apparatus according to the sixth embodiment of the present invention.
Fig. 30 is a block diagram describing a configuration example of a gas supplying apparatus of a first modification according to the sixth embodiment of the present invention.
Fig. 31 is a flowchart illustrating a control example of the gas supplying apparatus of the first modification according to the sixth embodiment of the present invention.
Fig. 32 is a block diagram describing a configuration example of a gas supplying apparatus of a second modification according to the sixth embodiment of the present invention.
Fig. 33 is a cross-sectional view of an orifice provided in a gas supplying channel according to the sixth embodiment of the present invention.
Fig. 34 is a flowchart illustrating a control example of the gas supplying apparatus of the second modification according to the sixth embodiment of the present invention.
Fig. 35 is a diagram illustrating the configuration of a laparoscope surgery system wherein a monitoring apparatus and a respirator serving as an external apparatus according to a seventh embodiment of the present invention are illustrated.
Fig. 36 is a diagram describing the configuration of the laparoscope surgery system including a gas supplying system according to the seventh embodiment of the present invention.
Fig. 37 is a diagram for describing a central operating panel according to the seventh embodiment of the present invention.
Fig. 38 is a diagram for describing the central operating panel according to the seventh embodiment of the present invention.
Fig. 39 is a configuration diagram illustrating the internal configuration of a gas supplying apparatus according to the seventh embodiment of the present invention.
Fig. 40 is a diagram for describing a panel portion of the gas supplying apparatus according to the seventh embodiment of the present invention.
Fig. 41 is a flowchart illustrating a control example in the event of the gas supplying apparatus according to the seventh embodiment of the present invention supplying gas to an abdominal cavity and a lumen.
Fig. 42 is a flowchart for describing a control example of confirmation of terminal-expiratory carbon dioxide partial pressure according to the seventh embodiment of the present invention.
Fig. 43 is a flowchart for describing a control example of an operation for decreasing the setting pressure of an abdominal cavity and a lumen, according to the seventh embodiment of the present invention.
Fig. 44 is a flowchart for describing a control example of an operation for restoring the setting pressure of an abdominal cavity and a lumen to the original setting pressure, according to the seventh embodiment of the present invention.
Fig. 45 is a timing chart illustrating the relation of terminal-expiratory carbon dioxide partial pressure, abdominal cavity pressure, and lumen pressure, according to the seventh embodiment of the present invention.
Fig. 46 is a diagram describing the configuration of a laparoscope surgery system including a gas supplying system according to an eighth embodiment of the present invention.
Fig. 47 is a diagram for describing a water tank according to the eighth embodiment of the present invention.
Fig. 48 is a configuration diagram illustrating the internal configuration of a gas supplying apparatus according to the eighth embodiment of the present invention.
Fig. 49 is a flowchart for describing a control example of an operation for decreasing the setting pressure of an abdominal cavity and a lumen, according to the eighth embodiment of the present invention.
Fig. 50 is a diagram for describing an alarm screen to be displayed on the panel of a display panel according to the eighth embodiment of the present invention.
Fig. 51 is a diagram describing an existing laparoscope surgery system wherein in addition to an endoscope to be inserted into an abdominal cavity, an insertion portion of the endoscope is inserted into a lumen such as large intestine to perform a technique for treating a treated portion.

### Best Mode for Carrying Out the Invention

Embodiments of the present invention will be described below with reference to the drawings.

### (First Embodiment)

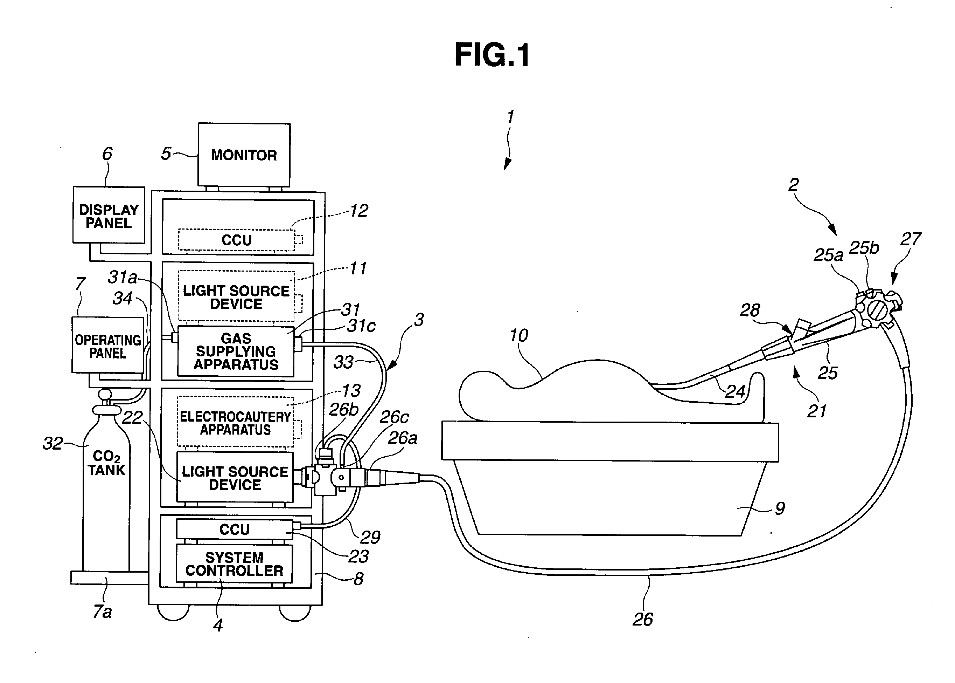

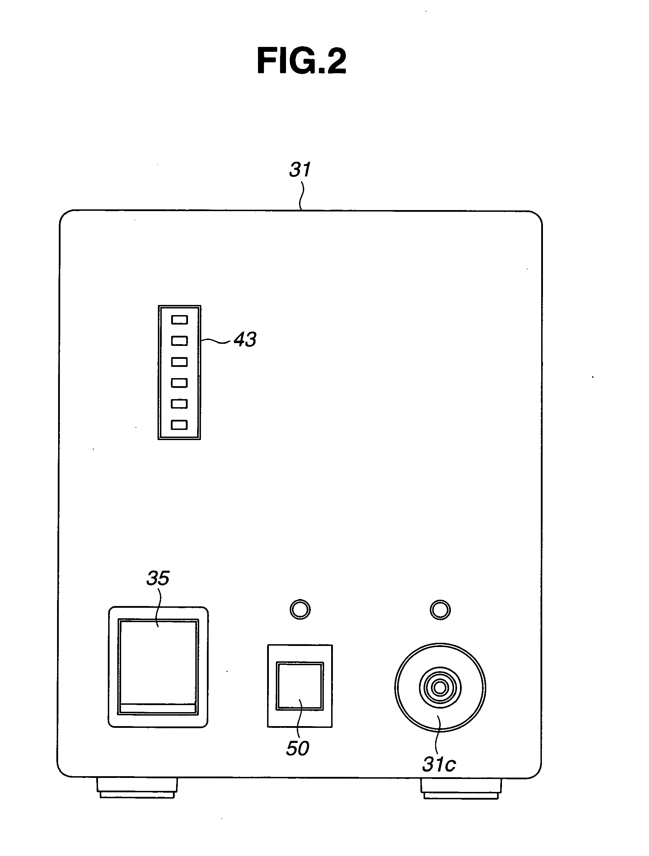

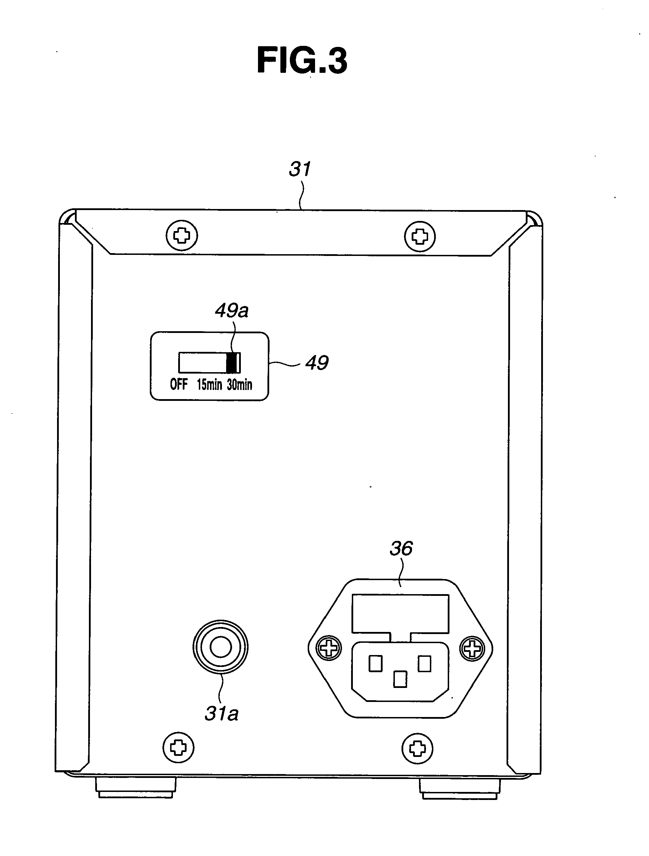

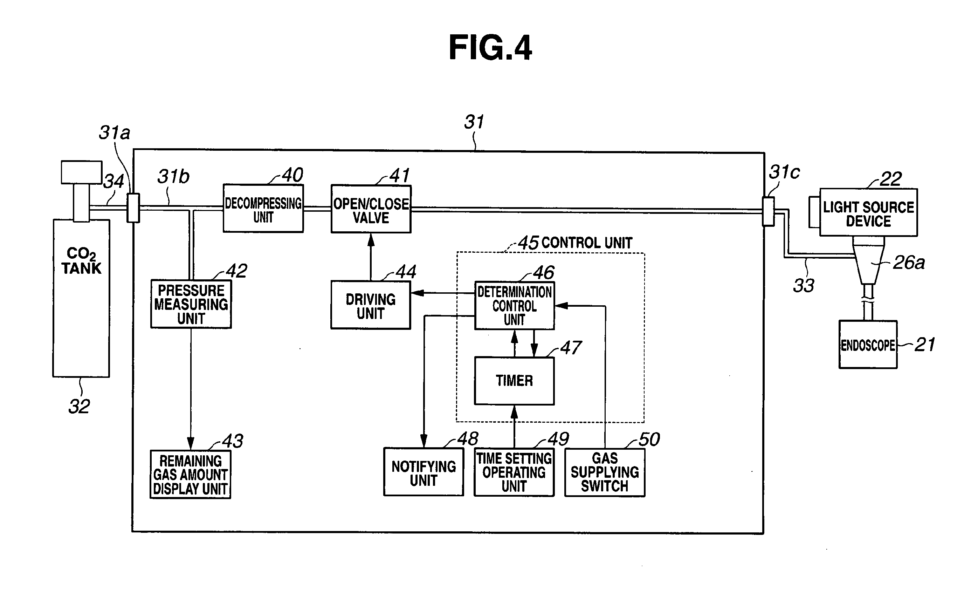

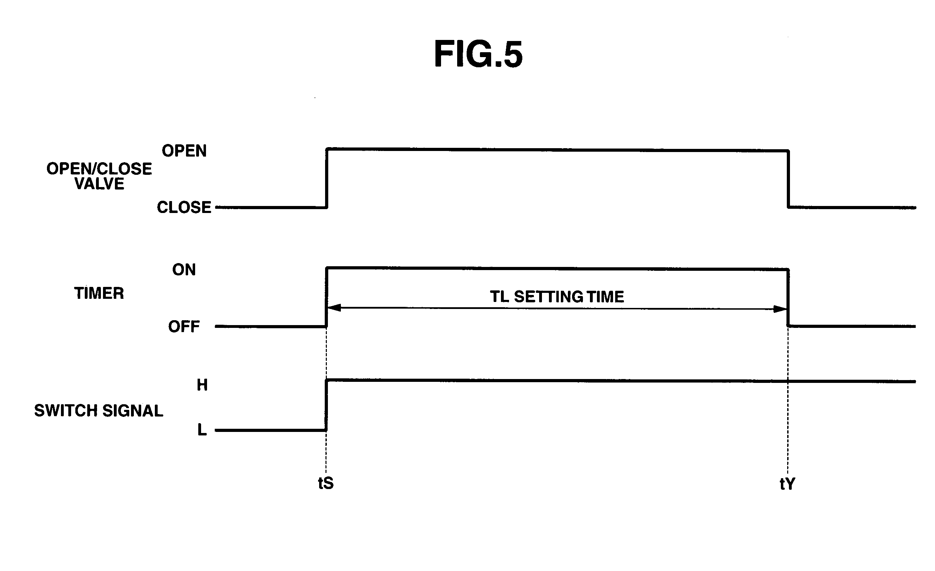

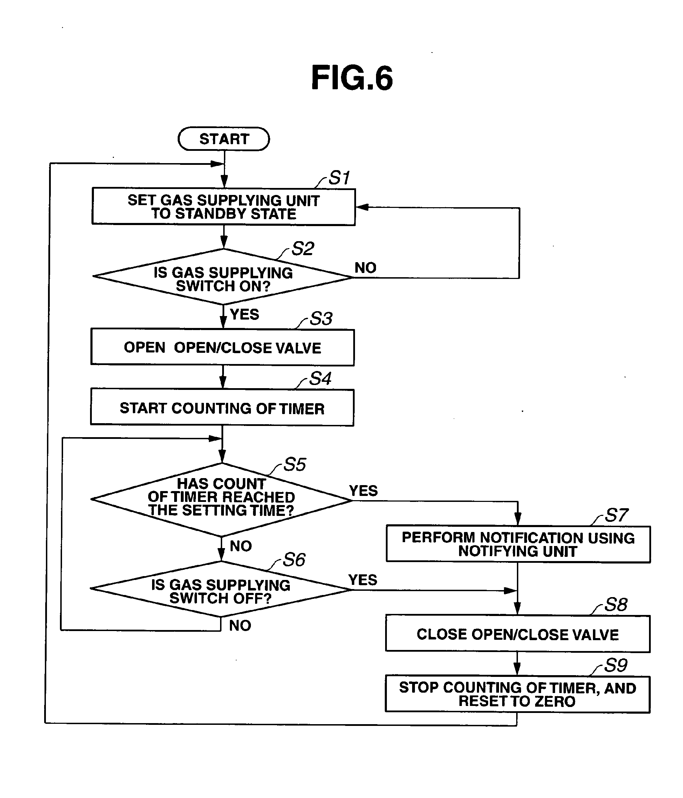

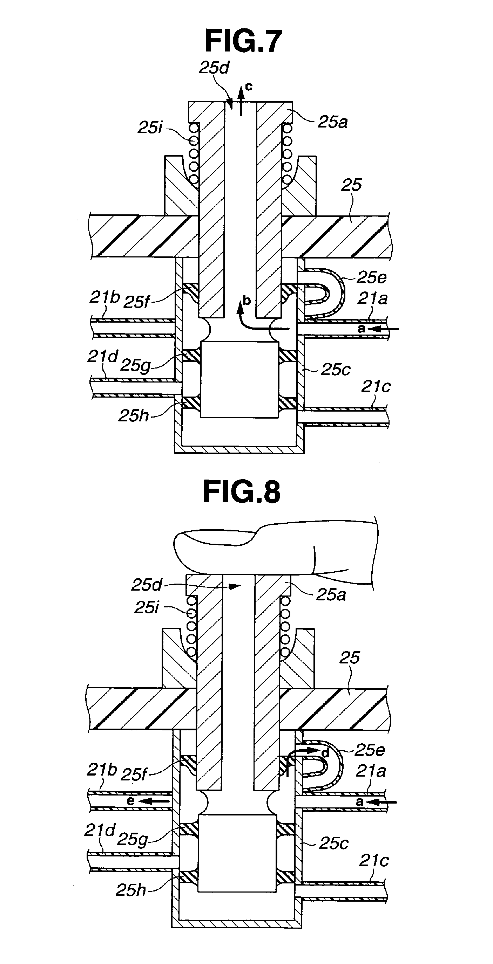

Fig. 1 through Fig. 8 relate to a first embodiment of the present invention, wherein Fig. 1 is a diagram illustrating a configuration example of an endoscope system including a gas supplying apparatus, Fig. 2 is a front view of the gas supplying apparatus shown in Fig. 1, Fig. 3 is a rear view of the gas supplying apparatus shown in Fig. 1, Fig. 4 is a block diagram describing a configuration example of the inside of the gas supplying apparatus shown in Fig. 1, Fig. 5 is a timing chart describing an operation of the gas supplying apparatus, Fig. 6 is a flowchart illustrating a control example of the gas supplying apparatus, Fig. 7 is a cross-sectional view describing a leaked state in which carbon dioxide gas is ejected from a hole portion provided in a gas supplying/water supplying button, Fig. 8 is a cross-sectional view describing a state in which the hole portion provided in the gas supplying/water supplying button is closed, and carbon dioxide gas is supplied to an insertion portion side.

An endoscope system according to the present embodiment shown in Fig. 1 is a laparoscope surgery system (hereafter, referred to as surgery system) 1. The surgery system 1 principally comprises an endoscope system 2, a gas supplying system 3, a system controller 4, a monitor 5 serving as a display device, a central display panel 6, a central operating panel 7, and a cart 8. Note that a surgical table 9 upon which a patient 10 lies is provided in an operating room in which the surgery system 1 is disposed.

The endoscope system 2 includes an endoscope (flexible endoscope) 21 having a flexible insertion portion 24 to be inserted into a lumen such as the large intestine for example, a light source device 22 serving as illumination light supplying means, and a camera control unit (hereafter, referred to as CCU) 23.

The endoscope 21 comprises the insertion portion 24, an operation portion 25, and a universal cord 26. The distal end portion of the insertion portion 24 is, though not shown in the drawing, provided with an image pickup device such as CCD, CMOS, or the like for example.

The operation portion 25 is provided with a gas supplying/water supplying button 25a, a suction button 25b, a bending operation knob 27 for subjecting an unshown bending portion to bending operation, and a treatment-instrument insertion opening 28 communicatively connected to an unshown treatment instrument channel. The proximal end portion of the universal cord 26 is provided with an endoscope connector 26a.

The light source device 22 includes an illumination lamp (not shown) serving as illumination means which supplies illumination light to the endoscope 21, and so forth. The light source device 22 is configured to be detachably connected with the endoscope connector 26a, which includes a light source connector.

The endoscope connector 26a is connected to the light source device 22, thereby providing a layout state in which the light source connector stands face to face with the illumination lamp. Accordingly, illumination light emitted from the illumination lamp is transmitted through an unshown light guide fiber, and is emitted from an unshown illumination window provided at the distal end portion of the insertion portion 24.

Also, the endoscope connector 26a is provided with a gas supplying connector 26c communicatively connected to an upstream-side gas supplying channel 21 a via a gas supplying channel within the universal cord 26. The gas supplying connector 26c is connected with one end portion of a gas supplying tube 33. With the gas supplying tube 33, an arrangement is made wherein the proximal end portion thereof is communicatively connected to a later-described gas supplying apparatus 31 of the gas supplying system 3, thereby supplying gas.

Note that a water supplying tube, though not shown in the drawing, extends from the endoscope connector 26a, and is connected to a water supplying tank. For example, water serving as liquid is stored within the water supplying tank.

Gas supplied from the gas supplying system 3 passes through a gas supplying channel (not shown) within the endoscope connector 26a, and a gas supplying channel within the universal cord 26, and is transmitted to the operation portion 25 via the upstream-side gas supplying channel 21a (see Fig. 5). Subsequently, the gas is transmitted from the gas supplying/water supplying button 25a provided on the operation portion 25. Also, simultaneously, the gas sent from the gas supplying system 3 pressurizes the inside of the water supplying tank via a channel within the water supplying tube (not shown).

Therefore, a surgeon operates the gas supplying/water supplying button 25a to cause the upstream-side water supplying channel 21 a and a later-described downstream-side water supplying channel 21 b to have a communicative connection state (see Fig. 6), thereby ejecting the gas or water transmitted to the water supplying channel of the endoscope 21 from a gas supplying base provided at the unshown distal end portion of the insertion portion 24.

The CCU 23 drives and controls the image pickup device provided at the unshown distal end portion of the insertion portion 24 of the endoscope 21, and converts an electric signal photoelectrically-converted from an image formed at the image pickup device into a video signal. The video signal converted at the CCU 23 is outputted to the monitor 5 or central display panel 6 for example. Thus, an endoscope image of a subject captured by the endoscope 21 is displayed on the monitor 5 or a screen of the central display panel 6. Note that reference numeral 29 denotes an electric cable configured to electrically connect between the electric connector 26b provided at the endoscope connector 26a and the CCU 23.

The gas supplying system 3 is a system configured to supply predetermined gas into a lumen, such as carbon dioxide gas (hereafter, referred to carbon dioxide) which is readily absorbed by a living body, for example. The gas supplying system 3 principally comprises the gas supplying apparatus 31, a gas tank for lumen (hereafter, referred to as tank) 32 in which carbon dioxide serving as predetermined observation gas is liquidized and stored, and a gas supplying tube 33.

The gas supplying apparatus 31 is provided with a high-pressure connector 31 a, and a gas supplying connector 31c. The gas supplying connector 31c is communicatively connected with one end portion of the gas supplying tube 33, and the other end portion of the gas supplying tube 33 is communicatively connected to the gas supplying connector 26c of the endoscope connector 26a connected to the light source device 22. Note that the gas supplying tube 33 is formed of silicon or Teflon (registered trademark).

A high-pressure gas tube 34 extending from the gas tank 32 is communicatively connected to the high-pressure connector 31a provided in the gas supplying apparatus 31.

The system controller 4 centrally controls the entire surgery system 1. The system controller 4 is connected with the central display panel 6, central operating panel 7, light source device 22, CCU 23, and gas supplying apparatus 31, via an unshown communication line, so as to be capable of two-way communication.

An endoscope image of a subject captured by the endoscope 21 is arranged to be displayed on the screen of the monitor 5 in response to the video signal outputted from the CCU 23. A display screen such as a liquid crystal display or the like is provided on the central display panel 6. The central display panel 6 is connected to the system controller 4. Accordingly, in the case of including an endoscope peripheral device, an operation state of the endoscope peripheral device can be displayed on the display screen in a central manner as well as the above-mentioned endoscope image of the subject.

The central operating panel 7 comprises a display portion such as a liquid crystal display or the like, and a touch sensor portion (not shown) integrally provided on the display screen of the display portion. The display portion of the central operating panel 7 includes a display function for displaying the operating switches and so forth of the endoscope peripheral device as a setting screen, and an operation function for operating an operating switch displayed by touching a predetermined area of the touch sensor portion.

In other words, the central operating panel 7 is connected to the system controller 4, whereby the same operation as in the case of directly operating the operating switch corresponding to the displayed endoscope peripheral device can be performed by operating the touch sensor portion displayed on the display portion as appropriate. That is to say, the surgeon can remotely perform various types of operations or settings or the like of the endoscope peripheral device on the central operating panel 7.

The light source device 22, CCU 23, gas supplying apparatus 31, system controller 4, monitor 5, central display panel 6, central operating panel 7, gas tank 32, which are peripheral devices, and so forth are mounted on the cart 8.

Note that the surgery system 1 according to the present embodiment may include, in addition to the endoscope system 2, a second endoscope system comprising a light source device 11, CCU 12, unshown rigid endoscope, and an endoscope camera, an electrocautery apparatus 13 serving as an endoscope peripheral device, and so forth, which are configured to perform the laparoscope surgery of a patient 10.

Next, the configuration of the above-mentioned gas supplying apparatus 31 will be described with reference to Fig. 2 through Fig. 4. As shown in Fig. 2, a power switch 25, a switch 50, and a gas supplying connector 31 c are provided in the front lower side of the gas supplying apparatus 31.

The power switch 25 is an operating switch configured to turn on/off the power of the gas supplying apparatus 31. The switch 50 is an operating switch configured to turn on/off supply of gas by the gas supplying apparatus 31. The gas supplying connector 31 c is connected with one end portion of the gas supplying tube 33 from the endoscope connector 26a.

Also, a remaining gas amount display unit 43 is provided in the front upper side of the gas supplying apparatus 31. The remaining gas amount display unit 43 displays the remaining amount of carbon dioxide gas within the gas tank 32.

As shown in Fig. 3, the high-pressure connector 31 a and a power connector 36 are provided in the rear lower side of the gas supplying apparatus 31. The high-pressure connector 31a is connected with a high-pressure gas tube 34 extending from the gas tank 32 as described above. The power connector 36 is connected with the connector of an unshown power cable configured to supply power to the gas supplying apparatus 31.

Also, a time setting operating unit 49 is provided in the rear upper side of the gas supplying apparatus 31. The time setting operating unit 49 is an operating switch configured to set the operation time of the gas supplying apparatus 31 using a later-described timer function.

For example, the time setting operating unit 49 is a three-step-type switch made up of "timer function OFF", "15 min", and "30 min", as shown in Fig. 3, and any one of the three types of timer time can be set by a lever 49a being slid.

Note that "timer function OFF" is a mode employing no timer function. Also, the time setting operating unit 49 is not restricted to the two-step operation time (gas supplying time) according to the level of skill of the surgeon employing the gas supplying apparatus 31, of "15 min", and "30 min" at the time of performing the timer function, and further is not restricted to such a operation time range, but rather various types of operation time setting may be performed arbitrarily.

Also, the time setting operating unit 49 is configured by employing a slide-type switch, but is not restricted to this, but rather may be configured, for example, by employing a volume-type switch wherein a knob is rotated, thereby enabling the operation time to be set arbitrarily.

Next, as shown in Fig. 4, description will be made regarding the internal configuration of the gas supplying apparatus 31 capable of setting the operation time serving as gas supplying time using the timer function set by the time setting operating unit 49. As shown in Fig. 4, the gas supplying apparatus 31 includes a decompressing unit 40, an open/close valve 41 serving as switching means and also a switching unit, a pressure measuring unit 42, the remaining gas amount display unit 43, a driving unit 44, a channel gas supplying control unit (hereafter, referred to as control unit) 45 serving as control means, a notifying unit 48 serving as notifying means, the time setting operating unit 49, and a switch 50.

The high-pressure connector 31 a of the gas supplying apparatus 31 is connected with a high-pressure gas tube 34 extending from the gas tank 32, and carbon dioxide gas is arranged to be supplied from the gas tank 32 via the high-pressure gas tube 34.

The high-pressure connector 31a is connected with a gas supplying channel 31 b, and the gas supplying channel 31 b is communicatively connected to the decompressing unit 40, and the pressure measuring unit 42. Also, the gas supplying channel 31 b is communicatively connected to a gas supplying connector 31 c via the decompressing unit 40 and the open/close valve.

The pressure measuring unit 42 measures the pressure of the carbon dioxide gas evaporated and supplied from the gas tank 32, and outputs measurement result to the remaining gas amount display unit 43. The remaining gas amount display unit 43, as shown in Fig. 2, displays the residual amount of the carbon dioxide gas within the gas tank 32 based on the measurement result from the pressure measuring unit 42.

The decompressing unit 40 decompresses the carbon dioxide supplied via the high-pressure connector 31a to predetermined pressure.

The open/close valve 41 performs open/close operation based on a driving signal outputted from the driving unit 44. Thus, the gas supplying flow rate at the output side of the open/close valve 41 is regulated. The output of the open/close valve 41 is supplied to the gas supplying connector 31c via the gas supplying channel 31b.

The driving unit 44 generates a driving signal for opening/closing the open/close valve 41 based on a control signal from the later-described control unit 45. Also, the driving unit 44 supplies the driving signal, thereby controlling the open/close operation of the open/close valve 41.

The driving unit 44, notifying unit 48, time setting operating unit 49, and switch 50 are electrically connected to the control unit 45.

The notifying unit 48 is made up of, for example, either of speakers which emit sound or a display portion, or a combination of the speakers and the display portion. The notifying unit 48 is configured to notify the surgeon or the like of completion (idle state) of the operation time of the gas supplying apparatus 31 by sound under the control of the control unit 45.

The time setting operating unit 49 outputs a setting signal of the operation time set as described above to the timer 47 serving as the time measuring means of the control unit 45, which makes up a time measuring unit. Also, the switch 50 outputs a switch signal configured to control supply of gas by the gas supplying apparatus 31 to be switched to the determination control unit 46.

The control unit 45 controls the operation of the whole gas supplying apparatus 31, and includes the determination control unit 46, and the timer 47.

The timer 47 includes an unshown timer counter. The timer 47 counts the operation time (setting time) based on the setting signal from the time setting operating unit 49 employing the timer counter, and simultaneously outputs the count value (time information) thus counted to the determination control unit 46.

Also, with the timer 47, counting by the timer counter is configured to be reset under the control of the determination control unit 46.

The switch signal from the switch 50, and the time information from the timer 47 are inputted to the determination control unit 46. The determination control unit 46 performs the notification control of the notifying unit 48, the open/close control of the open/close valve 41 via the driving unit 44, the reset control of the timer counter in the timer 47, and so forth using the time information from the timer 47 based on a later-described program stored in an unshown storing unit (see Fig. 6).

For example, upon the switch signal from the switch 50 being inputted, the determination control unit 46 controls the driving unit 44 to turn the open/close valve 41 to an open state, and simultaneously starts counting of the timer counter of the timer 47.

The determination control unit 46 compares the count value (time information) of the timer 47 to be supplied and the operation time (setting time) set by the time setting operating unit 49. Upon the count value reaching the operation time, the determination control unit 46 drives the notifying unit 48 to notify the surgeon or the like of the effect that the operation of the gas supplying apparatus 31 is stopped by sound and (or) display, and simultaneously controls the driving unit 44 to turn the open/close valve 41 to a closed state.

Thus, the surgeon can stop supply of gas by the gas supplying apparatus 31 at predetermined operation time after completion of observation by the endoscope guided into the lumen, operation, or the like. Thus, even if the gas supplying/water supplying button 25a is in an opened state, the carbon dioxide gas stored within the gas tank is prevented from flowing continuously wastefully.

Next, description will be made regarding the supply operation of carbon dioxide gas to a lumen by the gas supplying apparatus 31 provided in the surgery system 1 thus configured with reference to Fig. 5 through Fig. 8.

With the gas supplying apparatus 31 provided in the surgery system 1 according to the present embodiment, upon power being turned on, the determination control unit 46 activates a program shown in Fig. 6.

As shown in Fig. 6, the determination control unit 46 turns the gas supplying apparatus 31 into a standby state capable of supplying gas by the processing in step S1 (S1). The determination control unit 46 determines whether or not the switch 50 is ON based on the switch signal supplied from the gas supplying switch (switch) 50 by the processing in step S2 (S2). In this case, in the event that determination is made by the determination control unit 46 that the switch 50 is not ON (when the switch signal is in a low level, see Fig. 5), the processing is returned to step S1, where standby is performed until the switch 50 is turned on.

In the event of determining that the switch 50 is ON (when the switch signal is in a high level, see Fig. 5), the determination control unit 46 controls the driving unit 44 to open the open/close valve 41 by the subsequent processing in step S3 at point-in-time tS shown in Fig. 5 (S3). Thus, carbon dioxide gas is supplied to the inside of the lumen from a gas supplying base provided at the distal end portion of the insertion portion 24.

In this case, the carbon dioxide gas supplied from the gas supplying connector 31c of the gas supplying apparatus 31 reaches the operation portion 25 via the gas supplying tube 33, a gas supplying channel (not shown) within the endoscope connector 26a, a gas supplying channel within the universal cord 26, and an upstream-side gas supplying channel 21e (see Fig. 5). The carbon dioxide gas reaches a gas supplying/water supplying button cylinder (hereafter, referred to as gas supplying/water supplying cylinder) 25c where the gas supplying/water supplying button 25a provided on the operation portion 25 is disposed.

Now, in the event that a hole portion 25d provided in the gas supplying/water supplying button 25a is in an opened state, as shown in Fig. 7, the carbon dioxide gas is turned into a leaked state in which the carbon dioxide gas is discharged from the hole portion 25d to the outside as shown with an arrow a, arrow b, and arrow c in the drawing.

On the other hand, as shown in Fig. 8, in the event that the hole portion 25d provided in the gas supplying/water supplying button 25a is closed by a finger of the surgeon, the carbon dioxide gas supplied via the upstream-side gas supplying channel 21 a is supplied to the downstream-side gas supplying channel 21 b via a crooked tube 25e without being leaked out from the hole portion 25d such as shown with an arrow a, arrow d, and arrow e in the drawing. This provides a state of carbon dioxide gas supplying to the inside of the lumen via the base.

Note that reference numeral 21c denotes an upstream-side water supplying channel, reference numeral 21d denotes a downstream-side water supplying channel, reference numeral 25f denotes a check valve, reference numerals 25g and 25h denote packing, and reference numeral 25i denotes a spring.

Also, in the state shown in Fig. 8, upon the gas supplying/water supplying button 25a being depressed for a predetermined amount against the pressing force of the spring 25i, the positions of the check valve 25f, packing 25g, and packing 25h are moved, which results in a state in which the upstream-side water supplying channel 21 c and the downstream-side water supplying channel 21d are communicatively connected.

The determination control unit 46, following completion of the processing in step S3, controls the timer counter of the timer 47 to start counting thereof in the subsequent processing in step S4 (S4).

The determination control unit 46 compares between the count value (time information) of the timer 47 supplied from the timer 47, and the operation time set beforehand by the time setting operating unit 49 (setting time TL shown in Fig. 5) by the subsequent processing in step S5, and determines whether or not the count value reaches the setting time (S5).

In this case, in the event of determining that the count value has not reached the setting time TL, the determination control unit 46 determines whether or not the switch 50 is OFF by the subsequent determination processing in step S6 (S6). In the event that the switch 50 is not OFF, the determination control unit 46 returns to the processing in step S5, but in the event of OFF, proceeds to later-described processing in step S8.

On the other hand, in the event of determining that the count value has reached the above-mentioned setting time in the determination processing in step S5, the determination control unit 46 drives the notifying unit 48 to notify the surgeon or the like of the effect that the operation of the gas supplying apparatus 31 is stopped, by sound and (or) display, at time tY shown in Fig. 5 (S7). Thus, the gas supplying apparatus 31 according to the present embodiment can notify the surgeon or the like to the effect that the count value has reached the setting time TL set beforehand, and the supply of gas is automatically stopped.

The determination control unit 46 controls the driving unit 44 to turn the open/close valve 41 to a closed state at the time tY by the subsequent processing in step S8 (S8). That is to say, supply of gas to the lumen by the gas supplying apparatus 31 is stopped.

Thus, in the event that the gas supplying/water supplying button 25a is in an opened state (in a state in which the surgeon does not close the hole portion 25d of the gas supplying/water supplying button 25a as shown in Fig. 7), the gas supplying apparatus 31 according to the present invention can prevent carbon dioxide gas from flowing continuously wastefully via the gas supply/water supplying button 25a.

Subsequently, the determination control unit 46 outputs a timer-counting reset signal of the timer 47 to the timer 47 by the subsequent processing in step S9, thereby stopping counting of the timer 47, controlling the timer 47 to reset counting to zero, and returning the processing to the above-mentioned step S1 (S9).

Therefore, according to the present embodiment, performing control such as described above enables supply of gas by the gas supplying apparatus 31 to be automatically stopped after the setting time TL set beforehand elapses since the start of supplying gas. Therefore, the gas supplying apparatus 31 according to the present embodiment can prevent the turning off of the switch from being forgotten after completion of observation by the endoscope 21 guided to the inside of the lumen, or operation, and also can prevent carbon dioxide gas stored within the gas tank from flowing continuously wastefully from the gas supplying/water supplying button 25a.

Incidentally, the setting time set beforehand by the gas supplying apparatus 31 according to the present embodiment is set depending on the level of skill of the surgeon in the case of performing large intestine endoscopy for example, but it is not necessarily the case that all the surgeons finish observation or treatment or the like within the above-mentioned setting time TL. The present invention has improved such a point, and such an embodiment example will be shown below.

### (Second Embodiment)

Next, description will be made regarding a second embodiment.

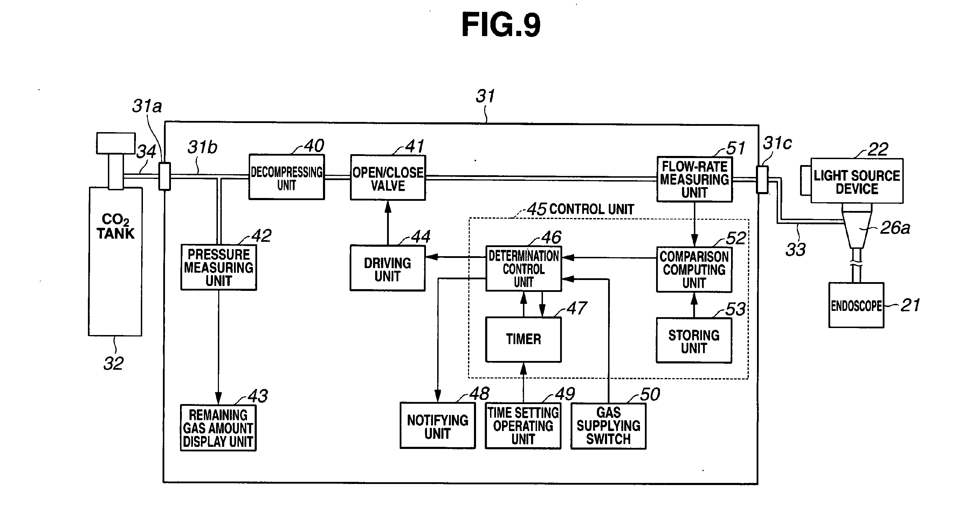

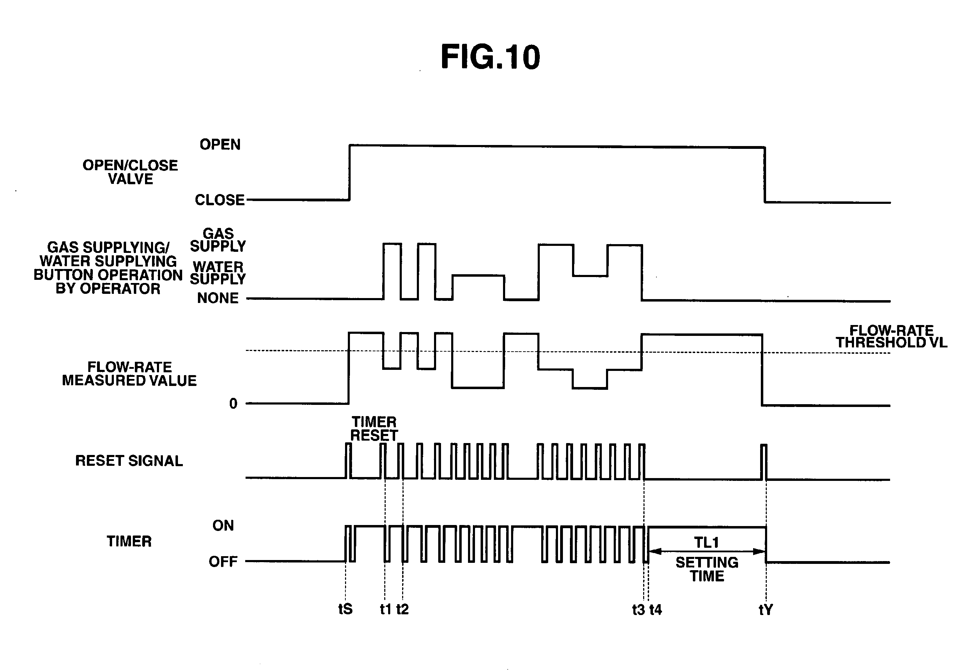

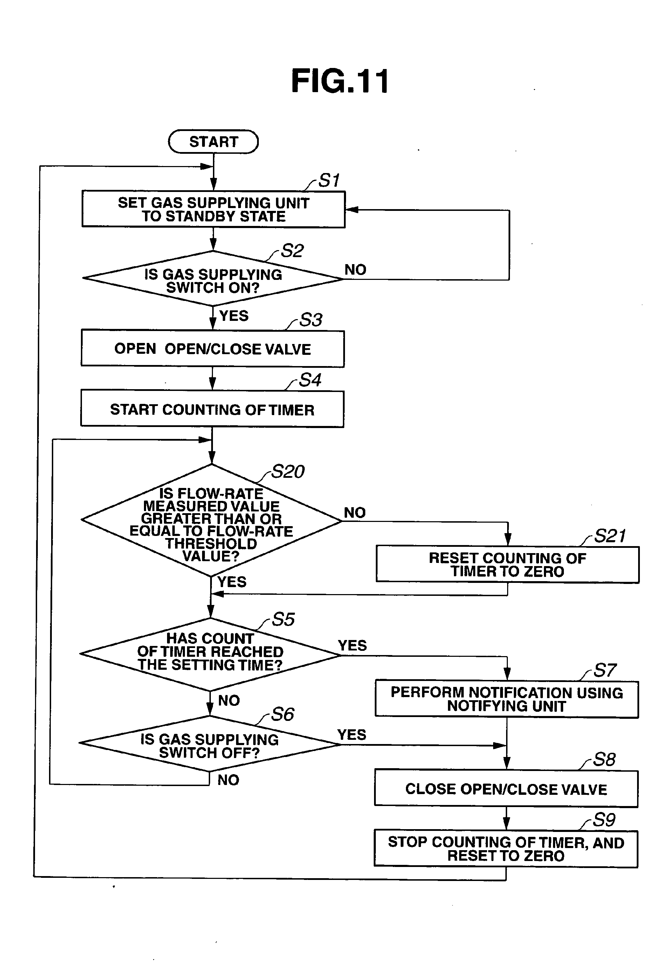

Fig. 9 through Fig. 11 relate to the second embodiment of the present invention, wherein Fig. 9 is a block diagram describing a configuration example of a gas supplying apparatus, Fig. 10 is a timing chart describing an operation of the gas supplying apparatus, and Fig. 11 is a flowchart illustrating a control example of the gas supplying apparatus. Note that as for Fig. 9 through Fig. 11, the same reference numerals and the same step S numbers are provided to the same components and processing contents as those in the first embodiment, description thereof will be omitted, and only different portions will be described.

The overall configuration of an surgery system according to the present embodiment is generally the same as the configuration shown in Fig. 1 according to the first embodiment, but the configuration of the gas supplying apparatus 31 employed for the surgery system 1 differs.

As shown in Fig. 9, the gas supplying apparatus 31 has generally the same configuration as the configuration shown in Fig. 4, but further includes a flow-rate measuring unit 51, a comparison computing unit 52 provided within the control unit 45, and a storing unit 53.

The flow-rate measuring unit 51 is, for example, a flow-rate sensor, and is disposed so as to be communicatively connected to a gas supplying channel 31 b between the open/close valve 41 and the gas supplying connector 31 c.

The flow-rate measuring unit 51 detects the flow rate of carbon dioxide gas supplied to the gas supplying connector 31 c, and outputs the detection result to the comparison computing unit 52.

The storing unit 53 stores, for example, a flow-rate threshold VL (see Fig. 10) that can be set arbitrarily. Note that, for example, an operation portion by which the threshold VL can be set is provided in the front of the gas supplying apparatus 31, and the surgeon can set the threshold VL arbitrarily using the operation portion, and can store this in the storing unit 53.

The comparison computing unit 52 performs calculation processing such that the flow-rate threshold VL is read out from the storing unit 53, and the readout flow-rate threshold VL and the flow-rate measured value serving as the detection result from the flow-rate measuring unit 51 are compared. Subsequently, the comparison computing unit 52 outputs the comparison result to the determination control unit 46 of the control unit 45.

That is to say, the comparison computing processing by the comparison computing unit 52 is for determining whether or not there is a technique such as observation or the like by the gas supplying/water supplying button 25a by comparing the flow-rate measured value and the threshold VL, thereby leading to the counting start timing of the timer 47.

Consequently, in the event that the flow-rate measured value is greater than the threshold VL as the comparison result from the comparison computing unit 52, the determination control unit 46 determines that a technique, such as observation or the like by the gas supplying/water supplying button 25a being operated, has not been performed, and performs counting of the timer counter at the timer 47.

On the other hand, in the event that the flow-rate measured value is smaller than the threshold VL, the determination control unit 46 determines that technique, such as observation or the like by the gas supplying/water supplying button 25a being operated, has been performed, and resets the timer counter at the timer 47. That is to say, the counting of the timer 47 is reset, thereby determining the counting start timing of the timer 47.

Thus, in the event that a technique, such as observation or the like by the gas supplying/water supplying button 25a being operated, has not been performed, the timer 47 is counted, but on the other hand, in the event that a technique, such as observation or the like by the gas supplying/water supplying button 25a being operated, has been performed, counting of the timer 47 is reset. Consequently, the counting start timing of the timer 47 which counts the setting time TL is automatically modified.

Thus, the gas supplying apparatus 31 according to the present embodiment can prevent stopping of supply of gas during the technique, such as observation or the like by the gas supplying/water supplying button 25a being operated. Also, the overall operation time of the gas supplying apparatus 31 is extended, thereby preventing supply of gas by the gas supplying apparatus 31 from being stopped even in the event that the technique has not been completed within the setting time TL. Further, the gas supplying apparatus 31 forcibly stops supply of gas after the setting time TL1 has elapsed since the technique, such as observation or the like by the gas supplying/water supplying button 25a being operated, was stopped.

Note that with the present embodiment, the setting time TL1 shown in Fig. 10 can be set arbitrarily using the time setting operating unit 49 for example, which is arranged to be set by time such as five minutes or the like for example. Note that the other configurations of the gas supplying apparatus 31 are the same as those in the first embodiment.

Next, description will be made regarding supply operation of carbon dioxide gas to the lumen by the gas supplying apparatus 31 provided in the surgery system 1 according to the present embodiment with reference to Fig. 10 and Fig. 11. Note that in Fig. 10, each timing is illustrated of an opened/closed state of the open/close valve 41, the button operation of the gas supplying/water supplying button 25a by a surgeon, a flow-rate measured value, the flow-rate threshold VL, a reset signal, and an ON/OFF state of the timer 47.

With the gas supplying apparatus 31 included in the surgery system according to the present embodiment, upon power being turned on, the determination control unit 46 activates a program shown in Fig. 11 stored in the unshown storing unit.

As shown in Fig. 11, with the program according to the present embodiment, determination processing in step S20 and processing in step S21 are provided between the processing in step S4 and the processing in step S5 with the program according to the first embodiment (see Fig. 6), and the other processing procedures and processing contents are the same as those in the first embodiment.

Accordingly, the determination control unit 46, as shown in Fig. 11, controls the driving unit 44 to open the open/close valve 41 by the processing in step S3 via step S1 and step S2 as with the first embodiment at the time tS shown in Fig. 10 to supply carbon dioxide gas to the inside of the lumen. Subsequently, the determination control unit 46 starts counting of the timer 47 by the processing in step S4.

The determination control unit 46 performs calculation processing such that the comparison computing unit 52 compares the flow-rate threshold VL read out from the storing unit 53 and the flow-rate measured value serving as the detection result from the flow-rate measuring unit 51 by the determination processing in step S20 newly added with the present embodiment (S20).

In this case, with the comparison result from the comparison computing unit 52, in the event that the flow-rate measured value is greater than the threshold VL, the determination control unit 46 determines that a technique, such as observation or the like by the gas supplying/water supplying button 25a being operated, has not been performed, and starts counting by the timer 47, and also proceeds to processing in the next step S5. Conversely, in the event that the flow-rate measured value is smaller than the threshold VL, the determination control unit 46 determines that the technique, such as observation or the like by the gas supplying/water supplying button 25a being operated, has been performed, and proceeds to processing in step S21.

The processing in step S21 is performed in the case of the determination control unit 46 determining that the flow-rate measured value is smaller than the threshold VL by the determination processing in step S20, so with the processing in step S21, the determination control unit 46 outputs a reset signal of counting at the timer 47 to the timer 47 at time t1 (time t2, time t3, and time tY) shown in Fig. 10, and stops counting of the timer 47 to reset to zero (S21). Subsequently, the determination control unit 46 proceeds to the processing in step S5.

With the following processing, the count value of the timer 47 and the setting time TL1 (see Fig. 10) are compared in step S5 as with the first embodiment, determination processing in step S6 or notification control processing by the notifying unit 48 in step S7 is performed depending on the comparison result.

The processing in step S6 is performed in the case of the determination control unit 46 determining that the count value has not reached the setting time TL1, so with the processing in step S6, the determination control unit 46 determines whether or not the switch 50 is OFF (S6). In the event that the switch 50 is not OFF, the determination control unit 46 returns to the processing in step S5, but in the event of OFF, proceeds to processing in step S8.

On the other hand, following the notification control processing being performed by the notifying unit 48 in step S7, the determination control unit 46, as with the first embodiment, controls the driving unit 44 to turn the open/close valve 41 to a closed state at time tY shown in Fig. 10 by processing in step S8 (S8).

That is to say, detection is ultimately made at time t3 that the technique, such as observation or the like by the gas supplying/water supplying button 25a being operated, has not been performed, supply of gas by the gas supplying apparatus 31 is stopped along with this detection following the setting time TL1 (e.g., five minutes) having elapsing since time t4 when the timer 47 was reset.

Therefore, according to the gas supplying apparatus 31 according to the present embodiment, the counting start timing of the timer 47 configured to count the setting time TL1 can be automatically modified, thereby preventing stopping supply of gas during the technique, such as observation or the like, by the gas supplying/water supplying button 25a being operated. Also, the overall operation time is extended, whereby the gas supplying apparatus 31 does not stop supplying the gas even if the technique is not completed within the setting time TL.

Also, the gas supplying apparatus 31 according to the present embodiment forcibly stops supply of gas after the setting time TL1 has elapsed since technique, such as observation or the like by the gas supplying/water supplying button 25a being operated, was stopped, whereby, as with the first embodiment, the carbon dioxide gas stored within the gas tank can be prevented from flowing continuously wastefully from the gas supplying/water supplying button 25a.

Note that with the gas supplying apparatus 31 according to the present embodiment, a flow rate while supplying gas/water, while leaking from the gas supplying/water supplying button 25a depends on the type of the endoscope 21. Accordingly, there is a possibility of erroneous determination by employing the same flow-rate threshold regardless of the type of the endoscope 21.

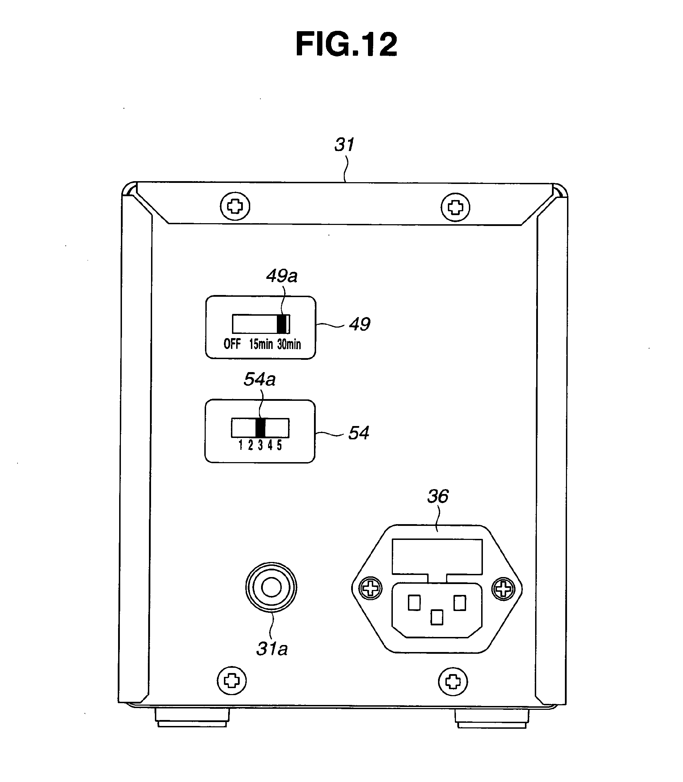

Therefore, in order to prevent such erroneous determination, the gas supplying apparatus 31 according to the present embodiment may be configured such as shown in a later-described first modification and second modification. Next, the first and second modifications of the gas supplying apparatus 31 according to the present embodiment will be described with reference to Fig. 12 through Fig. 16.

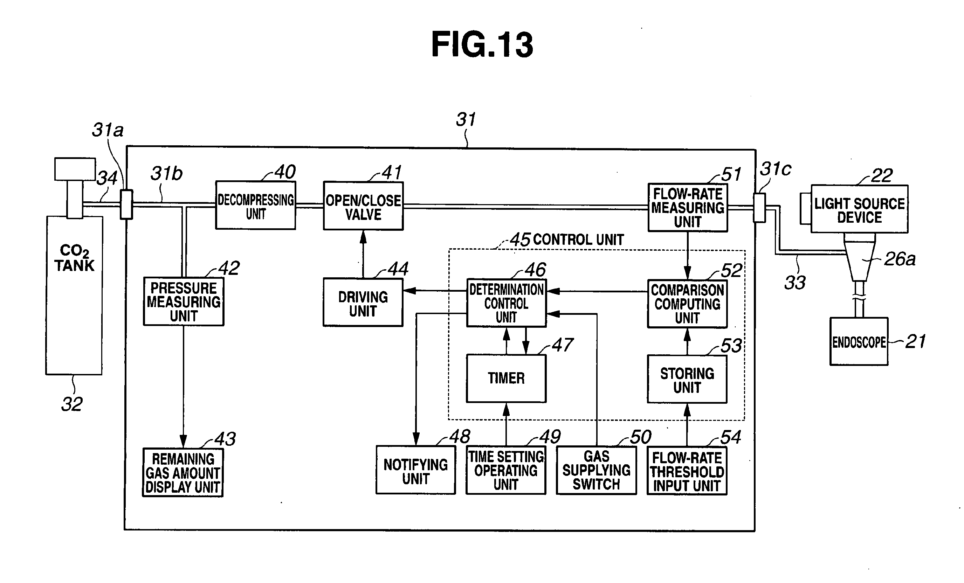

Fig. 12 and Fig. 13 are diagrams describing the first modification, wherein Fig. 12 is a rear view of a gas supplying apparatus according to the first modification, and Fig. 13 is a block diagram describing an internal configuration example of the gas supplying apparatus according to the first modification. Note that in Fig. 12 and Fig. 13, the same components as those in the above-mentioned embodiments are provided with the same reference numerals, and only the different portions will be described.

As shown in Fig. 12 and Fig. 13, with the gas supplying apparatus 31 according to the first modification, in addition to the configuration in the second embodiment, a flow-rate threshold input unit 54 is provided.

The flow-rate threshold input unit 54 is, as shown in Fig. 12, provided in the vicinity of the time setting operating unit 49 at the rear side of the gas supplying apparatus 31. The flow-rate threshold input unit 54 is a five-step-type switch capable of switching five-step levels whose flow-rate thresholds differ for example. Specifically, the flow-rate threshold input unit 54 is capable of setting any one of the five levels of flow-rate threshold by sliding a lever 54a.

For example, the five-step levels are flow-rate thresholds from "level 1" to "level 5" whose flow-rate thresholds differ, which are set beforehand in accordance with the available types of the endoscope 21, "level 1" represents the smallest flow-rate threshold, and "level 5" represents the largest flow-rate threshold.

The flow-rate threshold input unit 54 outputs the flow-rate threshold set by the lever 54a being operated to the storing unit 53. The flow-rate threshold of the level thus set is stored in the storing unit 53.

That is to say, the flow-rate threshold VL employed at the comparison computing unit 52 according to the second embodiment becomes the flow-rate threshold set by the flow-rate threshold input unit 54.

Note that the flow-rate threshold input unit 54 may be variable resistance type switch which can be set arbitrarily by a sliding operation of a lever within a flow-rate threshold range determined depending on the employed endoscope 21 beforehand. The other configurations of the gas supplying apparatus 31 are the same as those in the above-mentioned embodiments.

The gas supplying apparatus 31 according to the present modification is operated by generally the same control method as that in the second embodiment (program shown in Fig. 11 ), but with the determination processing in step S20, the comparison computing unit 52 performs comparison determination processing as to the flow-rate measured value by employing the flow-rate threshold VL set by the flow-rate threshold input unit 54. The processing contents other than that are the same processing contents in the respective steps S shown in Fig. 11.

Therefore, according to the gas supplying apparatus 31 according to the present modification, in addition to the advantages of the second embodiment, the flow-rate threshold corresponding to the employed endoscope 21 can be set, whereby erroneous determination can be prevented regarding whether or not the gas supplying/water supplying button 25a has been operated, and also gas supplying stop control with sufficient precision depending on the type of the endoscope 21 can be performed.

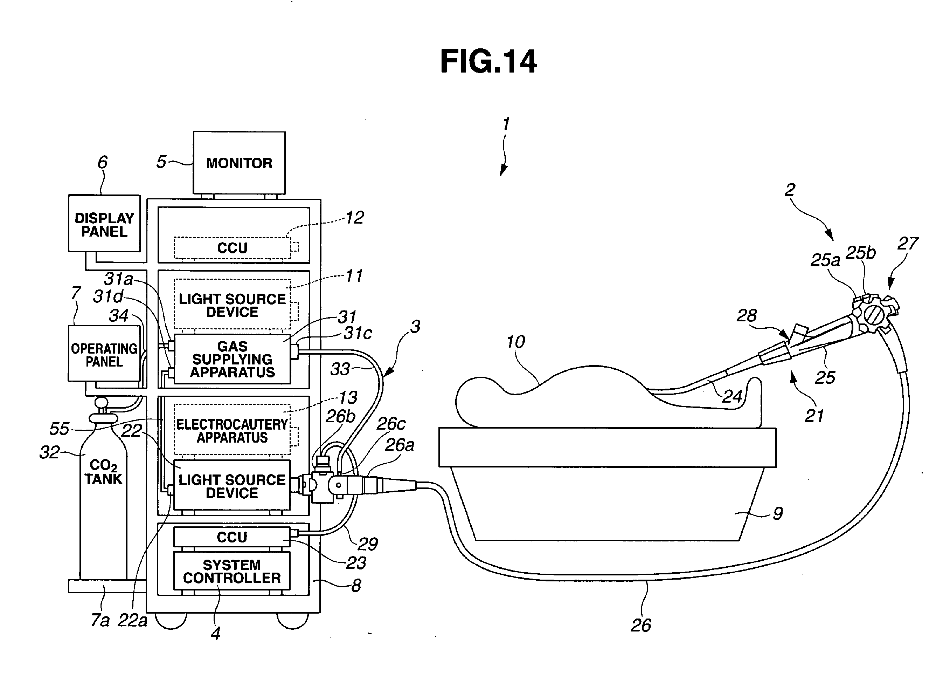

Next, description will be made regarding the second modification with reference to Fig. 14 through Fig. 16.

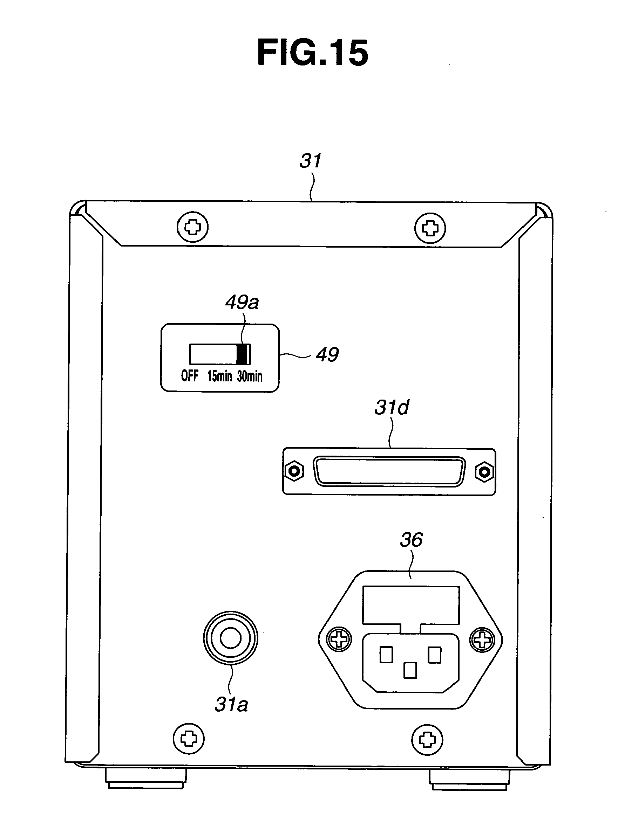

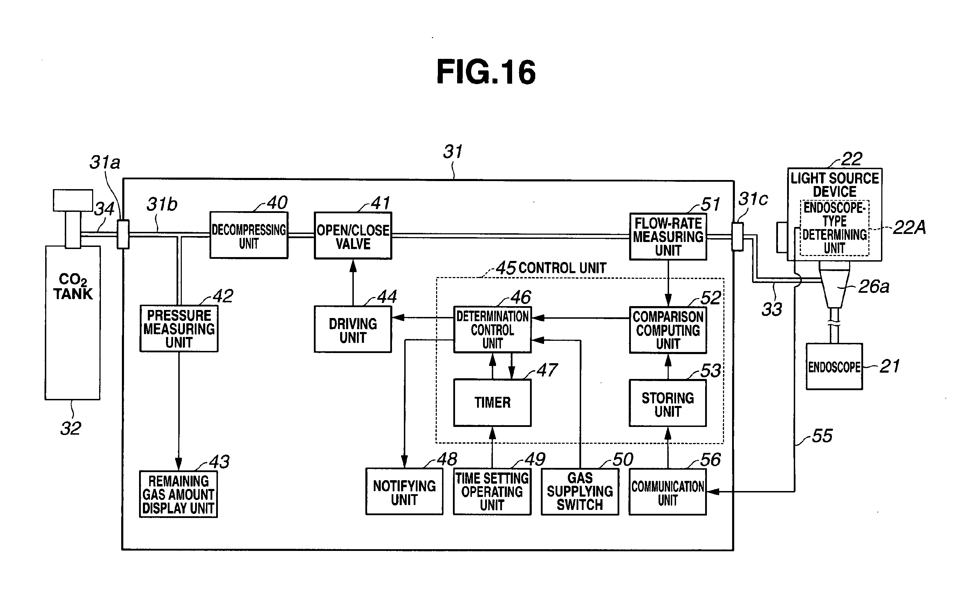

Fig. 14 through Fig. 16 are diagrams describing the second modification, wherein Fig. 14 is a configuration diagram illustrating a configuration example of an endoscope system including a gas supplying apparatus according to the second modification, Fig. 15 is a rear view of the gas supplying apparatus according to the second modification, and Fig. 16 is a block diagram describing configuration examples of the inside of the gas supplying apparatus and a light source device according to the second modification. Note that in Fig. 14 through Fig. 16, the same components as those in the above-mentioned embodiments are provided with the same reference numerals, and only the different portions will be described.

With the first modification, the flow-rate threshold VL corresponding to the endoscope 21 is inputted via the flow-rate threshold input unit 54, but on the other hand, with the gas supplying apparatus 31 according to the present embodiment, the flow-rate threshold based on the type of the endoscope 21 connected from the light source device 22 is detected and acquired, and the flow-rate threshold VL corresponding to the endoscope 21 is set.

As shown in Fig. 14 and Fig. 15, a connection connector 31d configured to perform communication by electrically being connected to the light source device 22 is provided at the rear side of the gas supplying apparatus 31 according to the present modification. The connection connector 31d is connected with a connector (not shown) provided on one end portion of a connection cable 55. The other end portion of the connection cable 55 is configured to be connected to a connector 22a provided in the light source device 22.

The endoscope 21 employed for the present modification includes, though not shown in the drawing, an ID signal generating unit capable of transmitting an ID signal indicating the type of the endoscope 21. The ID signal generating unit transmits an ID signal to the light source device 22 when the endoscope 21 is connected to the light source device 22 via the endoscope connector 26a.

An endoscope classifying unit 22A configured to receive an ID signal is, as shown in Fig. 16, provided in the light source device 22. The endoscope classifying unit 22A determines the type of the endoscope 21 connected based on the received ID signal. Also, the endoscope classifying unit 22A determines the flow-rate threshold based on the determined endoscope 21 using an unshown flow-rate threshold table, and transmits the determined flow-rate threshold to the gas supplying apparatus 31 side via the connection cable 55.

The gas supplying apparatus 31 according to the present modification includes a communication unit 56 electrically connected to the connection connector 31d. The communication unit 56 receives the flow-rate threshold transmitted from the light source device 22 via the connection cable 55 and the connection connector 31d, and outputs the threshold to the storing unit 53 as with the first modification.

Thus, with the first modification, the gas supplying apparatus 31 obtains a flow-rate threshold corresponding to the endoscope 21 using the flow-rate threshold input unit 54, but with the second modification, the gas supplying apparatus 31 can automatically obtain a flow-rate threshold corresponding to the connected endoscope 21 only by connecting the endoscope 21 to the light source device 22.

The other configurations of the gas supplying apparatus 31 are the same as those in the above-mentioned embodiments.

Also, the control method of the gas supplying apparatus 31 according to the present modification is the same as that in the first modification.

Therefore, according to the gas supplying apparatus 31 according to the present modification, in addition to the advantages of the first modification, a flow-rate threshold corresponding to the employed endoscope 21 can be automatically obtained without input operations, whereby a flow-rate threshold corresponding to the endoscope 21 can be simply set.

### (Third Embodiment)

Next, description will be made regarding a third embodiment.

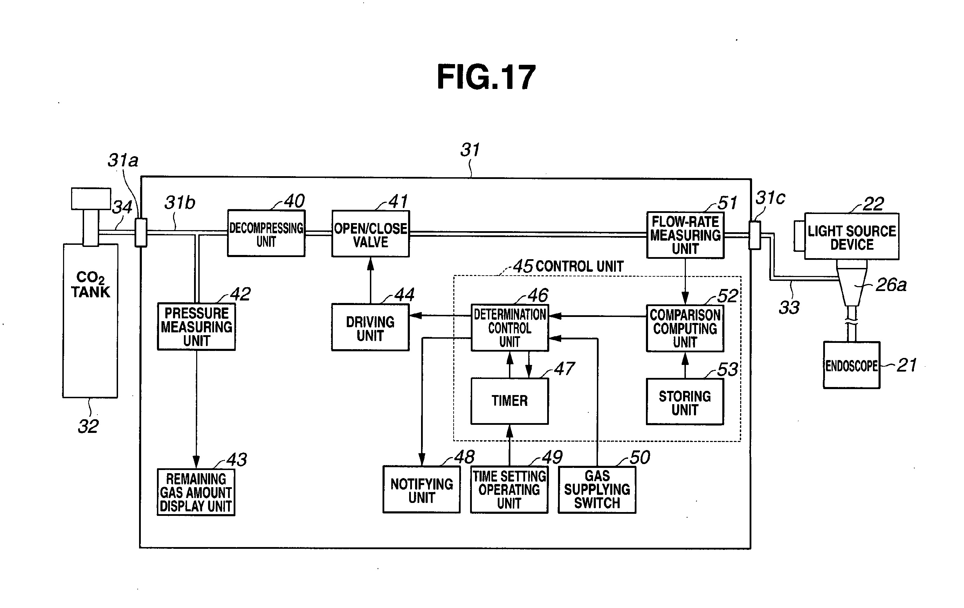

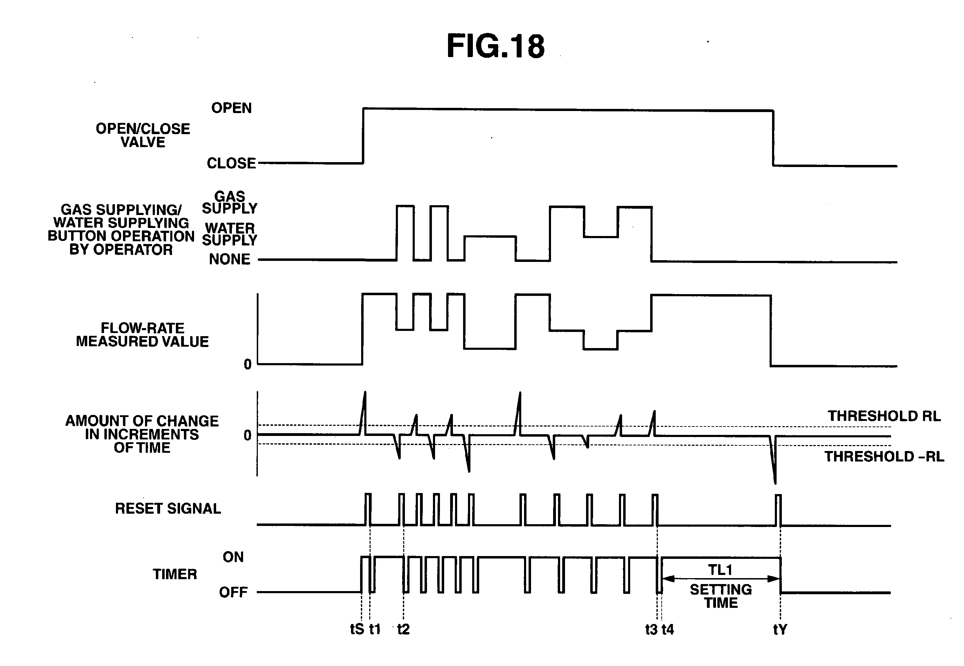

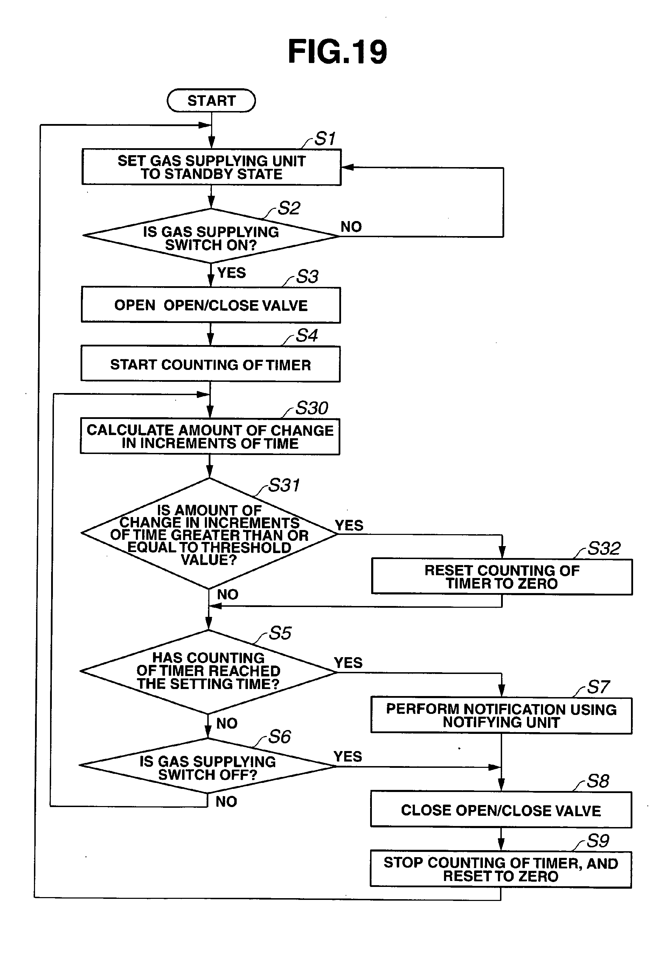

Fig. 17 through Fig. 19 relate to the third embodiment of the present invention, wherein Fig. 17 is a block diagram describing a configuration example of a gas supplying apparatus, Fig. 18 is a timing chart describing an operation of the gas supplying apparatus, and Fig. 19 is a flowchart illustrating a control example of the gas supplying apparatus. Note that as for Fig. 17 and Fig. 18, the same reference numerals and the same step S numbers are provided to the same components and processing contents as those in the above-mentioned embodiments, description thereof will be omitted, and only different portions will be described.

A gas supplying apparatus 31 employed for an surgery system 1 according to the present embodiment is, as shown in Fig. 17, generally the same as the configuration shown in Fig. 9 according to the second embodiment, but the comparison computing processing contents by the comparison computing unit 52 differ.

Note that the storing unit 53 stores a positive flow-rate threshold RL and a negative flow-rate threshold -RL, which are necessary for performing the comparison computing processing by the comparison computing unit 52.

Employing the flow-rate measured value before the setting time set beforehand, and the current flow-rate measured value, the comparison computing unit 52 calculates the amount of change in a flow rate in increments of time (corresponding to the above-mentioned setting time). The comparison computing unit 52 performs calculation processing so as to compare the calculated amount of change in a flow rate in increments of time with the flow-rate thresholds RL and-RL (absolute values) read out from the storing unit 53, and outputs the comparison result to the determination control unit 46 of the control unit 45.

That is to say, the comparison computing processing by the comparison computing unit 52 is for determining with sufficient accuracy whether or not there is technique such as observation or the like by the gas supplying/water supplying button 25a being operated, by comparing the amount of change in a flow rate in increments of time and the flow-rate thresholds RL and -RL, thereby obtaining the counting start timing (start period) of the timer 47.

That is to say, with the comparison result from the comparison computing unit 52, in the event that the amount of change in a flow rate in increments of time is greater than the flow-rate thresholds RL and -RL (absolute values), the determination control unit 46 determines that technique such as observation or the like by the gas supplying/water supplying button 25a being operated has been performed, resets the timer counter of the timer 47. In other words, the counter of the timer 47 is reset, thereby determining the counting start timing of the timer 47.

Note that as shown in Fig. 18, the amount of change in a negative flow rate in increments of time means that gas supplying operation is performed in the case of the gas supplying/water supplying button 25a being operated from a gas supplying state, and thus the amount of the gas supplying flow rate decreases. Accordingly, the flow-rate threshold for performing comparison as to the amount of change in a negative flow rate becomes the flow-rate threshold -RL, and the determination control unit 46 is arranged to employ the absolute values of the flow-rate thresholds RL and -RL.

On the other hand, in the event that the amount of change in a flow rate in increments of time is smaller than (equal to zero in some cases) the flow-rate thresholds RL and -RL (absolute values), the determination control unit 46 determines that technique such as observation or the like by the gas supplying/water supplying button 25a being operated has not been performed, and counts the timer counter of the timer 47.

Thus, in the event that technique such as observation or the like by the gas supplying/water supplying button 25a being operated has been performed, the determination control unit 46 resets the timer 47, but on the other hand, in the event that technique such as observation or the like by the gas supplying/water supplying button 25a being operated has not been performed, counts the timer 47, whereby the counting start timing (start period) of the timer 47 configured to count the setting time TL can be automatically modified.

Thus, the gas supplying apparatus 31 is prevented from stopping supply of gas during technique such as observation or the like by the gas supplying/water supplying button 25a being operated. Also, the overall operation time is extended, whereby the gas supplying apparatus 31 does not stop the supply of gas even if technique is not completed within the setting time TL.

Also, the gas supplying apparatus 31 forcibly stops supply of gas after the setting time TL1 has elapsed since technique, such as observation or the like by the gas supplying/water supplying button 25a being operated, was stopped. Further, with the gas supplying apparatus 31 according to the present embodiment, the comparison determination processing by the determination control unit 46 can be performed with a sufficient accuracy without obtaining the flow-rate threshold corresponding to the type of the endoscope 21 such as the first and second modifications of the second embodiment.

Note that with the present embodiment, the setting time TL1 shown in Fig. 18 can be set arbitrarily, for example, by using the time setting operating unit 49 as with the second embodiment, and let us say that the setting time TL1 is set to a time such as five minutes or the like.

The other configurations of the gas supplying apparatus 31 are the same as those in the above-mentioned embodiments.

Next, description will be made regarding carbon dioxide gas supply operation to the lumen by the gas supplying apparatus 31 provided in the surgery system 1 according to the present embodiment with reference to Fig. 18 and Fig. 19. Note that in Fig. 18, each timing is illustrated of an opened/closed state of the open/close valve 41, the button operation of the gas supplying/water supplying button 25a by a surgeon, a flow-rate measured value, the amount of change in increments of time (the amount of change in a flow rate), the flow-rate thresholds RL and -RL, a reset signal, and an ON/OFF state of the timer 47.

With the gas supplying apparatus 31 included in the surgery system 1 according to the present embodiment, upon power being turned on, the determination control unit 46 activates a program shown in Fig. 19 stored in the unshown storing unit.

As shown in Fig. 19, with the program according to the present embodiment, processing in step S30, determination processing in step S31, and processing in step S32 are provided instead of the determination processing in step S20 and the processing in step S21 between step S4 and step S5 with the program according to the second embodiment (see Fig. 11), and the other processing procedures and processing contents are the same as those in the second embodiment.

Accordingly, the determination control unit 46, as shown in Fig. 19, controls the driving unit 44 to open the open/close valve 41 by the processing in step S3 via step S 1 and step S2 as with the second embodiment at the time tS shown in Fig. 19. Thus, carbon dioxide gas is supplied to the inside of the lumen from the gas supplying base provided at the distal end portion of the insertion portion 24. Subsequently, the determination control unit 46 starts counting of the timer 47 by the processing in step S4.

Subsequently, the determination control unit 46 controls the comparison computing unit 52 to calculate the amount of change in a flow rate in increments of time (equivalent to setting time) using the flow-rate measured value before the setting time set beforehand, and the current flow-rate measured value by the processing in step S30 newly added to the present embodiment (S30).

Subsequently, the determination control unit 46 performs calculation processing such that the comparison computing unit 52 compares the amount of change in a flow rate in increments of time calculated in step S30, and the flow-rate thresholds RL and -RL (absolute values) read out from the storing unit 53 by the subsequent determination processing in step S31 (S31).

In this case, with the comparison result from the comparison computing unit 52, in the event that the amount of change in a flow rate in increments of time is smaller than the flow-rate thresholds RL and -RL, the determination control unit 46 determines that a technique, such as observation or the like by the gas supplying/water supplying button 25a being operated, has been performed, and starts counting by the timer 47, and also proceeds to processing in the next step S5. On the other hand, in the event that the amount of change in a flow rate in increments of time is greater than the flow-rate thresholds RL and -RL (absolute values), the determination control unit 46 determines that a technique, such as observation or the like by the gas supplying/water supplying button 25a being operated, has not been performed, and proceeds to processing in step S31.

With the processing in step S31, as with the processing in step S21 (see Fig. 11) in the second embodiment, the determination control unit 46 outputs a reset signal of counting at the timer 47 to the timer 47 at time t1 (time t2, time t3, and time tY) shown in Fig. 18, and stops counting of the timer 47 to reset to zero (S32), and proceeds to the processing in step S5.

With the following processing, the count value of the timer 47 and the setting time TL1 (see Fig. 18) are compared in step S5 as with the first embodiment, determination processing in step S6 or notification control processing by the notifying unit 48 in step S7 is performed depending on the comparison result.

The processing in step S6 is performed in the case of the determination control unit 46 determining that the count value has not reached the setting time TL1, so with the processing in step S6, the determination control unit 46 determines whether or not the switch 50 is OFF (S6), and in the event that the switch 50 is not OFF, the determination control unit 46 returns to the processing in step S5, but in the event of OFF, proceeds to processing in step S8.

On the other hand, following the notification control processing being performed by the notifying unit 48 in step S7, the determination control unit 46, as with the second embodiment, controls the driving unit 44 to turn the open/close valve 41 to a closed state at time tY shown in Fig. 18 by processing in step S8 (S8).

That is to say, detection is ultimately made at time t3 that a technique, such as observation or the like by the gas supplying/water supplying button 25a being operated, has not been performed, supply of gas by the gas supplying apparatus 31 is stopped along with this detection following the setting time TL1 (e.g., five minutes) having elapsing since time t4 when the timer 47 was reset.

Therefore, according to the present embodiment, the counting start timing of the timer 47 configured to count the setting time TL1 can be automatically modified, thereby preventing stopping the supply of gas during the technique, such as observation or the like by the gas supplying/water supplying button 25a being operated. Also, the overall operation time of the gas supplying apparatus 31 is extended, whereby the gas supplying apparatus 31 does not stop supply of gas even if the technique is not completed within the setting time TL.

Also, the gas supplying apparatus 31 according to the present embodiment can forcibly stop supply of gas after the setting time TL1 has elapsed since technique, such as observation or the like by the gas supplying/water supplying button 25a being operated, was stopped, whereby, with the second embodiment, carbon dioxide gas stored within the gas tank can be prevented from flowing continuously wastefully from the gas supplying/water supplying button 25a.

Further, with the gas supplying apparatus 31 according to the present embodiment, the comparison determination processing by the determination control unit 46 can be performed with a sufficient accuracy without obtaining the flow-rate threshold corresponding to the type of the endoscope 21 like the first and second modifications of the second embodiment.

Note that with the above-mentioned first through third embodiments, the gas supplying apparatus 31 which prevents the carbon dioxide gas, serving as gas for examination which is stored within the gas tank 32, from being consumed wastefully following completion of observation or operation by a flexible endoscope introduced into the lumen, and the surgery system 1 having the endoscope system 2 including the gas supplying apparatus 31, can be realized.

### (Fourth Embodiment)

Next, description will be made regarding a fourth embodiment.

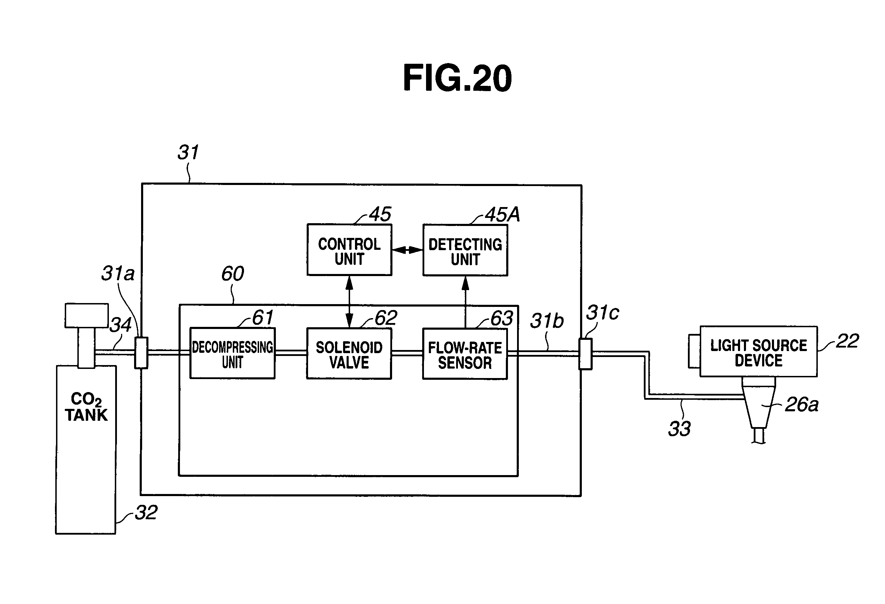

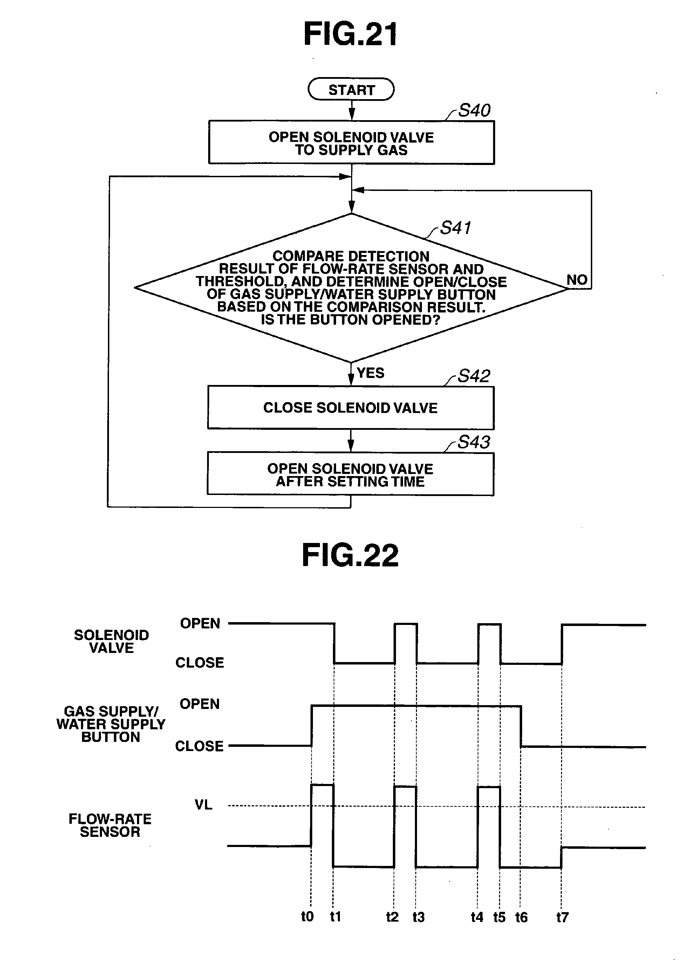

Fig. 20 through Fig. 22 relate to the fourth embodiment of the present invention, wherein Fig. 20 is a configuration diagram describing a configuration example of an endoscope system including a gas supplying apparatus, Fig. 21 is a block diagram illustrating a configuration example of the gas supplying apparatus in Fig. 20, and Fig. 22 is a flow chart describing a control example of the gas supplying apparatus, Fig. 4 is a timing chart describing an operation of the gas supplying apparatus. Note that with description of the present embodiment, the same reference numerals are provided to the same components as those in the above-mentioned embodiments, description thereof will be omitted, and only different portions will be described.

Next, description will be made regarding the configuration of the gas supplying apparatus 31 according to the present embodiment with reference to Fig. 20.

As shown in Fig. 20, the gas supplying apparatus 31 according to the present embodiment includes a valve unit 60, a detection unit 45A serving as detection means, and a lumen gas supplying control unit (hereafter, referred to as control unit) 45 serving as control means.

The high-pressure connector 31a of the gas supplying apparatus 31 is connected with the high-pressure gas tube 34 extending from the gas tank 32, and carbon dioxide gas is arranged to be supplied from the gas tank 32 via the high-pressure gas tube 34. Subsequently, the high-pressure connector 31 a is connected with the gas supplying channel 31 b, and the gas supplying channel 31 b is communicatively connected to the valve unit 60.

The valve unit 60 includes, for example, a decompressing unit 61, a solenoid valve 62 making up a gas supplying unit serving as gas supplying means, a flow-rate sensor 63 making up a flow-rate measuring unit serving as flow-rate measuring means, and so forth.

The decompressing unit 61 decompresses carbon dioxide gas supplied via the high-pressure connector 31a into a predetermined pressure.

The solenoid valve 62 performs open/close operation based on the control signal outputted from the control unit 45. Thus, the gas supplying flow rate at the output side of the solenoid valve 62 is arranged to be regulated.

The output of the solenoid valve 62 is supplied to the gas supplying connector 31c via the flow-rate sensor 63, and the gas supplying channel 31b.

The flow-rate sensor 63 detects the flow rate of carbon dioxide gas to be supplied to the gas supplying connector 31 c, and outputs the detection result to the detection unit 45A.

The detection unit 45A acquires the detection result, and detects whether or not carbon dioxide gas to be supplied into the body cavity is leaking from the gas supplying/water supplying button 25a of the endoscope 21 based on the detection result, and supplies the detection result to the control unit 45.

For example, the detection unit 45A first compares the flow-rate measured value serving as the detection result from the flow-rate sensor 63, and the threshold VL set beforehand (see Fig. 4). In the event that the flow-rate measured value is greater than the threshold VL, the detection unit 45A detects that the carbon dioxide gas to be supplied into the body cavity is leaking from the gas supplying/water supplying button 25a of the endoscope 21. On the other hand, in the event that the flow-rate measured value is smaller than the threshold VL, the detection unit 45A detects that the carbon dioxide gas to be supplied into the body cavity is not leaking from the gas supplying/water supplying button 25a of the endoscope 21.

That is to say, in the event that the detection unit 45A determines that the carbon dioxide gas to be supplied into the body cavity is leaking from the gas supplying/water supplying button 25a of the endoscope 21, the gas supplying/water supplying button 25a is in an opened state (state in which the surgeon is not closing the hole portion 25d of the gas supplying/water supplying button 25a). On the other hand, in the event that the detection unit 45A determines that the carbon dioxide gas to be supplied into the body cavity is not leaking from the gas supplying/water supplying button 25a of the endoscope 21, the gas supplying/water supplying button 25a is in a closed state (state in which the surgeon is closing the hole portion 25d of the gas supplying/water supplying button 25a).

The control unit 45 controls the operation of the valve unit 60. Specifically, the control unit 45 controls the open/close operation of the solenoid valve 62 based on the detection result from the detection unit 45A. Note that the control unit 45 is provided with an unshown timer, and the control unit 45 obtains point-in-time information from this timer.

The control unit 45 executes detection by the detection unit 45A using point-in-time information from the timer, determination processing, and the open/close control of the solenoid valve 62 based on the detection result, based on a later-described program stored in the unshown storing unit (see Fig. 21).

Next, description will be made regarding the supply operation of carbon dioxide gas to the lumen by the gas supplying apparatus 31 provided in the surgery system 1 thus configured with reference to Fig. 21, Fig. 22, and Fig. 7 and Fig. 8 employed for the first embodiment. With the gas supplying apparatus 31 included in the surgery system 1 according to the present embodiment, upon power being turned on, the control unit 45 activates a program shown in Fig. 21.

As shown in Fig. 21, the control unit 45 controls the solenoid valve 62 to open so as to supply carbon dioxide gas to the inside of the body cavity by the processing in step S40 (S40).

In this case, the carbon dioxide gas from the gas supplying connector 31c of the gas supplying apparatus 31 reaches the gas supplying/water supplying button cylinder (hereafter, referred to as gas supplying/water supplying cylinder) 25c where the gas supplying/water supplying button 25a provided on the operation portion 25 is disposed via the gas supplying tube 33, the gas supplying channel (not shown) within the endoscope connector 26a, the gas supplying channel within the universal cord 26, and the upstream-side gas supplying channel 21a (see Fig. 7).

In the event that the hole portion 25d provided in the gas supplying/water supplying button 25a is in an opened state, as shown in Fig. 7, carbon dioxide gas is in a leaked state of being discharged from the hole portion 25d to the outside such as shown with an arrow a, arrow b, and arrow c in the drawing.

On the other hand, as shown in Fig. 8, in the event that the hole portion 25d provided in the gas supplying/water supplying button 25a is closed by a finger of the surgeon, the carbon dioxide gas supplied via the upstream-side gas supplying channel 21a is supplied to the downstream-side gas supplying channel 21 b via the crooked tube 25e without being leaked to the outside from the hole portion 25d such as shown with an arrow a, arrow d, and arrow e in the drawing. This results in an intra-lumen carbon dioxide gas supplying state in which the carbon dioxide gas is supplied to the inside of the lumen via the base.

Note that reference numeral 21 c denotes an upstream-side water supplying channel, reference numeral 21d denotes a downstream-side water supplying channel, reference numeral 25f denotes a check valve, reference numeral 25g and reference numeral 25h denote packing, and reference numeral 25i denotes a spring.

Also, in the state shown in Fig. 8, upon the gas supplying/water supplying button 25a being depressed for a predetermined amount against the pressing force of the spring 25i, the positions of the check valve 25f, packing 25g, and packing 25h are moved, which results in a state in which the upstream-side water supplying channel 21c and the downstream-side water supplying channel 21d are communicatively connected.

The control unit 45 controls the detection unit 45A to compare the flow-rate measured value serving as the detection result from the flow-rate sensor 63, and the threshold VL set beforehand (see Fig. 4) by the determination processing in step S41.

With the comparison results, in the event that the flow-rate measured value is greater than the threshold VL, the control unit 45 detects that the carbon dioxide gas to be supplied into the body cavity is leaking from the gas supplying/water supplying button 25a of the endoscope 21. That is to say, the control unit 45 determines that the gas supplying/water supplying button 25a is in an opened state (state in which the surgeon is not closing the hole portion 25d of the gas supplying/water supplying button 25a as shown in Fig. 7).

On the other hand, with the comparison results, in the event that the flow-rate measured value is smaller than the threshold VL, the control unit 45 detects that the carbon dioxide gas to be supplied into the body cavity is not leaking from the gas supplying/water supplying button 25a of the endoscope 21. That is to say, the control unit 45 determines that the gas supplying/water supplying button 25a is in a closed state (state in which the surgeon is closing the hole portion 25d of the gas supplying/water supplying button 25a as shown in Fig. 8).

Thus, the opened/closed state of the gas supplying/water supplying button 25a is detected during the gas supplying operation of the gas supplying apparatus 31 by the determination processing in step S41.

In the event of determining in the determination processing in step S41 that the gas supplying/water supplying button 25a is in an opened state (state in which supply of gas is not performed), the control unit 45 proceeds to the processing in step S43. On the other hand, in the event of determining that the gas supplying/water supplying button 25a is in a closed state (state in which supplying of gas is performed), the control unit 45 repeatedly executes step S41.

With the processing in step S42, the gas supplying/water supplying button 25a is in an opened state (leaked state), so the control unit 45 controls the solenoid valve 62 to close to prevent carbon dioxide gas from being consumed wastefully, thereby stopping supply of the gas. Subsequently, upon the setting time set beforehand elapsing, the control unit 45 controls the solenoid valve 62 to open after the setting time by the subsequent processing in step S43, and returns to the processing in step S41.

Fig. 22 illustrates a timing chart of the flow-rate measured value of the flow-rate sensor 63, the open/close operation of the gas supplying/water supplying button 25a, and the open/close operation of the solenoid valve 62, when actually performing such a control example.

As shown in Fig. 22, with the gas supplying apparatus 31, at and before point-in-time t0, the solenoid valve 62 is turned to an open state by the processing in step S40 under the control of the control unit 45, whereby carbon dioxide gas is supplied to the endoscope 21 via the gas supplying connector 31 c.

Subsequently, let us say that the surgeon takes off his/her finger from the hole portion 25d of the gas supplying/water supplying button 25a to turn the gas supplying/water supplying button 25a to an open state at point-in-time t1. Thus, the gas supplying/water supplying button 25a turns into a leaked state, thereby greatly increasing the gas supplying flow rate of the carbon dioxide gas.

Thus, at point-in-time t0, the control unit 45 performs the determination processing in step S41, whereby the detection unit 45A detects that the flow-rate measured value is greater than the threshold VL. That is to say, the control unit 45 determines that the gas supplying/water supplying button 25a is in an opened state.

Subsequently, the control unit 45 controls the solenoid valve 62 so as to close by the processing in step S42 based on the detection result. Thus, as a result of the solenoid valve 62 being closed, the gas supplying flow rate of the carbon dioxide gas decreases.

With the present embodiment, the control unit 45 performs the determination processing in step S41 for each setting time. Note that the setting time indicates time from point-in-time t1 to point-in-time t2 in Fig. 22. The setting time may be set arbitrarily, for example, by providing an operation portion capable of setting time on the central operating panel 7, and using the operation portion.

Subsequently, following the setting time elapsing, i.e., at point-in-time t2, the control unit 45 controls the solenoid valve 62 so as to open by the processing in step S43, thereby increasing the gas supplying flow rate of the carbon dioxide gas.

Subsequently, at point-in-time t3, the control unit 45 performs the determination processing in step S41 again, whereby the detection unit 45A detects that the flow-rate measured value is greater than the threshold VL. In this case also, determination is made that the gas supplying/water supplying button 25a is in an opened state.

Subsequently, based on the detection result, the control unit 45 controls the solenoid valve 62 so as to close by the processing in step S43 in the same way. Thus, as a result of the solenoid valve 62 being closed at point-in-time t3, the gas supplying flow rate of the carbon dioxide gas decreases.

Thus, the processing in step S41 through step S43 is executed within a period when the gas supplying/water supplying button 25a is in an opened state.

Thus, in the event that the gas supplying/water supplying button 25a is in an opened state, the control unit 45 performs the determination processing in step S41, and the open/close control processing of the solenoid valve 62 in step S42 and step S43 for each setting time. Consequently, the gas supplying flow rate to be leaked from the hole portion 25d of the gas supplying/water supplying button 25a can be reduced as compared with the gas supplying flow rate of carbon dioxide gas heretofore.

The surgeon closes the hole portion 25d of the gas supplying/water supplying button 25a by his/her finger at point-in-time t6, i.e., turns the gas supplying/water supplying button 25a to a closed state. Thus, the gas supplying flow rate of the carbon dioxide gas decreases.

Subsequently, the control unit 45 performs the determination processing in step S41 at point-in-time t7, whereby the detection unit 45A detects that the flow-rate measured value is smaller than the threshold VL. That is to say, the control unit 45 determines that the gas supplying/water supplying button 25a is in a closed state. At this time, the control unit 45 continues supply of the carbon dioxide gas without closing the solenoid valve 62.

Therefore, according to the present embodiment, the gas supplying flow rate of the carbon dioxide gas is reduced when the surgeon takes off his/her finger from the hole portion 25d of the gas supplying/water supplying button 25a, whereby the consumption of the carbon dioxide gas can be reduced.

### (Fifth Embodiment)

Next, description will be made regarding a fifth embodiment.

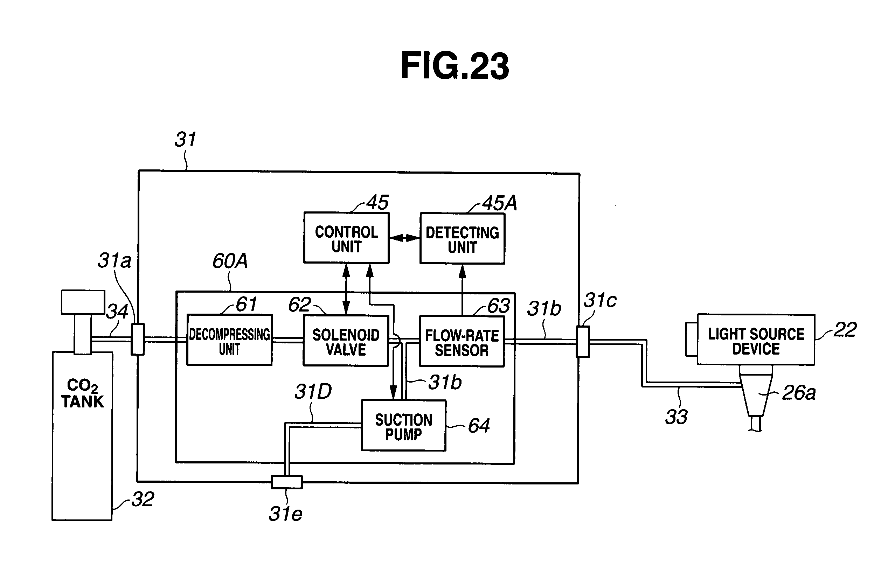

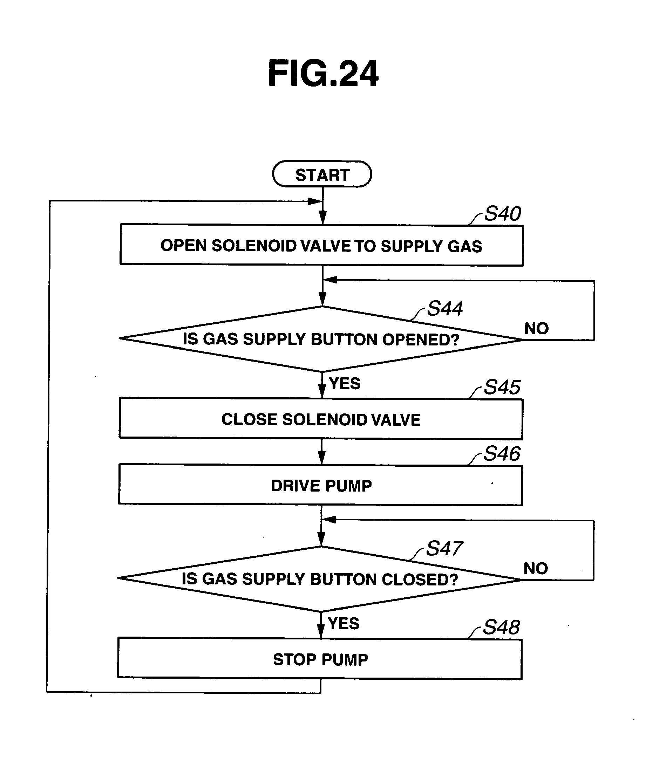

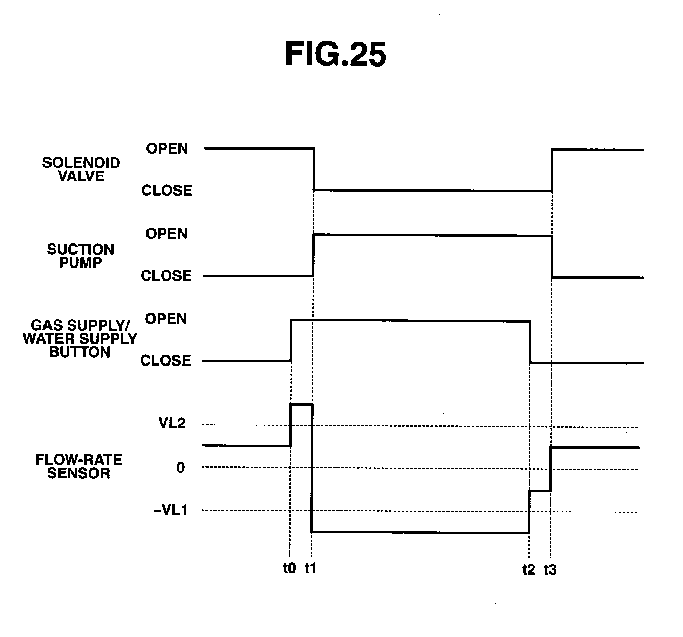

Fig. 23 through Fig. 25 relate to the second embodiment, wherein Fig. 23 is a block diagram describing a configuration example of a gas supplying apparatus according to the second embodiment, Fig. 24 is a flowchart illustrating a control example of the gas supplying apparatus, and Fig. 25 is a timing chart describing an operation of the gas supplying apparatus. Note that the same reference numerals and the same step S numbers are provided to the same components and processing contents as those in the above-mentioned embodiments and the fourth embodiment, so description thereof will be omitted, and only different portions will be described.

The overall configuration of an surgery system according to the present embodiment is generally the same as the configuration shown in Fig. 1 according to the above-mentioned embodiments, but the configuration of a gas supplying apparatus 31 employed for the surgery system 1 differs.

As shown in Fig. 23, the gas supplying apparatus 31 has generally the same configuration as that shown in Fig. 20 according to the fourth embodiment, but further includes a valve unit 60A having a suction pump 64, a connection tube 31 D connected to the suction pump 64, and an exhaust port 31e, which is connected with the proximal side of the connection tube 31D, configured to discharge the gas aspirated by the suction pump 64 into the atmosphere.

With the valve unit 60A, the suction pump 64 is disposed so as to communicatively connect to the gas supplying channel 31 b between the solenoid valve 62 and the flow-rate sensor 63.

Accordingly, the suction pump 64 is communicatively connected to the hole portion 25d (see Fig. 7) of the gas supplying/water supplying button 25a of the operation portion 25 via the gas supplying channel 31b, gas supplying tube 33, gas supplying channel (not shown) within the endoscope connector 26a, gas supplying channel within the universal cord 26, and the upstream-side gas supplying channel 21a (see Fig. 7).

The suction pump 64 is for suctioning the gas within the gas supplying channel 31b by being driven, and is arranged to be controlled by the control unit 45.

Upon being driven, the suction pump 64 aspirates the gas in the atmosphere via the inside of the gas supplying channel 31b, i.e., the hole portion 25d (see Fig. 5) of the gas supplying/water supplying button 25a communicatively connected to the gas supplying channel 31b.

The suction pump 64 discharges the aspirated gas into the atmosphere via the upstream-side gas supplying channel 21a (see Fig. 7), gas supplying channel within the universal cord 26, gas supplying channel (not shown) within the endoscope connector 26a, gas supplying tube 33, gas supplying channel 31b, inside of the suction pump 64, connection tube 31d, and exhaust port 31e, which is the opposite route at the time of supplying gas.

When the suction pump 64 stops, and also the solenoid valve 62 opens, the detection unit 45A compares the flow-rate measured value serving as the detection result from the flow-rate sensor 63, and the threshold VL2 (see Fig. 25) set beforehand. When the flow-rate measured value is greater than the threshold VL2, the detection unit 45A detects that the carbon dioxide gas to be supplied into the body cavity is leaking from the gas supplying/water supplying button 25a of the endoscope 21 (the gas supplying/water supplying button 25a is in an opened state).

On the other hand, when the solenoid valve 62 closes, and also the suction pump 64 is driven, the detection unit 45A compares the flow-rate measured value serving as the detection result from the flow-rate sensor 63, and the threshold -VL1 (see Fig. 25) set beforehand. Subsequently, when the flow-rate measured value is smaller than the threshold -VL1, the detection unit 45A detects that the gas in the atmosphere is aspirated from the gas supplying/water supplying button 25a of the endoscope 21 (the gas supplying/water supplying button 25a is in an opened state).

Note that the threshold VL2 and the threshold -VL1 can be set arbitrarily as with the fourth embodiment.

The control unit 45 performs driving control of the solenoid valve 62 and the suction pump 64 based on the comparison result from the detection unit 45A.

The other configurations are the same as those in the above-mentioned embodiments.

Next, description will be made regarding the supply operation of carbon dioxide gas to the lumen by the gas supplying apparatus 31 provided in the surgery system 1 according to the present embodiment.

With the gas supplying apparatus 31 provided in the surgery system 1 according to the present embodiment, upon power being turned on, the control unit 45 activates a program shown in Fig. 24 stored in the unshown storing unit.

As shown in Fig. 24, the control unit 45 controls the solenoid valve 62 to open so as to supply carbon dioxide gas into the body cavity by the processing in step S40.

Thus, the carbon dioxide gas from the gas supplying connector 31c of the gas supplying apparatus 31 reaches the gas supplying/water supplying cylinder 25c where the gas supplying/water supplying button 25a provided on the operation portion 25 is disposed via the gas supplying tube 33, the gas supplying channel (not shown) within the endoscope connector 26a, the gas supplying channel within the universal cord 26, and the upstream-side gas supplying channel 21a (see Fig. 7).

At this time, the control unit 45 controls the detection unit 45A to compare the flow-rate measured value serving as the detection result from the flow-rate sensor 63, and the threshold VL2 (see Fig. 25) set beforehand.

In the event that the flow-rate measured value is smaller than the threshold VL2, the control unit 45 determines that the carbon dioxide gas is supplied into the body cavity without being leaked from the gas supplying/water supplying button 25a. That is to say, the control unit 45 determines that the gas supplying/water supplying button 25a is in a closed state. In the event of determining that the gas supplying/water supplying button 25a is in a closed state, the control unit 45 repeatedly executes the processing in step S44 while continuing supply of the carbon dioxide.

On the other hand, in the event that the flow-rate measured value is greater than the threshold VL2, the control unit 45 determines that the carbon dioxide gas to be supplied into the body cavity is leaking from the gas supplying/water supplying button 25a of the endoscope 21. That is to say, the control unit 45 determines that the gas supplying/water supplying button 25a is in an opened state. In the event of determining that the gas supplying/water supplying button 25a is in an opened state, the control unit 45 proceeds to the processing in step S45.

The control unit 45 closes the solenoid valve 62 in step S45, and drives the suction pump 64 in step S46, thereby starting suction.

Upon the control unit 45 starting suction, the gas in the atmosphere is aspirated from the hole portion 25d (see Fig. 7) of the gas supplying/water supplying button 25a, and reaches the suction pump 64 via the upstream-side gas supplying channel 21a (see Fig. 7), gas supplying channel within the universal cord 26, gas supplying channel (not shown) within the endoscope connector 26a, gas supplying tube 33, and gas supplying channel 31b. The gas that has reached the suction pump 64 is discharged into the atmosphere via the connection tube 31d and exhaust port 31e.

At this time, the control unit 45 in the determination processing in step S47 controls the detection unit 45A to compare the flow-rate measured value serving as the detection result from the flow-rate sensor 63, and the threshold -VL1 (see Fig. 25) set beforehand (negative sign "-" represents that the gas flows in the opposite direction of the direction at the time of supplying carbon dioxide gas).

In the event that the flow-rate measured value is smaller than the threshold - VL1 (the flow rate of the gas flowing in the opposite direction of the direction at the time of supplying carbon dioxide gas is great), the control unit 45 determines that the gas in the atmosphere is aspirated from the gas supplying/water supplying button 25a. That is to say, the control unit 45 determines that the gas supplying/water supplying button 25a is in an opened state. In the event of determining that the gas supplying/water supplying button 25a is in an opened state, the control unit 45 repeatedly executes step S47 while continuing suction of the gas in the atmosphere.

On the other hand, in the event that the flow-rate measured value is greater than the threshold -VL1 (the flow rate of the gas flowing in the opposite direction of the direction at the time of supplying carbon dioxide gas is small), the control unit 45 determines that the gas in the atmosphere is not aspirated from the gas supplying/water supplying button 25a. That is to say, the control unit 45 determines that the gas supplying/water supplying button 25a is in a closed state. In the event of determining that the gas supplying/water supplying button 25a is in a closed state, the control unit 45 stops the suction pump 64 in step S48, and proceeds to the processing in step S40, thereby resuming supply of the carbon dioxide gas.

Fig. 25 illustrates a timing chart of the flow-rate measured value of the flow-rate sensor 63, the open/close operation of the gas supplying/water supplying button 25a by a surgeon, the open/close operation of the solenoid valve 62 and the open/close operation of the suction pump 64 in the gas supplying apparatus 31, when actually performing such a control example.

At and before point-in-time t0 shown in Fig. 25, the surgeon is closing the hole portion 25d of the gas supplying/water supplying button 25a, i.e., returns the gas supplying/water supplying button 25a to a closed state. Also, the control unit 45 controls the solenoid valve 62 to open so as to supply carbon dioxide gas into the body cavity by the processing in step S40.

Subsequently, the control unit 45 performs the determination processing in step S44, but the flow-rate measured value is smaller than the threshold VL2, so the control unit 45 fails to detect that the gas supplying/water supplying button 25a has opened, and consequently repeats the determination processing in step S44 until point-in-time t0.

The surgeon takes off his/her finger from the hole portion 25d of the gas supplying/water supplying button 25a at point-in-time t0, i.e., returns the gas supplying/water supplying button 25a to an open state. Thus, the carbon dioxide gas is leaking from the hole portion 25d of the gas supplying/water supplying button 25a, thereby increasing the gas supplying flow rate of the carbon dioxide gas.

Subsequently, the control unit 45 performs the determination processing in step S44 at point-in-time t1, whereby the detection unit 45A detects that the flow-rate measured value is greater than the threshold VL2. That is to say, the control unit 45 determines that the gas supplying/water supplying button 25a is in an opened state.

Subsequently, the control unit 45 controls the solenoid valve 62 so as to close by the processing in step S45, and controls the suction pump 64 so as to be driven by the processing in step S46, based on the detection result. Thus, at point-in-time t1, supply of the carbon dioxide gas is stopped, and suction of gas in the atmosphere by the suction pump 64 is started. Consequently, the gas supplying flow rate in the opposite direction of the direction at the time of supplying carbon dioxide gas increases greatly.

Subsequently, the control unit 45 performs the determination processing in step S47, but the flow-rate measured value is smaller than the threshold -VL, so the control unit 45 fails to detect that the gas supplying/water supplying button 25a has closed, and consequently repeats the determination processing in step S 13 until point-in-time t2.

The surgeon closes the hole portion 25d of the gas supplying/water supplying button 25a by his/her finger at point-in-time t2, i.e., returns the gas supplying/water supplying button 25a to a closed state. Thus, the suction pump 64 fails to aspirate the gas in the atmosphere, and accordingly the gas supplying flow rate in the opposite direction of the direction at the time of supplying carbon dioxide gas decreases.

Subsequently, the control unit 45 performs the determination processing in step S47 at point-in-time t3, whereby the detection unit 45A detects that the flow-rate measured value is greater than the threshold -VL. That is to say, the control unit 45 determines that the gas supplying/water supplying button 25a is in a closed state.

The control unit 45 controls the suction pump 64 so as to be stopped by the processing in step S48, and controls the solenoid valve 62 so as to open by the processing in step S40, based on the detection result. Thus, suction of the gas in the atmosphere by the suction pump 64 is stopped, and supply of carbon dioxide gas is resumed.

Therefore, according to the present embodiment, supply of carbon dioxide gas is not performed when the surgeon takes off his/her finger from the hole portion 25d of the gas supplying/water supplying button 25a, whereby consumption of carbon dioxide gas can be reduced.

### (Sixth Embodiment)

Next, description will be made regarding a sixth embodiment.

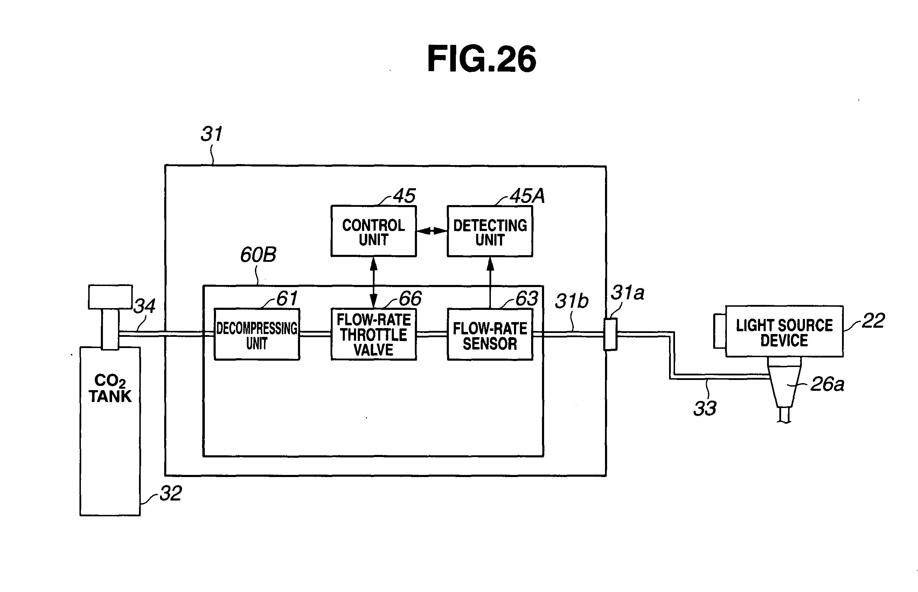

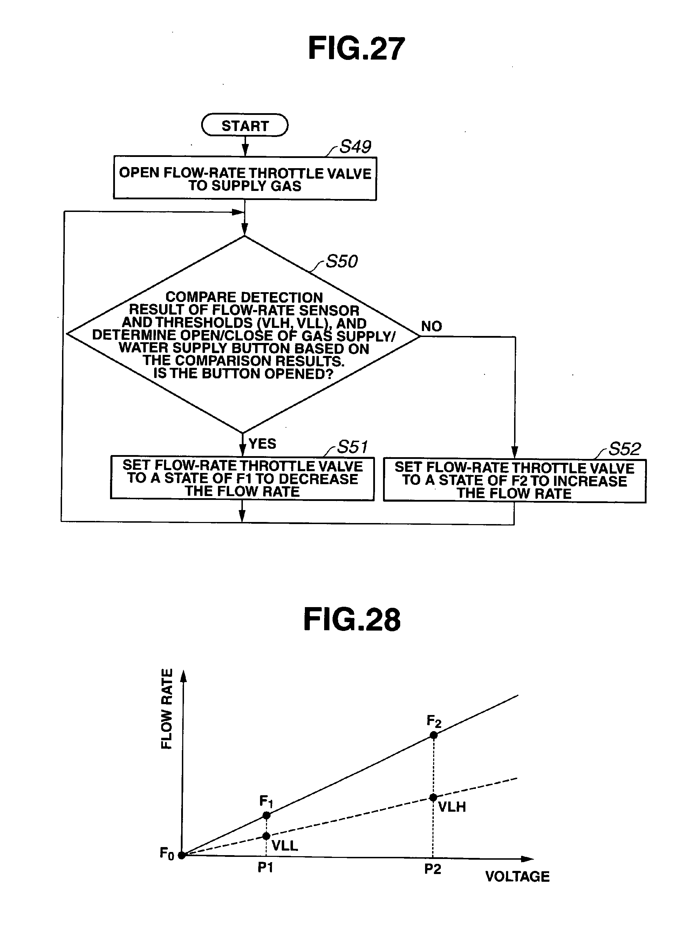

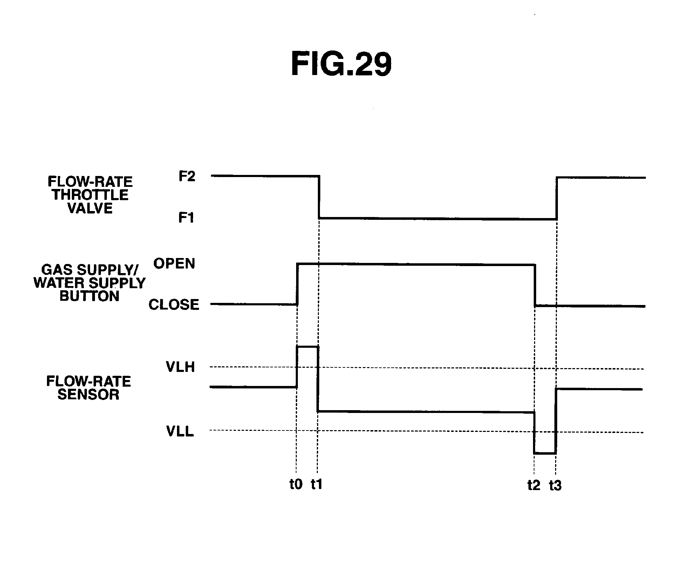

Fig. 26 through Fig. 29 relate to the fifth embodiment of the present invention, wherein Fig. 26 is a block diagram describing a configuration example of a gas supplying apparatus, Fig. 27 is a flowchart illustrating a control example of the gas supplying apparatus, Fig. 28 is a graph illustrating voltage-to-flow rate properties of a flow-rate throttle valve shown in Fig. 26, Fig. 29 is a timing chart describing an operation of the gas supplying apparatus. Note that the same reference numerals are provided to the same components as those in the above-mentioned embodiments, description thereof will be omitted, and only different portions will be described.

The overall configuration of an surgery system according to the present embodiment is generally the same as the configuration shown in Fig. 1 according to the above-mentioned embodiments. The gas supplying apparatus 31 employed for the surgery system 1, as shown in Fig. 26, includes a valve unit 60B whose internal configuration differs from those in the fourth and fifth embodiments.

The valve unit 60B is provided with a flow-rate throttle valve 66 instead of the solenoid valve 62 in the valve unit 60 shown in Fig. 20. With the flow-rate throttle valve 66, the opening degree of the throttle can be changed arbitrarily depending on the voltage of a control signal from the control unit 45.

That is to say, the control unit 45 controls the voltage of the control signal so as to be changed to regulate the opening degree of the throttle of the flow-rate throttle valve 66, whereby the gas supplying flow rate of carbon dioxide gas to be supplied can be regulated.

The flow-rate throttle valve 66 has, as shown in Fig. 28, properties wherein if we say that a horizontal axis is the voltage value of the control signal, and a vertical axis is a flow rate value, the flow rate value when a voltage value is P1 is F1, and the flow rate value when a voltage value is P2 which is greater than the voltage value P 1 is F2 which is greater than the flow rate value F1.

With the present embodiment, as with the fifth embodiment, the opened/closed state of the gas supplying/water supplying button is detected by the detection unit 45A comparing the flow-rate measured value in the flow-rate sensor 63 and two thresholds VLL and VLH. Note that with the present embodiment, the two thresholds VLL and VLH are thresholds corresponding to the flow rate values F1 and F2 respectively, which satisfies a relation of VLH > VLL.

In this case, the threshold VLH is a threshold for determining that the gas supplying/water supplying button 25a is in an opened state, and the threshold VLL is a threshold for determining that the gas supplying/water supplying button 25a is in a closed state.

With the present embodiment, as for an operation state of the flow-rate throttle valve 66, there are two throttle operation states of a throttle operation state at the time of the flow-rate value F1 (hereafter, referred to as F1 state), and a throttle operation state at the time of the flow-rate value F2 (hereafter, referred to as F2 state).

Note that the two F1 and F2 states are not restricted to the flow rate values thereof, but rather can be set arbitrarily. Similarly, the two thresholds VLH and VLL can be set arbitrarily in the same way as the fifth embodiment.

The other configurations are generally the same as those in the above-mentioned embodiments.

Next, description will be made regarding the supply operation of carbon dioxide gas to the lumen by the gas supplying apparatus 31 provided in the surgery system 1 according to the present embodiment with reference to Fig. 27 and Fig. 29.

With the gas supplying apparatus 31 provided in the surgery system 1 according to the present embodiment, upon power being turned on, the control unit 45 activates a program shown in Fig. 27 stored in the unshown storing unit.

As shown in Fig. 27, the control unit 45 controls the flow-rate throttle valve 66 to open its throttle to supply carbon dioxide gas into the body cavity by the processing in step S49 such that the flow-rate throttle valve 66 turns into the F2 state.

In this case, the carbon dioxide gas from the gas supplying connector 31 c of the gas supplying apparatus 31 reaches the gas supplying/water supplying button cylinder 25c where the gas supplying/water supplying button 25a provided on the operation portion 25 is disposed via the gas supplying tube 33, the gas supplying channel (not shown) within the endoscope connector 26a, the gas supplying channel within the universal cord 26, and the upstream-side gas supplying channel 21a (see Fig. 7).

The control unit 45 controls the detection unit 45A to compare the flow-rate measured value serving as the detection result from the flow-rate sensor 63, and the thresholds VLH and VLL set beforehand (see Fig. 28 and Fig. 29) by the determination processing in step S50.

With the comparison result, in the event that the flow-rate measured value is greater than the threshold VLH, the control unit 45 detects that the carbon dioxide gas to be supplied into the body cavity is leaking from the gas supplying/water supplying button 25a of the endoscope 21. That is to say, the control unit 45 determines that the gas supplying/water supplying button 25a is in an opened state.

On the other hand, with the comparison result, in the event that the flow-rate measured value is smaller than the threshold VLL, the control unit 45 detects that the carbon dioxide gas to be supplied into the body cavity is not leaking from the gas supplying/water supplying button 25a of the endoscope 21. That is to say, the control unit 45 determines that the gas supplying/water supplying button 25a is in a closed state.

Thus, the opened/closed state of the gas supplying/water supplying button 25a is detected during the gas supplying operation of the gas supplying apparatus 31 by the determination processing in step S50.

In the event of determining by the determination processing in step S50 that the gas supplying/water supplying button 25a is in an opened state (state in which supply of gas is not performed), the control unit 45 proceeds to the processing in step S51. On the other hand, in the event of determining that the gas supplying/water supplying button 25a is in a closed state (state in which supply of gas is performed), the control unit 45 proceeds to the processing in step S52.

With the processing in step S51, the gas supplying/water supplying button 25a is in an opened state (leaked state), so the control unit 45 controls the flow-rate throttle valve 66 to turn into the F1 state to prevent carbon dioxide gas from being consumed wastefully, thereby decreasing the gas supplying flow rate. Subsequently, the control unit 45 returns to the processing in step S50.

On the other hand, with the processing in step S52, carbon dioxide gas is in a supplied state (the gas supplying/water supplying button 25a is in a closed state), so the control unit 45 controls the flow-rate throttle valve 66 so as to be turned into the F2 state to increase the gas supplying flow rate. Subsequently, the control unit 45 returns to the processing in step S50.

Fig. 29 illustrates a timing chart of the flow-rate measured value of the flow-rate sensor 63, the open/close operation of the gas supplying/water supplying button 25a, and the throttle operation of the flow-rate throttle valve 66, when actually performing such a control example.

As shown in Fig. 29, with the gas supplying apparatus 31, at and before point-in-time t0, the flow-rate throttle valve 66 is turned into the F2 state by the processing in step S49 under the control of the control unit 45, whereby carbon dioxide gas is supplied to the endoscope 21 via the gas supplying connector 31 c.

Let us say that the surgeon takes off his/her finger from the hole portion 25d of the gas supplying/water supplying button 25a to turn the gas supplying/water supplying button 25a to an open state at point-in-time t0. Thus, the gas supplying/water supplying button 25a turns into a leaked state, thereby increasing the gas supplying flow rate of the carbon dioxide gas.

Thus, at point-in-time t1, the control unit 45 performs the determination processing in step S50, whereby the detection unit 45A detects that the flow-rate measured value is greater than the threshold VLH. That is to say, the control unit 45 determines that the gas supplying/water supplying button 25a is in an opened state.

The control unit 45 controls the flow-rate throttle valve 66 so as to be turned into the F1 state by the processing in step S51 based on the detection result. Thus, the gas supplying flow rate of the carbon dioxide gas decreases at point-in-time t1.

The surgeon closes the hole portion 25d of the gas supplying/water supplying button 25a by his/her finger at point-in-time t2, i.e., turns the gas supplying/water supplying button 25a to a closed state. Thus, the gas supplying flow rate of the carbon dioxide gas decreases.

Subsequently, the control unit 45 performs the determination processing in step S50 at point-in-time t3, whereby the detection unit 45A detects that the flow-rate measured value is smaller than the threshold VLL. That is to say, the control unit 45 determines that the gas supplying/water supplying button 25a is in a closed state.

The control unit 45 controls the flow-rate throttle valve 66 so as to be turned into an F2 state by the processing in step S52 based on the detection result. Thus, the gas supplying flow rate of the carbon dioxide gas increases.

Therefore, according to the present embodiment, the gas supplying flow rate of the carbon dioxide gas is decreased when the surgeon takes off his/her finger from the hole portion 25d of the gas supplying/water supplying button 25a, whereby the consumption of the carbon dioxide gas can be reduced.

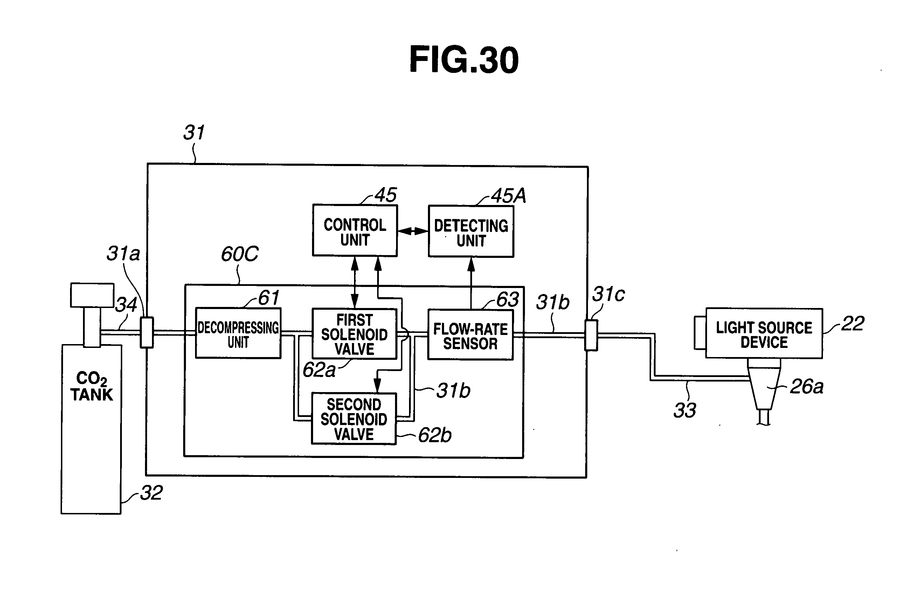

Note that the gas supplying apparatus 31 according to the present embodiment may be configured such as shown in a later-described first modification and second modification. Now, the first and second modifications of the gas supplying apparatus 31 according to the present embodiment will be described with reference to Fig. 30 through Fig. 34.

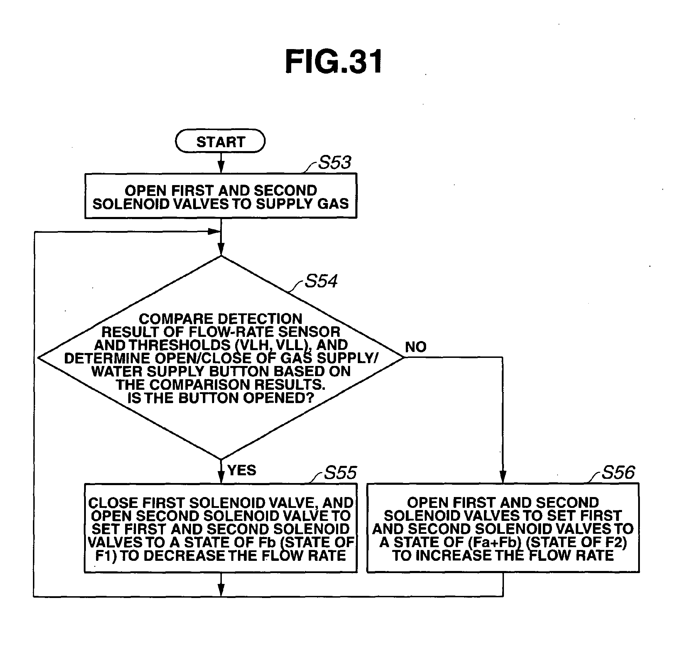

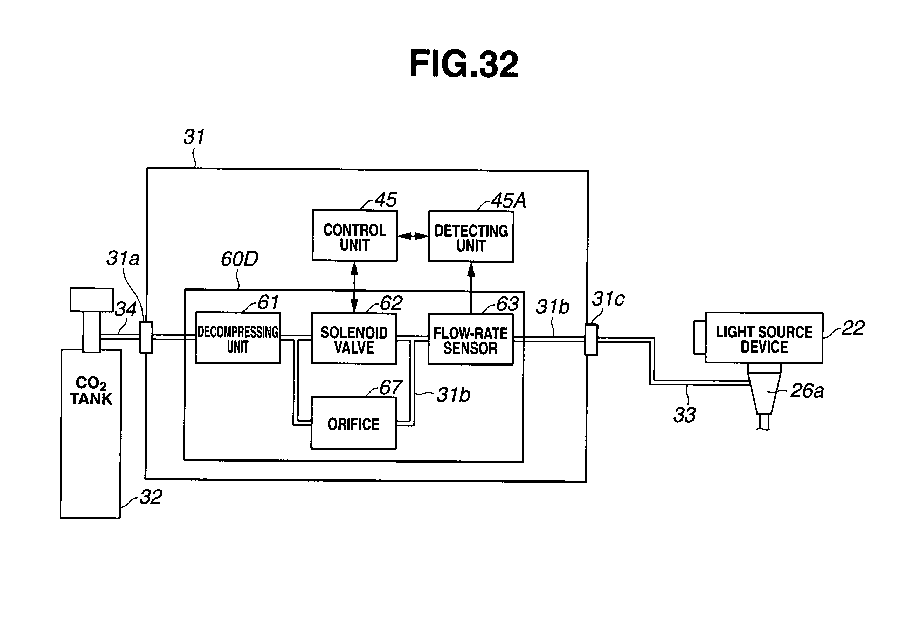

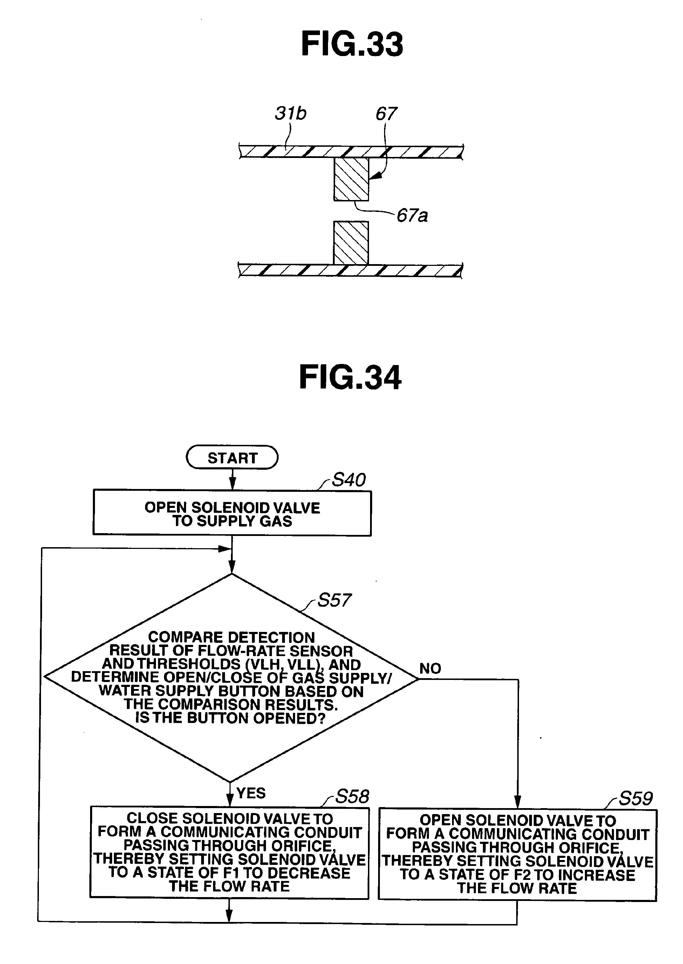

Fig. 30 and Fig. 31 are diagrams describing the first modification, wherein Fig. 30 is a block diagram describing a configuration example of a gas supplying apparatus of the first modification, Fig. 31 is a flowchart illustrating a control example of the gas supplying apparatus. Fig. 32 through Fig. 34 are diagrams describing the second modification, wherein Fig. 32 is a block diagram describing a configuration example of a gas supplying apparatus of the second modification, Fig. 33 is a cross-sectional view of an orifice provided in the gas supplying channel shown in Fig. 32, and Fig. 34 is a flowchart illustrating a control example of the gas supplying apparatus.

As shown in Fig. 30, the gas supplying apparatus 31 according to the first modification includes a valve unit 60C in which fist and second solenoid valves 62a and 62b are provided instead of the flow-rate throttle valve 66 according to the present embodiment.

With the valve unit 60C, the open/close operation of the first and second solenoid valves 62a and 62b are controlled based on the control signal outputted from the control unit 45 in the same way as the flow-rate throttle valve 66. Note that the second solenoid valve 62b is disposed in the gas supplying channel 31b so as to be in parallel with the first solenoid valve 62a.

The first solenoid valve 62a includes properties capable of supplying carbon dioxide gas with a great flow rate value Fa when turned to an open state, and the second solenoid valve 62b includes properties capable of supplying carbon dioxide gas with a small flow rate value Fb when turned to an open state.

That is to say, the present modification assumes that the above-mentioned F1 state in the flow-rate throttle valve 66 is equivalent to a state in which the gas supplying flow rate becomes the flow rate value Fb by closing the first solenoid valve 62a, and also opening the second solenoid valve 62b.

Also, the present modification assumes that the above-mentioned F2 state in the flow-rate throttle valve 66 is equivalent to a state in which the gas supplying flow rate becomes (flow rate value Fa + flow rate value Fb) by opening the first solenoid valve 62a, and the second solenoid valve 62b.

Thus, the same operation as that in the flow-rate throttle valve 66 according to the present embodiment can be obtained.

The gas supplying apparatus 31 according to the present modification operates in accordance with generally the same control method (program shown in Fig. 27) as that in the gas supplying apparatus according to the present embodiment, but as shown in Fig. 31, the processing contents in step S53, step S55, and step S56 differ.

That is to say, the control unit 45 according to the present modification controls the first and second solenoid valves 62a and 62b to open to supply carbon dioxide gas into the body cavity by the processing in step S29 such that the first and second solenoid valves 62a and 62b become the F2 state.

In step S54 wherein the control unit 45 performs the same determination processing as that in step S20 (see Fig. 27), the control unit 45 controls the detection unit 45A to compare the flow-rate measured value serving as the detection result from the flow-rate sensor 63, and the thresholds VLH and VLL set beforehand (see Fig. 28 and Fig. 29). Thus, the control unit 45 can detect the opened/closed state of the gas supplying/water supplying button 25a as with the present embodiment.

In the event of determining that the gas supplying/water supplying button 25a is in an opened state (leaking state) by the determination processing in step S54, the control unit 45 proceeds to the processing in step S55. On the other hand, in the event of determining that the gas supplying/water supplying button 25a is in a closed state (not leaked state), the control unit 45 proceeds to the processing in step S56.

With the processing in step S55, the gas supplying/water supplying button 25a is in an opened state (leaking state), so the control unit 45 controls the first solenoid valve 62a to close, and controls the second solenoid valve 62b to open such that the gas supplying flow rate turns into the F1 state serving as the small flow rate value Fb (F1 state of the flow-rate throttle valve 66 according to the present embodiment), thereby decreasing the gas supplying flow rate. Subsequently, the control unit 45 returns to the processing in step S54.

On the other hand, with the processing in step S56, carbon dioxide gas is in a supplied state (the gas supplying/water supplying button 25a is in a closed state), so the control unit 45 controls the first and second solenoid valves 62a and 62b to open such that the gas supplying flow rate is in a state of (flow rate value Fa + flow rate value Fb) (F2 state of the flow-rate throttle valve 66 according to the present embodiment) to increase the gas supplying flow rate. Subsequently, the control unit 45 returns to the processing in step S54. Thus, generally the same operations and advantages as those in the present embodiment can be obtained.

Therefore, according to the first modification, generally the same operations and advantages as those in the flow-rate throttle valve 66 can be obtained by employing the first and second solenoid valves 62a and 62b instead of the flow-rate throttle valve 66, whereby the gas supplying apparatus 31 which is cheaper than the gas supplying apparatus according to the present embodiment can be obtained.

The gas supplying apparatus 31 according to the second modification, as shown in Fig. 32, includes a valve unit 60D in which a solenoid valves 62 and an orifice 67 serving as a flow rate regulating member are provided instead of the flow-rate throttle valve 66 according to the present embodiment.

With the valve unit 60D, the solenoid valve 62 includes properties capable of supplying carbon dioxide gas with a great flow rate value Fa when turning to an open state as with the first modification. Also, the orifice 67 is provided in the gas supplying channel 31b so as to be in parallel with the solenoid valve 62.

The orifice 67 includes, for example as shown in Fig. 33, a small gas supplying hole 67a, thereby forming a communicative connection channel by providing a flow-rate regulating member configured to restrict the gas supplying flow rate by reducing the channel diameter of the gas supplying channel 31 b, and bypass the solenoid valve 62 within a predetermined place of the gas supplying channel 31 b. That is to say, the communicative connection channel of the gas supplying channel 31 b formed by providing the orifice 67 includes properties capable of supplying carbon dioxide gas with the small flow rate value Fb as with the first modification.

Accordingly, the gas flow rate becomes the flow rate value Fb only by closing the solenoid valve 62, which realizes the F1 state in the flow-rate throttle valve 66 according to the present embodiment.

Also, the gas flow rate becomes (flow rate value Fa + flow rate value Fb) only by opening the solenoid valve 62, which realizes the F2 state in the flow-rate throttle valve 66 according to the present embodiment.

Note that the communicative connection channel (gas supplying channel 31 b) formed by providing the orifice 67 is always communicatively connected to the gas supplying connector 31 c, so in the event that the gas supplying tube 33 is not connected with the gas supplying connector 31 c, carbon dioxide gas is leaking from the gas supplying connector 31 c into the atmosphere. However, with the second modification, an unshown check valve is provided in the gas supplying connector 31c, and in the event that the gas supplying connector 31 c is not connected to the gas supplying tube 33, the channel within the gas supplying connector 31c is shielded to prevent carbon dioxide gas from being leaked.

According to the above configuration, the same operation as that in the flow-rate throttle valve 66 according to the present embodiment can be obtained.

The gas supplying apparatus 31 according to the second modification operates in accordance with generally the same control method (program shown in Fig. 27) as that in the gas supplying apparatus according to the present embodiment, but as shown in Fig. 34, the processing contents in step S58 and step S59 differ.

That is to say, in the event of determining that the gas supplying/water supplying button 25a is in an opened state (leaking state) by the determination processing in step S57, the control unit 45 closes the solenoid valve 62 to prevent carbon dioxide gas from being consumed wastefully. Thus, the communicative connection channel passing through the orifice 67 is formed, and the control unit 45 controls the gas supplying flow rate so as to become the small flow rate value Fb state (F 1 state of the flow-rate throttle valve 66 according to the present embodiment) to decrease the gas supplying flow rate. Subsequently, the control unit 45 returns to the processing in step S57.

On the other hand, in the event of determining that the gas supplying/water supplying button 25a is in a closed state (not leaking state), the control unit 45 opens the solenoid valve 62 by the determination processing in step S59. Thus, the communicative connection channel passing through the orifice 67 is formed, and the control unit 45 controls the gas supplying flow rate so as to turn into the state of (flow-rate value Fa + flow-rate value Fb) (F2 state of the flow-rate throttle valve 66 according to the present embodiment) to increase the gas supplying flow rate. Subsequently, the control unit 45 returns to the processing in step S57. Thus, generally the same operations and advantages as those in the present embodiment can be obtained.

Therefore, according to the second modification, generally the same operations and advantages can be obtained by employing the solenoid valves 62 and orifice 67 instead of the flow-rate throttle valve 66, whereby the gas supplying apparatus 31 which is cheaper than the gas supplying apparatus according to the present embodiment can be obtained. Also, the second modification is more simple and cheaper than the above-mentioned first modification.

Note that the above-mentioned fourth through sixth embodiments can realize the control method of the gas supplying apparatus 31 capable of preventing the carbon dioxide gas serving as gas for examination stored within the gas tank 32 from being consumed wastefully, the gas supplying apparatus 31, and the surgery system 1 having the endoscope system 2 including the gas supplying apparatus 31.

### (Seventh Embodiment)

Next, description will be made regarding a seventh embodiment with reference to the drawings. Note that the same reference numerals are provided to the same components as those in the above embodiments, description thereof will be omitted, and only different configurations, operations, and advantages will be described.

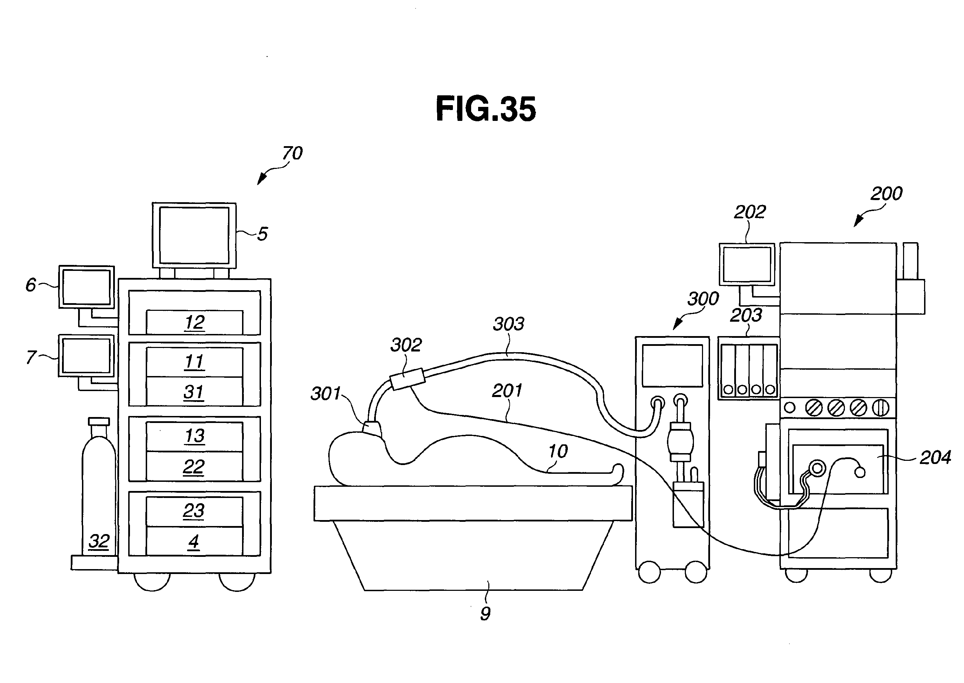

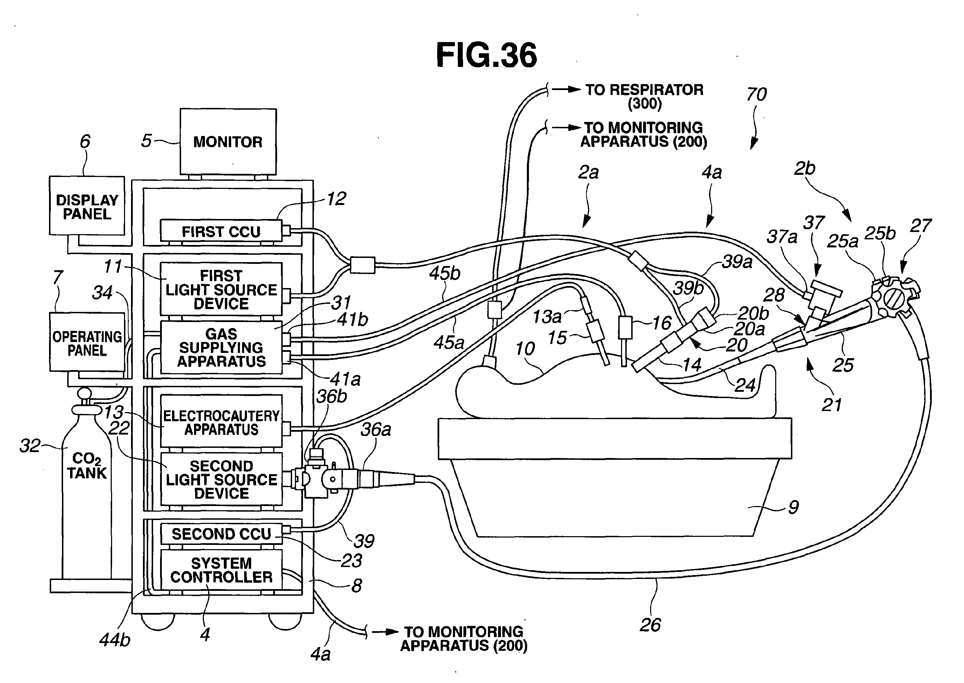

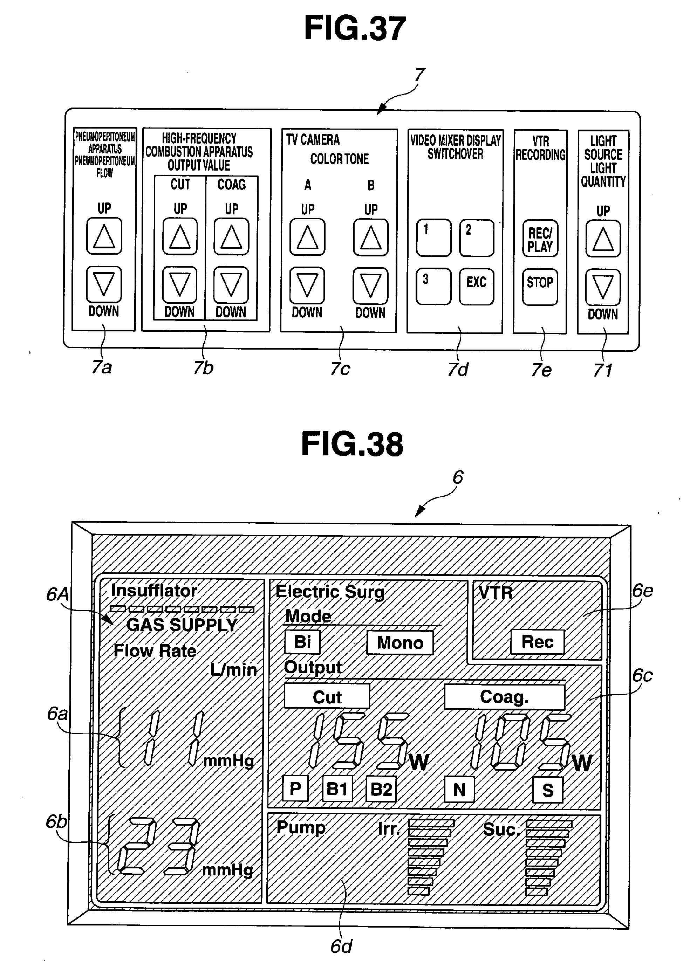

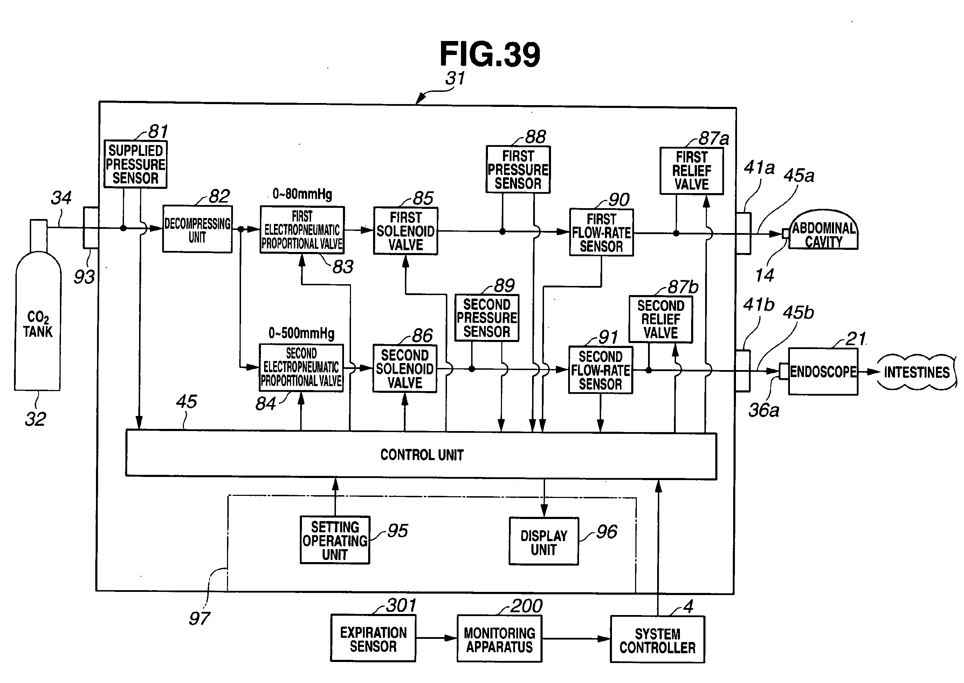

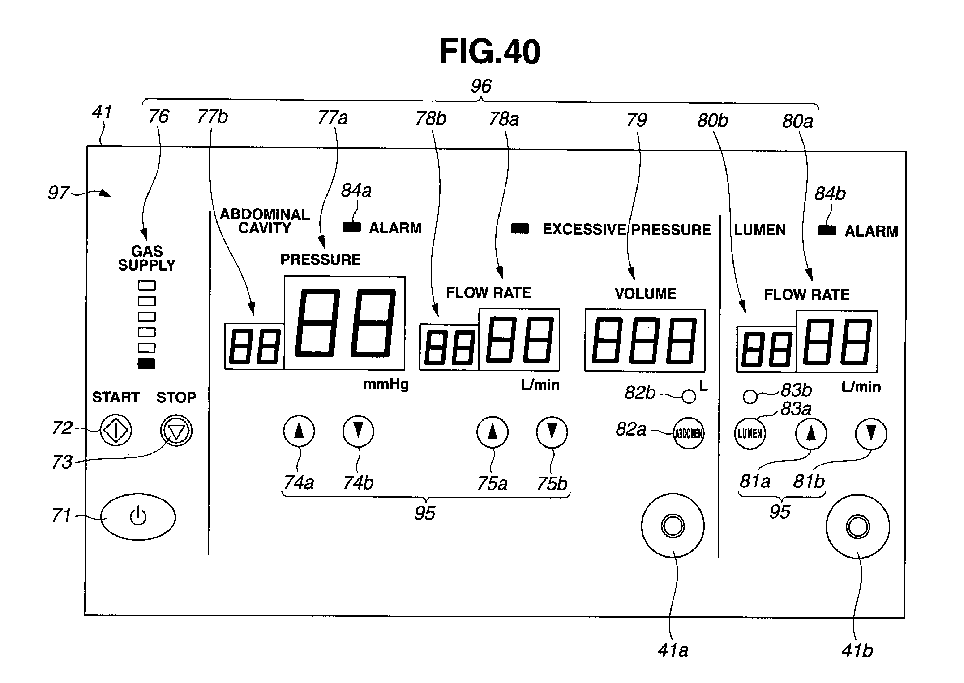

Fig. 35 through Fig. 40 relate to the seventh embodiment of the present invention, wherein Fig. 35 is a diagram illustrating a configuration of a laparoscope surgery system, showing a monitoring apparatus and a respirator serving as external apparatuses, Fig. 36 is a diagram describing the configuration of the laparoscope surgery system including a gas supplying system, Fig. 37 is a diagram for describing a central operating panel, Fig. 38 is a diagram for describing the central operating panel, Fig. 39 is a configuration diagram illustrating the internal configuration of a gas supplying apparatus, and Fig. 40 is a diagram for describing a panel portion of the gas supplying apparatus. The present embodiment will also use the same reference numerals as with the above-described embodiments, and description thereof will be omitted.

As shown in Fig. 35, with the laparoscope surgery system according to the present embodiment, a laparoscope surgery system 70, a monitoring apparatus 200 (also referred to as a patient monitoring apparatus) and a respirator (artificial respirator) 300 serving as external apparatuses are prepared.

A patient 10 on a surgical table 9 has the oral cavity covered with a breathing mask 301. A breathing hose 303, which is connected to the respirator 300 at one end, is connected to the breathing mask 301 at the other end. A breathing sensor 302 is introduced partway along the breathing hose 303.

The breathing sensor 302 is electrically connected to a signal cable 201 extending from the monitoring apparatus.

The monitoring apparatus 200 will be described in brief now.

The monitoring apparatus 200 is principally configured of a multi-parameter monitor 202 displaying living body information such as blood pressure, pulse, breathing state, and so forth of the patient 10, a vital sign measurement device 203 to which information is inputted from various types of sensors attached to the patient 10, and a control unit 204 for processing the various types of living body information.

The vital sign measurement device 203 is supplied with various types of signals by various types of sensors attached to the patient 10, and unshown cables. The vital sign measurement device 203 includes a capnometer, the capnometer being connected to the breathing sensor 302 via the signal cable 201.

With the present embodiment, the concentration of carbon dioxide breathed out by the patient 10 is measured by the breathing sensor 302 disposed to the breathing hose 303 of the respirator 303, and information signals regarding the carbon dioxide concentration are supplied to the control unit 204 of the monitoring apparatus 200. The control unit 204 calculates the terminal-expiratory carbon dioxide partial pressure based on the concentration of carbon dioxide breathed out by the patient 10, and displays the numerical values thereof on the multi-parameter monitor 202.

While described later, the control unit 204 of the monitoring apparatus 200 is electrically connected with the system controller 4 of the laparoscope surgery system 70.

The laparoscope surgery system 70 according to the present invention will now be described in detail.

As shown in Fig. 36, the laparoscope surgery system (hereafter abbreviated as surgery system) 1 according to the present embodiment is configured of a first endoscope system 2a, a second endoscope system 2b, and a gas supplying system 4a, and also has a system controller 4, a monitor 5 which is a display device, a central display panel (hereafter abbreviated as display panel) 6, a central operating panel (hereafter abbreviated as operating panel) 7, and a cart 8.

The electrocautery apparatus 13 is connected to an electric scalpel 13a which is a surgical instrument. The first trocar 14 is a trocar for guiding the later-described endoscope to within the abdominal cavity. The second trocar 15 is a trocar for guiding a treatment instrument, such as an electric scalpel 13a or the like, for performing excision or treatment of tissue, to within the abdominal cavity. The third trocar 16 us a trocar for guiding carbon dioxide gas the same as with the above-described embodiments for example, which is a pneumoperitoneum gas to be supplied from a later-described gas supplying apparatus 31 making up the supplying system 4a, to within the abdominal cavity. Note that an arrangement may be made wherein the carbon dioxide gas is supplied from the first trocar 14, or the second trocar 15, to within the abdominal cavity.

The first endoscope system 2a is principally configured of a rigid endoscope 20 of which the insertion portion is rigid for example, serving as a first endoscope, a first light source device 11, a first camera control unit (hereafter abbreviated as first CCU) 12, and an endoscope camera 20b.

The insertion portion (not shown) of the rigid endoscope 20 is inserted through the first trocar 14, and disposed within the abdominal cavity. Provided inside the insertion portion is an illumination optical system configured of an observation optical system made up of a relay lens (not shown) for transmitting subject images or the like, and light guide (not shown), and so forth. An eyepiece 20a for observing optical images transmitted by the observation optical system is provided at the proximal end portion of the insertion portion. An endoscope camera 20b is detachably disposed at the eyepiece 20a. An image-pickup device (not shown) is provided within the endoscope camera 20b.

The first light source device 11 supplies illumination light to the rigid endoscope 20. The first CCU 12 converts the electric signals obtained by photoelectric conversion by imaging on the image-pickup device of the endoscope camera 20b into video signals, and outputs the video signals to, for example, the monitor 5 and the central display panel 6. Thus, the endoscope image of the subject captured by the rigid endoscope 20 is displayed on the monitor 5 and the central display panel 6.

Note that the rigid endoscope 20 and the first light source device 11 are connected by a light guide cable 39b extending from the side of the rigid endoscope 20. The first CCU 12 and the endoscope camera 20b are connected by an image-pickup cable 37a.

The second endoscope system 2b is principally configured of a second endoscope 21 having a flexible insertion portion 24 for insertion to lumen such as the large intestine and so forth, a second light source device 22, and a second camera control unit (hereafter abbreviated as second CCU) 23.

The second endoscope 21 is configured including an insertion portion 24 and universal cord 26. Provided on the operation portion 25 are a gas supplying/water supplying button 25a, a suction button 25b, a bending operation knob 27 for subjecting an unshown bending portion to bending operation, and a treatment-instrument insertion opening 38 communicatively connected to an unshown treatment instrument channel. The proximal end portion of the universal cord 26 is provided with an endoscope connector 26a.

The second CCU 23 converts the electric signals obtained by photoelectric conversion by imaging on an image-pickup device provided at an unshown distal end of the insertion portion 24 of the second endoscope 21 into video signals, and outputs the video signals to, for example, the monitor 5, or, the central display panel 6. Thus, the endoscope image of the subject captured by the second endoscope 21 is displayed on the monitor 5 or the central display panel 6. Note that reference numeral 39 denotes an electric cable electrically connecting the electric connector 36b provided to the light source connector 36a with the second CCU 23.

The gas supplying system 4a is principally configured of the gas supplying apparatus 31, gas tank 32 which is the supply source serving as the carbon dioxide supply unit, the second light source 22, and the system controller 4. Carbon dioxide is stored in the gas tank 32 in a liquid state.

Provided to the gas supplying apparatus 31 are an abdominal cavity supplying base which is a first supply base (hereafter called first base) 41 a, and a lumen supplying base which is a second supply base (hereafter called second base) 41b. One end of an abdominal cavity tube 45a which is a first tube is connected to the first base 41a, with the other end of the abdominal cavity tube 45a being connected to the third trocar 16.

One end of a lumen tube 45b which is a second tube is connected to the second base 41b, with the other end of the lumen tube 45b being connected to the second endoscope 21. The other end of the lumen tube 45b is connected to a base 39a of a connector 37 connected to the treatment instrument channel opening of the second endoscope 21.

That is to say, the carbon dioxide gas from the gas supplying apparatus 31 is supplied into the lumen via the lumen tube 45b and connector 37, passing through the treatment instrument channel of the second endoscope 21. Note that the gas supplying apparatus 31 and the gas tank are connected by a high-pressure gas tube 34.

The second light source device 22 which is a second gas supplying apparatus supplies illumination light to the second endoscope 21. A light source connector 36a is detachably connected to the second light source device 22. Connecting the light source connector 36a to the second light source device 22 causes the illumination light to be transmitted through an unshown light guide fiber and emitted from a illumination window provided at the unshown distal end of the insertion portion 24.

Also, a compressor for example, serving as gas supplying means whereby predetermined pressure adjustment is made for supplying air into the body cavity of the patient 10 via the universal cord 26 and insertion portion 24 of the second endoscope, making up a gas supplying unit, is built into the second endoscope 22.

The system controller 4 centrally controls the entire surgery system 70. The system controller 4 is connected with the central display panel 6, central operating panel 7, the electrocautery apparatus 13 serving as an endoscope peripheral device, light source device 11, CCUs 12 and 23, gas supplying apparatus 31, and so forth, via an unshown communication line, so as to be capable of two-way communication.

An endoscope image of a subject captured by the rigid endoscope 20 or second endoscope 21 is arranged to be displayed on the screen of the monitor 5 in response to the video signal outputted from the first CCU 12 or the second CCU 23.

A display screen such as a liquid crystal display or the like is provided on the central display panel 6. The central display panel 6 is connected to the system controller 4, whereby an operation state of the endoscope peripheral device can be displayed on the display screen in a central manner as well as the above endoscope image of the subject.

The central operating panel 7 comprises a display portion such as a liquid crystal display or the like, and a touch sensor portion integrally provided on the display screen of the display portion. The display portion of the central operating panel 7 includes a display function for displaying the operating switches and so forth of the endoscope peripheral device as a setting screen, and an operation function for operating an operating switch displayed by touching a predetermined area of the touch sensor portion.

The central operating panel 7 is connected to the system controller 4, and appropriately operating the touch sensor displayed on the display portion enables various types of operations, settings, and so forth, to be made remotely at the central operating panel 7, in the same way as with directly operating the operating switches provided to each of the endoscope peripheral devices.

The electrocautery apparatus 13, the light source devices 11 and 22, CCUs 12 and 23, gas supplying apparatus 31, system controller 4, central display panel 6, central operating panel 7, gas tank 32, which are peripheral devices, and so forth are mounted on the cart 8.

Now, a configuration example of the central operating panel 7 will be described based on Fig. 37.

As shown in Fig. 37, provided to the central operating panel 7 are a setting operating button 9a for adjusting the pneumoperitoneum flow by the gas supplying apparatus 31 for the abdominal cavity or for the lumen, an operating button 9b for adjusting the output value of the electric scalpel device (high-frequency cauterization device) 12, an operating button 9c for adjusting the color tone for the CCUs (TV cameras) 23 and 33, an operating button 9d for instructing switching display of video information displayed on the monitor 5, an operating button 9e for instructing recording, or stopping of recording, by VCR, and an operating button 9f for adjusting the light quantity of the first light source device 11 and second light source device 22.

Next, an example of the display screen of the display panel 6 will be described based on Fig. 38.

As shown in Fig. 4, settings and operating states relating to the functions of the gas supplying apparatus 31, electric scalpel device 13, which are devices controlled by communication by the system controller 4, are displayed on the display screen of the display panel 6 for example, as respective display areas 6A (6a, 6b), 6c, 6d, and 6e. Note that the display area 6A displays the setting and operating state relating to the gas supplying apparatus 31, displaying a lumen pressure display 6a, abdominal cavity pressure display 6b, remaining carbon dioxide gas amount display, flow rate display, and so forth.

Next, the configuration of the gas supplying apparatus 31 will be described based on Fig. 39.

As shown in Fig. 39, provided within the gas supplying apparatus 31 are mainly a supplied pressure sensor 81, a sensor 82, a first electropneumatic proportional valve 83, a second electropneumatic proportional valve 84, a first solenoid valve 85, a second solenoid valve 86, a first relief valve 87a which is a first pressure adjusting unit serving as first pressure adjusting means, a second relief valve 87b which is a second pressure adjusting unit serving as second pressure adjusting means, a first pressure sensor 88 which is a first detecting unit serving as first detecting means, a second pressure sensor 89 which is a second detecting unit serving as second detecting means, a first flow-rate sensor 90, a second flow-rate sensor 91, and a control unit 45. Also provided to the gas supplying apparatus 31 in addition to the bases 41a and 41 b are a high-pressure base 93, setting operating unit 95, and display unit 96. Note that the first electropneumatic proportional valve 83 and the first solenoid valve 85 which is first open/close means make up a first gas supplying unit which is first gas supplying means, and the second electropneumatic proportional valve 84 and the second solenoid valve 86 make up a second gas supplying unit which is second gas supplying means.

The output side of the sensor 82 to which the carbon dioxide gas is inputted via the high-pressure base 93 branches into two, with one being an abdominal flow channel which is a first channel configured by the first electropneumatic proportional valve 83, first solenoid valve 85, first pressure sensor 88, first flow-rate sensor 90, first base 41a, and abdominal cavity tube 45a being connected serially in that order, and the other being a lumen flow channel which is a second channel configured by the second electropneumatic proportional valve 84, second solenoid valve 86, second pressure sensor 89, second flow-rate sensor 91, second base 41b, and lumen tube 45b being connected serially in that order.

The high-pressure gas tube 34 is connected to the high-pressure base 93. The high-pressure gas tube 34 is connected to a carbon dioxide gas tank (hereafter abbreviated as gas tank) 42 provided externally from the gas supplying apparatus 31.

The setting operating unit 95 and display unit 96 make up a panel portion 97. The supplied pressure sensor 81 measures the pressure of the carbon dioxide gas supplied from the gas tank 32, and also outputs the measurement results thereof to the control unit 45. The sensor 82 decompresses the carbon dioxide gas, supplied to within the gas supplying apparatus 31 in a gaseous state via the high-pressure base 93, to a predetermined pressure.

The first electropneumatic proportional valve 83 adjusts the supplied gas pressure of the carbon dioxide, decompressed by the sensor 82, to a range between 0 to 80 mmHg, which is around a first pressure, based on control signals outputted from the control unit 45. On the other hand, the second electropneumatic proportional valve 84 adjusts the supplied gas pressure of the carbon dioxide, decompressed by the sensor 82, to around a range between 0 to 500 mmHg, which is a second pressure, based on control signals outputted from the control unit 45.

The first solenoid valve 85 and second solenoid valve 86 are operated so as to open and close, based on control signals outputted from the control unit 45. The first pressure sensor 88 measures the pressure within the abdominal flow channel at the output side of the first electropneumatic proportional valve 83, and outputs the measurement results thereof to the control unit 45. Based on the measurement results from the first pressure sensor 88, the control unit 45 calculates the pressure value within the abdominal cavity.

Also, the second pressure sensor 89 measures the pressure within the lumen flow channel at the output side of the second electropneumatic proportional valve 84, and outputs the measurement results thereof to the control unit 45. Based on the measurement results from the lumen pressure sensor 89, the control unit 45 calculates the pressure value within the lumen.

The first flow-rate sensor 90 and the second flow-rate sensor 91 measure the amount of carbon dioxide gas being supplied to the bases 41a and 41 b, and output the measurement results to the control unit 45.

That is to say, the carbon dioxide gas supplied from the gas tank is decompressed at the sensor 82, and then is supplied into the abdominal cavity via the abdominal cavity flow channel and into the lumen via the lumen flow channel, based on control signals outputted from the control unit 45.

Note that in the event that the measurement value at the first pressure sensor 88 exceeds the setting value for abdominal cavity pressure, the first relief valve 87a provided at the output side of the first flow-rate sensor 90 is set to an open state, based on control signals from the control unit 45. That is to say, opening the first relief valve 87a discharges the carbon dioxide gas into the atmosphere, and the abdominal cavity pressure is adjusted by decompression.

Also, in the event that the measurement value at the second pressure sensor 89 exceeds the setting value for lumen pressure, the second relief valve 87a provided at the output side of the second flow-rate sensor 91 is set to an open state, based on control signals from the control unit 45. Thus, lumen pressure is adjusted by decompression.

Further, the control unit 45 is connected to an external system controller 4, and supplies various types of detection information signals, various types of control signals, and so forth, to the system controller 4. Also note that the system controller 4 is supplied with information signals of terminal-expiratory carbon dioxide partial pressure calculated from the monitoring apparatus 200, based on the amount of inclusion of terminal-expiratory carbon dioxide gas discharged from the patent 10 which is supplied form the breathing sensor 301.

Next, the panel portion 97 of the gas supplying apparatus 31 will be described based on Fig. 40.

As shown in Fig. 6, a panel portion 97 having a setting operating unit 95 and display unit 96 is provided to one side of the gas supplying apparatus 31.

Provided on the panel portion 97 are a power source switch 71; a gas supply start button 72; a gas supply stop button 73; abdominal cavity pressure setting buttons 74a and 74b, abdominal cavity side gas supply flow-rate setting buttons 75a and 75b, and lumen side gas supply flow-rate setting buttons 91a and 81 b, which make up a setting operating portion 95; an abdominal cavity mode switchover switch 82a; a lumen mode switchover switch 83a; and a remaining gas display portion 76, abdominal cavity pressure display units 77a and 77b, abdominal cavity side flow-rate display units 78a and 78b, a total gas supply volume display unit 79, lumen side flow-rate display units 80a and 80b, an abdominal cavity mode display unit 82b, and a lumen mode display unit 83b, making up a display unit 96; and so forth.

Also, in the event that there is an abnormality in supply of carbon dioxide gas into the abdominal cavity, a first alarm display unit 84a which is a first alarm unit serving as first alarm notification means is lit by the control unit 45. In the event that there is an abnormality in supply of carbon dioxide gas into the lumen, a second alarm display unit 84b which is a second alarm unit serving as second alarm notification means is lit by the control unit 45. Note that the control unit sounds an unshown warning buzzer as well as lighting the alarm display units 84a and 84b.

The power switch 71 is a switch for switching the main power source of the gas supplying apparatus 31 between an on state and an off state. The gas supply start button 72 is a button for instructing starting of supply of carbon dioxide gas to the abdominal cavity side. The gas supply stop button 73 is a button for instructing stopping of supply of carbon dioxide gas to the abdominal cavity side.

Button operations of the abdominal cavity pressure setting button 74a and gas supply flow-rate setting buttons 75a and 81a enable the setting values to be gradually changed in the higher direction. On the other hand, button operations of the abdominal cavity pressure setting button 74b and gas supply flow-rate setting buttons 75b and 81 b enable the setting values to be gradually changed in the lower direction.

The remaining gas display portion 76 displays the remaining amount of carbon dioxide gas within the gas tank. The abdominal cavity pressure display unit 77a displays the measurement results of abdominal cavity pressure measured by the first pressure sensor 88. On the other hand, the abdominal cavity pressure display unit 77b displays the setting pressure set by button operation of the abdominal cavity pressure setting buttons 74a and 74b.

The abdominal cavity side flow-rate display unit 78a displays the measurement results measured by the first flow-rate sensor 90. On the other hand, the abdominal cavity side flow-rate display unit 78b displays the set flow-rate set by button operations of the abdominal cavity side gas supply flow-rate setting buttons 75a and 75b. The total gas supply volume display unit 79 displays the total gas supply volume obtained by computations made at the control unit 45, based on the measurement values of the first flow-rate sensor 90.

The lumen side flow-rate display unit 80a displays the measurement results measured by the second flow-rate sensor 91. On the other hand, the lumen side flow-rate display unit 80b displays the set flow-rate set by button operations of the lumen side gas supply flow-rate setting buttons 91 a and 81 b.

The abdominal cavity mode switchover switch 82a instructs supplying of carbon dioxide gas to the first base 41a, and the lumen mode switchover switch 83a instructs supplying of carbon dioxide gas to the second base 41 b. Upon the abdominal cavity mode switchover switch 82a being operated, the abdominal cavity mode display unit 82b is lit, and simultaneously with selection operation of the abdominal cavity mode, the lumen mode display unit 83b is turned off.

In the same way, upon the lumen mode switchover switch 83a being operated, the lumen mode display unit 83b is lit, and simultaneously with selection operation of the lumen mode, the abdominal cavity mode display unit 82b is turned off. Note that the settings of the abdominal cavity pressure, settings for the supply gas flow-rate at the abdominal cavity side and lumen side, and so forth, can also be made from the central operating panel 7.

Also, an arrangement may be made wherein the central display panel 6 can display one or multiple values specified by the operator beforehand, from the values displayed at the abdominal cavity pressure display units 77a and 77b, abdominal cavity side flow-rate display units 78a, 78b, 80a, and 80b and total gas supply volume display unit 79.

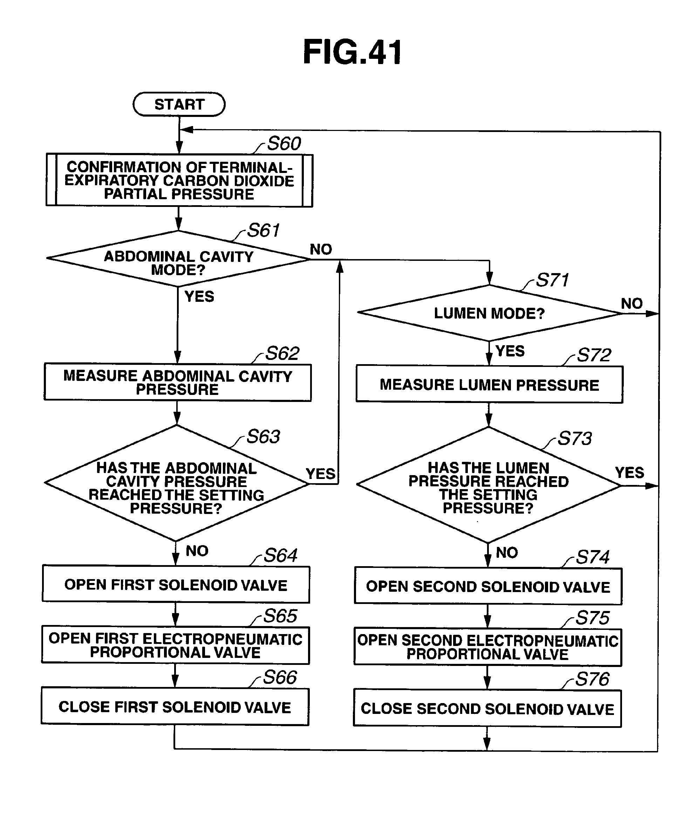

The operations of the surgery system 70 configured as described above will be described based on Fig. 41 through Fig. 45.

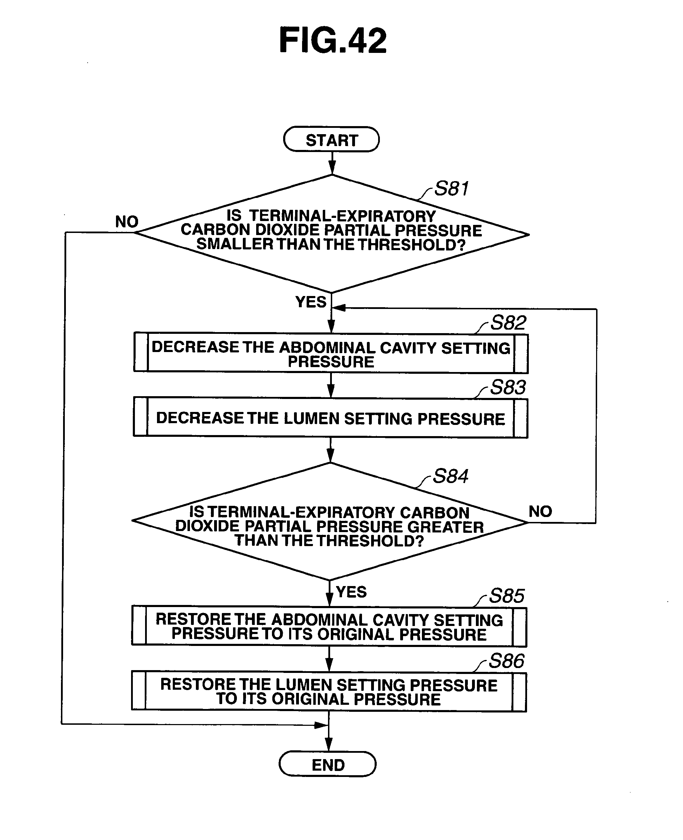

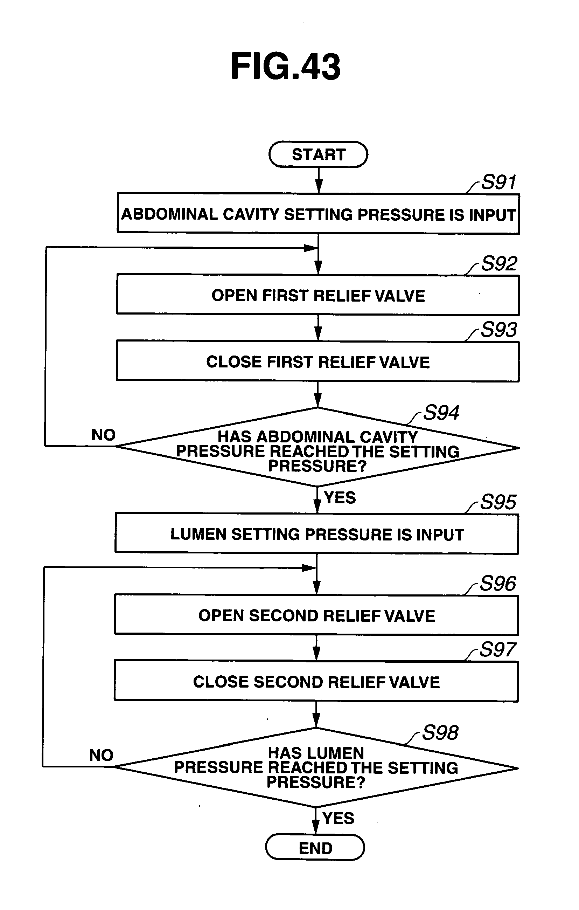

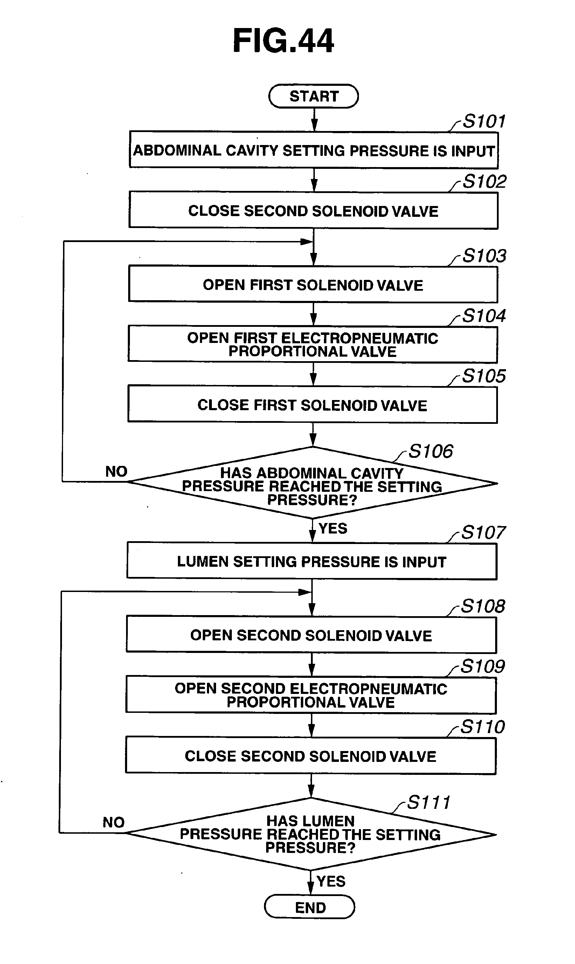

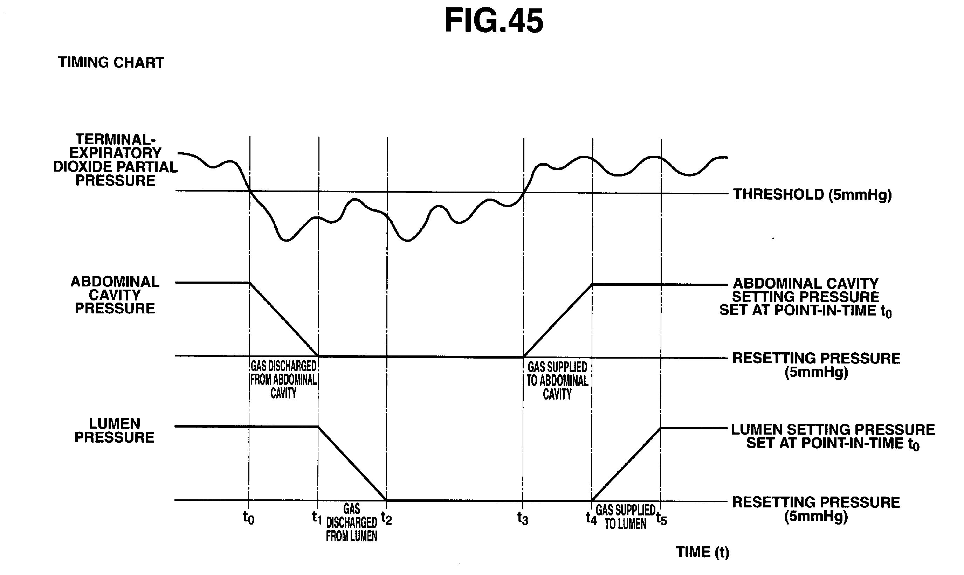

Fig. 41 is a flowchart for describing a control example of the gas supplying apparatus supplying gas to an abdominal cavity and a lumen, Fig. 42 is a flowchart for describing a control example of confirmation of terminal-expiratory carbon dioxide partial pressure, Fig. 43 is a flowchart for describing a control example of an operation for decreasing the setting pressure of an abdominal cavity and a lumen, Fig. 44 is a flowchart for describing a control example of an operation for restoring the setting pressure of an abdominal cavity and a lumen to the original setting pressure, and Fig. 45 is a timing chart illustrating the relation of terminal-expiratory carbon dioxide partial pressure, abdominal cavity pressure, and lumen pressure.

First, operations which the gas supplying apparatus 31 normally performs in the surgery system 70 will be described.

First, a doctor or a nurse turns the power source switch 71 of the gas supplying apparatus 31 shown in Fig. 40 on, and operates the abdominal cavity pressure setting buttons 74a and 74b, gas supply flow-rate setting buttons 75a and 81a, and gas supply flow-rate setting buttons 75b and 81 b, so as to set the internal pressure of the abdominal cavity and lumen, and the supply flow-rate of carbon dioxide gas to be supplied to the abdominal cavity and lumen.

The pressure within the abdominal cavity is set by a doctor or nurse to, for example, 8 to 15 mmHg. On the other hand, the pressure within the lumen is set by the gas supplying apparatus 31 to, for example, 10 mmHg, in accordance with the set flow-rate and abdominal cavity pressure that has been set by button operations of the lumen side gas supply flow-rate setting buttons 81a and 81b.

Upon the power switch 71 of the gas supplying apparatus 31 being turned on, the control unit 45 performs control based on the procedures of each step S (S) shown in the flowchart in Fig. 7. At this time, a first solenoid valve 85 and a second solenoid valve 86 are in a closed state.

Also, as shown in Fig. 41, upon the power switch 71 of the gas supplying apparatus 31 being turned on, the control unit 45 performs confirmation of terminal-expiratory carbon dioxide partial pressure (S60), and determines whether or not in the abdominal cavity mode (S61). Processing for the confirmation of the terminal-expiratory carbon dioxide partial pressure will be made by the control unit 45, based on the flowchart in Fig. 42. The detailed description thereof will be made later.

First, the operations of the gas supplying apparatus 31 in the abdominal cavity mode will be described.

The control unit 45, as described above, determines whether or not in the abdominal cavity mode (S61). Note that in the abdominal cavity mode, with the flow-rate control of carbon dioxide gas supplied into the abdominal cavity, a state wherein carbon dioxide gas flows and a state wherein the flow of carbon dioxide gas is shut off are repeated.

Specifically, first, the control unit 45 detects the actual pressure within the abdominal cavity by the first sensor 88 (S62) and displays the abdominal cavity pressure at the abdominal cavity pressure display unit 77a. At the same time, the gas supply pressure of the first electropneumatic proportional valve 83 is determined in accordance with the difference between the setting value displayed at the abdominal cavity pressure display unit 77b and the abdominal cavity pressure.

At this time, the control unit 45 is supplied with measurement results measured by the supplied pressure sensor 81 and the first flow-rate sensor 90, and determination is made regarding whether or not the abdominal cavity pressure has reached the setting value (S63). The remaining gas amount is displayed at the remaining gas display portion 76, the abdominal cavity pressure is displayed at the abdominal cavity pressure display unit 77a, the flow-rate is displayed at the abdominal cavity side flow-rate display unit 78a, and the total volume of supplied gas obtained by computation is displayed at the total gas supply volume display unit 79.

In the event of determining that the pressure within the abdominal cavity has not reached the set pressure, the control unit 45 opens the first solenoid valve 85 (S64), opens the first electropneumatic proportional valve 83 (S65), closes the first solenoid valve 85 after a predetermined amount of time elapsing (S66), and goes to step S10 again. The gas supplying apparatus 31 repeats the above control operation until the set abdominal cavity pressure is attained.

Thus, the carbon dioxide gas supplied from the gas tank 32 to within the gas supplying apparatus 31 is subjected to predetermined decompression by the sensor 82 and first electropneumatic proportional valve 83, and also predetermined flow-rate adjustment is performed, and passes through the first solenoid valve 85 and is supplied into the abdominal cavity via the first base 41a, abdominal cavity tube 45a, and third trocar 16.

Also, as described above, the pressure within the abdominal cavity is set to, for example, 8 to 15 mmHg, by a doctor or nurse. With the present embodiment, the pressure within the abdominal cavity that has been set by the doctor or nurse is, for example, 10 mmHg. Also, the pressure within the abdominal cavity of the patient at the point of starting the surgery is a pressure smaller than the set pressure (10 mmHg), i.e., generally the same as the atmospheric pressure. Accordingly, at the time of starting surgery normally, the gas supplying apparatus 31 determines that the pressure within the abdominal cavity has not reached the set pressure (10 mmHg).

In step S 13, in the event that the control unit 45 determines that the pressure within the abdominal cavity has reached the set pressure (10 mmHg), the flow advances to step S71 for determination regarding whether or not in the lumen mode.

That is to say, with control of the abdominal cavity pressure, a state wherein carbon dioxide gas flows and a state wherein the flow of carbon dioxide gas is shut off are repeated as long as determination is made that in the abdominal cavity mode. Upon the abdominal cavity pressure attaining the predetermined value around the setting value displayed at the abdominal cavity pressure display unit 77b, supply of gas to the abdominal cavity is placed in a stopped state.

Thus, space is formed within the abdominal cavity by a predetermined pressure, and the surgeon can perform treatment or the like with the electric scalpel 13a inserted into the abdominal cavity via the second trocar 15 while observing the portion to be treated with the rigid endoscope 20 placed in the first trocar 14. Note that in the event that the measurement results from the first pressure sensor 88 inputted to the control unit 45 are higher than the setting value displayed at the abdominal cavity pressure display unit 77b, the control unit 45 outputs a control signal to the first relief valve 87a. Thus, the first relief valve 87a is placed in an opened state, whereby the carbon dioxide gas within the abdominal cavity is discharged into the atmosphere, and the abdominal cavity pressure is reduced.

Next, the operations of the gas supplying apparatus 31 in the lumen mode will be described.

In the event that determination is made in step S61 that not in the abdominal cavity mode, or in the event that determination is made in step S63 that the abdominal cavity pressure has reached the set pressure, whether or not in the lumen mode is determined (S71). In the event that determination is made in step S71 that it is not in the lumen mode, the flow goes to step S60 again.

On the other hand, in the event of determination that it is in the lumen mode, the control unit 45 detects the actual pressure within the lumen with the second pressure sensor 89 (S72), and determines the supply gas pressure of the second electropneumatic proportional valve 84 in accordance with the set flow-rate and abdominal cavity pressure set by button operations of the lumen side gas supply flow-rate setting buttons 81 a and 81 b.

At this time, the control unit 45 is supplied with the measurement results measured by the supplied pressure sensor 81 and the second flow-rate sensor 91, and determination is made regarding whether or not the lumen pressure has reached the set value (S73). In the same way as with the abdominal cavity mode, the remaining gas amount is displayed at the remaining gas display portion 76, the flow-rate is displayed at the lumen side flow-rate display unit 80a, and the total volume of supplied gas obtained by computation is displayed at the total gas supply volume display unit 79.

In the event of determining that the pressure within the lumen has not reached the set pressure, the control unit 45 opens the second solenoid valve 86 (S74), opens the second electropneumatic proportional valve 84 (S75), closes the second solenoid valve 86 after a predetermined amount of time elapsing (S76), and goes to step S60 again. The gas supplying apparatus 31 repeats the control operation from step S74 to step S76 until the set lumen pressure is attained.

Also, as described above, the pressure within the lumen is set to, for example, 10 mmHg, by a doctor or nurse, in accordance with the set flow-rate and abdominal pressure set by button operations of the lumen side gas supply flow-rate setting buttons 81 a and 81 b. Also, the pressure within the lumen of the patient at the point of starting the surgery is a pressure smaller than the set pressure (10 mmHg), i.e., generally the same as the atmospheric pressure. Accordingly, at the time of starting surgery normally, the gas supplying apparatus 31 determines that the pressure within the lumen has not reached the set pressure (10 mmHg).

Thus, the carbon dioxide gas supplied from the gas tank 32 to within the gas supplying apparatus 31 is subjected to predetermined decompression by the sensor 82 and second electropneumatic proportional valve 84, and also predetermined flow-rate adjustment is performed, and passes through the second solenoid valve 86 and is supplied into the abdominal cavity via the second base 41b, lumen tube 45b, and second endoscope 21.

In step S73, in the event that the control unit 45 determines that the pressure within the lumen has reached the set pressure (10 mmHg), the flow goes to step S60.

That is to say, with control of the lumen pressure, as with the abdominal cavity mode, a state wherein carbon dioxide gas flows and a state wherein the flow of carbon dioxide gas is shut off are repeated as long as determination is made that it is in the lumen mode. Upon the lumen pressure attaining the predetermined value around the setting value (10 mmHg), supply of gas to the lumen is placed in a stopped state.

Accordingly, when in the lumen mode, the carbon dioxide gas supplied to within the gas supplying apparatus 31 from the gas tank 2 via the high-pressure gas tube 34 is decompressed to a predetermined pressure at the sensor 82 and second electropneumatic proportional valve 84, and is supplied into the lumen at a predetermined flow-rate through the second solenoid valve 86 and via the second flow-rate sensor 91, second base 41b, lumen tube 45b, and second endoscope 21.

In the state of supplying gas to the lumen, the measurement results measured by the supplied pressure sensor 81 and the second flow-rate sensor 91 are inputted to the control unit 45. Accordingly, the remaining gas amount is displayed at the remaining gas display portion 76, the flow-rate is displayed at the lumen side flow-rate display unit 80a, and the total volume of supplied gas obtained by computation is displayed at the total gas supply volume display unit 79.

While carbon dioxide gas is being supplied into the lumen, the first pressure sensor 88 and second pressure sensor 89 constantly detect pressure within the abdominal cavity and the lumen and monitor the same by the control unit 45. Now, in the event that the pressure within the abdominal cavity has risen to a value higher than the set value while supplying gas to the lumen, the control unit 45 closes the second solenoid valve 86 and the second electropneumatic proportional valve 84 and stops the supplying of gas to the lumen, and places the second relief valve 87b in an opened state. Thus, the second relief valve 87b is placed in an open state, and the carbon dioxide gas within the lumen is discharged into the atmosphere, and the lumen pressure is reduced to around the set value.

Next, the operations performed by the gas supplying apparatus 31 at the time of performing the confirmation of terminal-expiratory carbon dioxide partial pressure in step S10 shown in Fig. 41 will be described with the flowcharts in Fig. 42 through Fig. 44, and the timing chart in Fig. 45 illustrating the values of terminal-expiratory carbon dioxide partial pressure, abdominal cavity pressure, and lumen pressure.

First, the calculation value of terminal-expiratory carbon dioxide partial pressure from the monitoring apparatus 200 is inputted via the system controller 4. The control unit 45 then makes determination regarding whether or not the pressure value of the terminal-expiratory carbon dioxide partial pressure is smaller than the threshold value (S81), as shown in Fig. 42.

Note that the threshold value of the terminal-expiratory carbon dioxide partial pressure of the patient 10 in the present embodiment is a value wherein the terminal-expiratory carbon dioxide partial pressure is, for example, 5 mmHg.

In the event that determination is made in step S81 that the value of the terminal-expiratory carbon dioxide partial pressure is 5 mmHg, which is the threshold value, or higher, the control unit 45 ends confirmation of the terminal-expiratory carbon dioxide partial pressure, and goes to step S61 in Fig. 41.

On the other hand, in the event that determination is made in step S81 that the value of the terminal-expiratory carbon dioxide partial pressure is smaller than the threshold value which is 5 mmHg, the control unit 45 lowers the abdominal cavity pressure (S82), and lowers the lumen pressure (S83). After passage of a predetermined amount of time, the control unit 45 performs determination regarding whether or not the value of the terminal-expiratory carbon dioxide partial pressure inputted from the system controller 4 is greater than the threshold value (S84).

For example, with the present embodiment, the abdominal cavity setting pressure is reset to a value (5 mmHg) which is half of the set pressure (10 mmHg) value in step S82. Note that in the event that the terminal-expiratory carbon dioxide partial pressure does not reach 5 mmHg or higher after elapsing of a predetermined amount of time even though the pressure value within the abdominal cavity has been reset (5 mmHg), the abdominal cavity setting pressure is further re-reset so as to be a value half (2.5 mmHg) of the reset setting pressure value (5 mmHg).

In the same way, the lumen setting pressure is reset to a value (5 mmHg) which is half of the set pressure (10 mmHg) value in step S83. Note that in the event that the terminal-expiratory carbon dioxide partial pressure does not reach 5 mmHg or higher after elapsing of a predetermined amount of time even though the pressure value within the lumen has been reset (5 mmHg), the lumen setting pressure is further re-reset so as to be a value half (2.5 mmHg) of the reset setting pressure value (5 mmHg).

In the event that determination is made that the value of the terminal-expiratory carbon dioxide partial pressure inputted from the system controller 4 is greater than the threshold value (5 mmHg), the control unit 45 returns the abdominal cavity setting pressure to the initially-set value, i.e., the value set by the surgeon (10 mmHg) (S85). The lumen setting pressure value is returned to the initial value (10 mmHg) due to the relation between the setting flow-rate and abdominal cavity pressure set by the surgeon (S86), confirmation of the terminal-expiratory carbon dioxide partial pressure is ended, and the flow goes to step S61 show in Fig. 41.

Specific operations of the gas supplying apparatus 31 will be described below with reference to Fig. 43 through Fig. 45.

Note that in the following description, the operation for lowering the abdominal cavity setting pressure in step S82 shown in Fig. 42 corresponds to the operations of step S91 through step S94 in Fig. 43. Also, the operation for lowering the lumen setting pressure in step S83 shown in Fig. 42 corresponds to the operations of step S95 through step S98 in Fig. 43. Further, the operation for restoring the abdominal cavity setting pressure in step S85 shown in Fig. 42 corresponds to the operations of step S101 through step S106 in Fig. 44. The operation for restoring the lumen setting pressure in step S86 shown in Fig. 42 corresponds to the operations of step S107 through step S111 in Fig. 44.

For example, let us say that at point-in-time t0 in Fig. 45, the value of terminal-expiratory carbon dioxide partial pressure calculated by the monitoring apparatus 200 based on the terminal-expiratory carbon dioxide gas concentration of the patient 10 detected by the breathing sensor 301, drops below the threshold value (5 mmHg). At this time, the control unit 45 changes the abdominal cavity setting pressure (S91), opens the first relief valve 87a (S92), and closes the first relief valve 87a after a predetermined amount of time elapsing (S93), as shown in Fig. 43. The value of the abdominal cavity setting pressure to be changed is a value half (5 mmHg) of the abdominal cavity setting pressure before change (10 mmHg), as described above.

The control unit 45 then determines whether or not the abdominal cavity pressure has reached the reset pressure, based on the measurement results measured by the first pressure sensor 88 and first flow-rate sensor 90 (S94). That is to say, the first relief valve 87a repeats opening and closing operations until the abdominal cavity pressure achieves the reset pressure (5 mmHg). Accordingly, the carbon dioxide gas supplied within the abdominal cavity is externally discharged when the first relief valve 87a is on an opened state. That is to say, in the event that the abdominal cavity pressure has not reached the reset pressure in step S94, the control unit 45 goes to step S92 again.

For example, in the event that determination is made at point-in-time t1 in Fig. 45 that the abdominal cavity pressure has reached the reset pressure (5 mmHg), the lumen setting pressure is changed by the control unit 45 (S95). The value of the lumen setting pressure to be changed is a value half (5 mmHg) of the lumen setting pressure before change (10 mmHg), as described above.

At the point-in-time t1 in Fig.45 when the abdominal cavity pressure reaches the reset pressure (5 mmHg), the control unit 45 opens the second relief valve 87b to lower the pressure within the lumen (S96), closes the second relief valve 87b following a predetermined amount of time elapsing (S97), determines whether or not the lumen pressure has reached the reset pressure (5 mmHg), based on the measurement results measured by the second pressure sensor 89 and second flow-rate sensor 91 (S97). That is to say, the second relief valve 87b repeats opening and closing operations until the lumen pressure achieves the reset pressure (5 mmHg). Accordingly, the carbon dioxide gas supplied within the abdominal cavity is externally discharged when the second relief valve 87b is on an opened state.

For example, in the event that determination is made at point-in-time t2 in Fig. 45 that the lumen pressure has reached the reset pressure (5 mmHg), the control unit 45 advances the flow to step S84, as shown in Fig. 42. Note that the value of the lumen setting pressure to be changed is a value half (5 mmHg) of the lumen setting pressure before change (10 mmHg), as described above.

The control unit 45 makes determination whether or not the terminal-expiratory carbon dioxide partial pressure in step S84 is greater than the threshold value. For example, let us say that in the event that the value of terminal-expiratory carbon dioxide partial pressure calculated by the monitoring apparatus 200 based on the terminal-expiratory carbon dioxide gas concentration is greater than the threshold value (5 mmHg) at the point-in-time t3 in Fig. 45. At this time, a calculation signal of the terminal-expiratory carbon dioxide partial pressure is inputted to the control unit 45 via the system controller 4. The control unit 45 then restores the setting value to the initial abdominal cavity setting pressure, i.e., the abdominal cavity setting pressure set by the surgeon (10 mmHg) (S101) as shown in Fig. 44.

In order to raise the pressure within the abdominal cavity, the control unit 45 closes the second solenoid valve 86 (S102), opens the first solenoid valve 85 (S103), and opens the first electropneumatic proportional valve 83 (S104). Thus, the carbon dioxide gas supplied from the gas tank 32 to within the gas supplying apparatus 31 is subjected to predetermined decompression by the sensor 82 and first electropneumatic proportional valve 83, and also predetermined flow-rate adjustment is performed, and passes through the first solenoid valve 85 and is supplied into the abdominal cavity via the first base 41a, abdominal cavity tube 45a, and third trocar 16. The control unit 45 closes the first solenoid valve 85 after a predetermined amount of time elapses (S105).

Next, the control unit 45 is supplied with measurement results measured by the supplied pressure sensor 81 and the first flow-rate sensor 90, and the control unit 45 makes determination regarding whether or not the abdominal cavity pressure has attached the setting value (10 mmHg) (S 106). Thus, the operations of step S103 to step S106 are repeated for the abdominal cavity, until the internal pressure is the setting pressure (10 mmHg). Upon determining that the abdominal cavity pressure has reached the setting pressure (10 mmHg), the control unit 45 advances the flow to step S107. Thus, supply of gas to the abdominal cavity is stopped, and the pressure within the abdominal cavity is maintained at a constant, as shown at point-in-time t4 in Fig. 45, for example.

At this point-in-time t4, upon supply of gas to the abdominal cavity being stopped, the control unit 45 inputs the initial lumen setting pressure (10 mmHg) from the system controller 4 (S107). Based on the inputted setting pressure, in order to raise the lumen pressure the control unit 45 opens the second solenoid valve 86 (S108), opens the second electropneumatic proportional valve 84 (S109), and closes the second solenoid valve following a predetermined amount of time elapsing (S110). Accordingly, the carbon dioxide gas supplied to within the gas supplying apparatus 31 from the gas tank 32 is decompressed to a predetermined pressure at the sensor 82 and second electropneumatic proportional valve 84, and is supplied into the lumen at a predetermined flow-rate through the second solenoid valve 86 and via the second base 41b, lumen tube 45b, and second endoscope 21.

Next, the control unit 45 is supplied with measurement results measured by the second pressure sensor 89 and the second flow-rate sensor 91. The control unit 45 then makes determination regarding whether or not the lumen pressure has reached the setting value (10 mmHg) (S111). Thus, the operations of step S108 to step S111 are repeated for the lumen, until the internal pressure is the setting pressure (10 mmHg). Upon determining that the lumen pressure has reached the setting pressure (10 mmHg), the control unit 45 ends the control. Thus, as shown in Fig. 45, supply of gas to the lumen is stopped at point-in-time t5, and the lumen is maintained at the setting pressure (10 mmHg).

As described above, the gas supplying apparatus 31 depressurizes or pressurizes the abdominal cavity and lumen based on the terminal-expiratory carbon dioxide partial pressure calculated by the monitoring apparatus 200, from the terminal-expiratory carbon dioxide gas concentration detected by the breathing sensor 301 of the respirator 300, from the breath discharged from the patient 10.

In the event that the terminal-expiratory carbon dioxide partial pressure of the patient 10 drops, the gas supplying apparatus 31 according to the present embodiment discharges the carbon dioxide gas in the abdominal cavity first as shown in Fig. 45, adjusts the abdominal cavity to the reset pressure (5 mmHg in this case), and then discharges the carbon dioxide gas in the lumen to the reset pressure (5 mmHg in this case). Thus, the abdominal cavity region does not suddenly narrow during surgery, so the surgeon can continue treatment of the affected portion. Accordingly, the surgeon can perform temporary treatment of the affected portion in a lowered state of terminal-expiratory carbon dioxide partial pressure of the patient 10, and the surgery can be temporarily halted.

Note that with the present embodiment, description has been made with reference to operations of depressurizing once for each of the initial setting pressures for the abdominal cavity and lumen, but the gas supplying system 4a performs depressurizing operations of the pressure within the abdominal cavity and lumen in stages, in accordance with the lowered state of the terminal-expiratory carbon dioxide partial pressure of the patient 10.

Consequently, according to the gas supplying system 4a of the present embodiment, excessive supply of pneumoperitoneum gas to be supplied into the abdominal cavity and lumen of the patient 10 can be suppressed.

Note that while description has been made with the present embodiment that the threshold values of terminal-expiratory carbon dioxide partial pressure for the control unit 45 to determine in order to depressurize or pressurize the abdominal cavity and lumen are the same, but an arrangement may be made wherein two threshold values, i.e., a first threshold value for depressurizing the abdominal cavity and lumen, and a second threshold value for pressurizing the abdominal cavity and lumen, are each determined by the control unit 45.

### (Eighth Embodiment)

The following is a description of an eighth embodiment of the present invention, with reference to the drawings. Note that the same reference numerals are provided to the same components as those in the above embodiments, description thereof will be omitted, and only different configurations, operations, and advantages will be described.

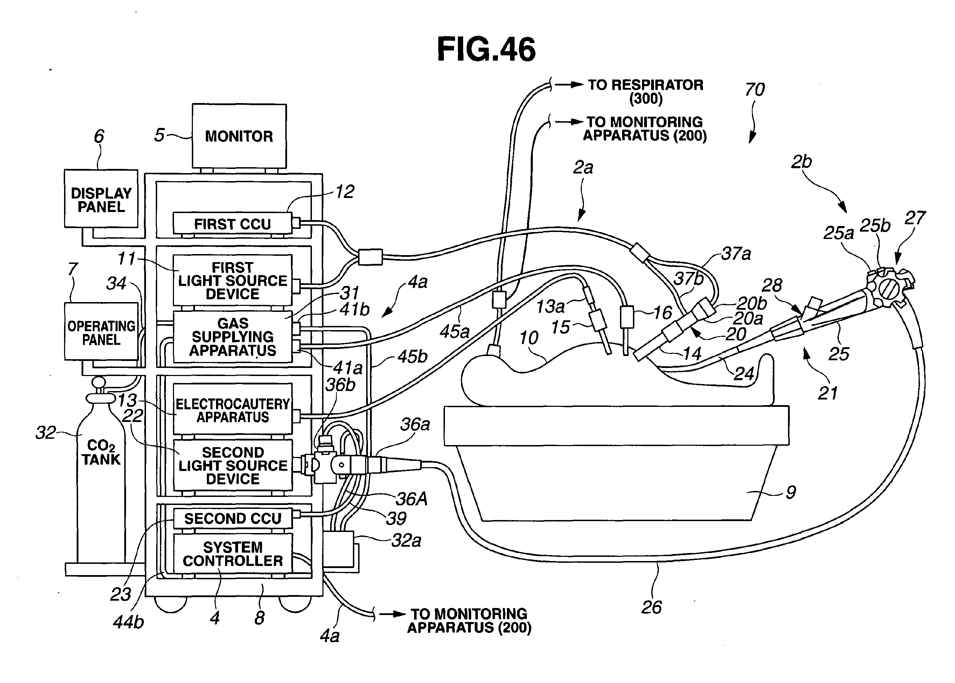

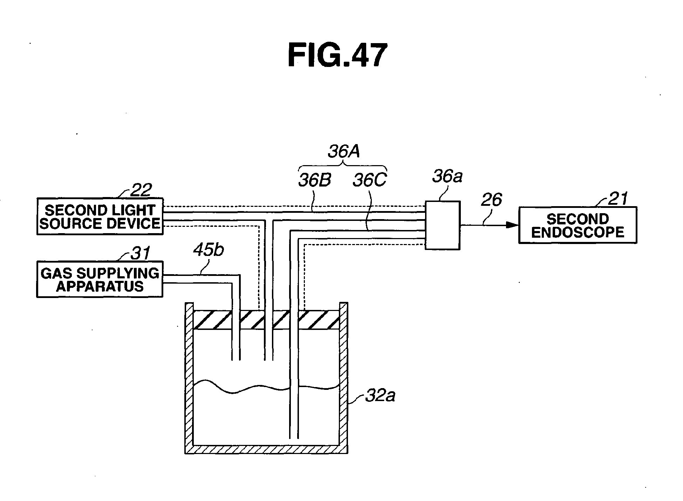

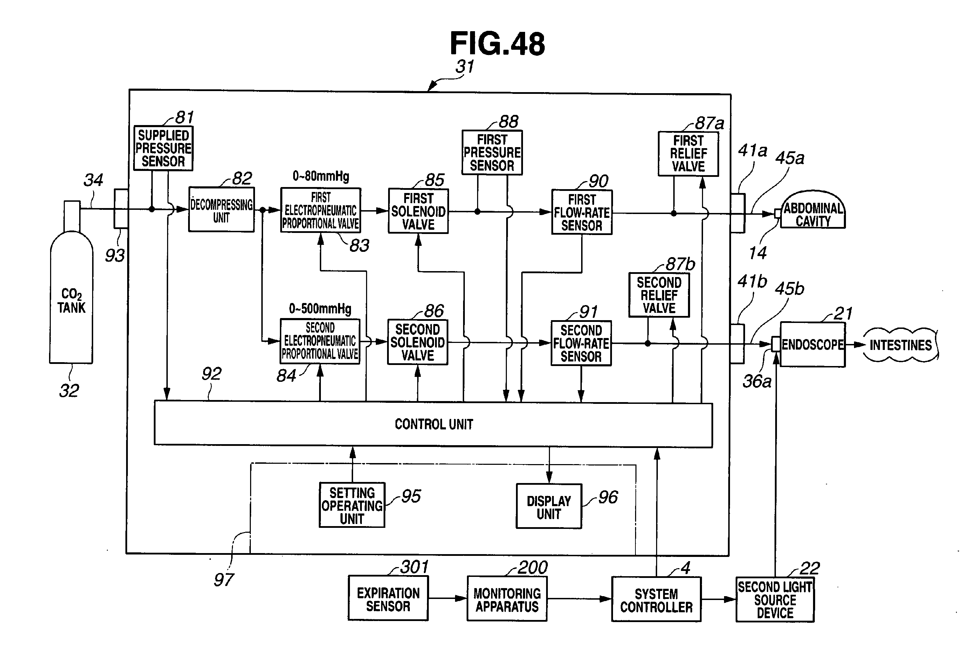

Fig. 46 is a diagram describing the configuration of a laparoscope surgery system including a gas supplying system according to the present embodiment, Fig. 47 is a diagram for describing a water tank, and Fig. 48 is a configuration diagram illustrating the internal configuration of a gas supplying apparatus.

As shown in Fig. 46, with the laparoscope surgery system 70 according to the present embodiment, the lumen tube 45b has one end connected to the gas supplying apparatus 31, and the other end connected to a water tank 32a disposed on the cart 8.

That is to say, carbon dioxide gas from the gas supplying apparatus 31 passes through the gas flow channel within the universal cord 26 via the lumen tube 45b, water tank 32a, gas/water supply tube 36A, and light source connector 36a, and is supplied into the lumen.

The light source connector 36a of the second endoscope 21 is also connected to the second light source 22. That is to say, air from an unshown compressor of the second light source 22 is supplied to the lumen from the light source connector 36a via the universal cord 26 of the second endoscope 21 and the insertion portion 24. Note that the compressor of this second light source device 22 is controlled by the system controller 4, and is controlled so as not to be driven generally.

More specifically, while carbon dioxide gas is being supplied into the lumen by the gas supplying apparatus 31, the system controller 4 stops driving of the compressor of the second light source device 22. That is to say, air from the compressor of the second light source device 22 is controlled so as not to be supplied to the lumen at the same time as supply of carbon dioxide gas by the gas supplying apparatus 31.

Accordingly, even in the event that the gas supplying/water supplying button 25a of the second endoscope 21 is operated by the user while carbon dioxide gas is being supplied into the lumen by the gas supplying apparatus 31, the compressor of the second light source device 22 is not driven.

Now, the water tank 32a shown in Fig. 47 will be described.

As shown in Fig. 47, a fluid such as distilled water is stored within the water tank 32a. The water tank 32a has the interior of the tank communicating with open portions at the tube ends of each of the gas/water supply tube 36A connected to the second light source device 22 and the lumen tube 45b connected to the gas supplying apparatus 31. Also, inserted into the gas/water supply tube 36A are a gas supply tube 36B and water supply tube 36C in a branched manner. The gas supply tube 36B has one end connected to the second light source device 22, and the other end branching to connect to the water tank 32a and the light source connector 36a. The water supply tube is immersed within the fluid in the water tank 32a at one end thereof, and the other end thereof is connected to the light source connector 36a.

Operating the gas supplying/water supplying button 25a of the second endoscope 21 selectively supplies carbon dioxide gas from the gas supplying apparatus 31, air from the second light source device 22, or distilled water within the water tank 32a, to be supplied into the lumen, from the distal end of the insertion portion 24 of the second endoscope 21. More specifically, the structure is such that, upon water supply being selected at the gas supplying/water supplying button 25a, the gas supply tube 36B is closed off within the operating unit 25 of the second endoscope 21.

The pressure within the water tank 32a increases due to the carbon dioxide gas from the gas supplying apparatus 31 or air from the second light source device 22 supplied into the water tank 32a, and the distilled water within the water tank 32a is forced along the water supply tube 36C and is supplied into the lumen from the distal end of the insertion portion 24 of the second endoscope 21 via the light source connector 36a and universal cord 26. Also, upon gas supply being selected at the gas supplying/water supplying button 25a, the water supply tube 36C is closed off within the operating unit 25 of the second endoscope 21.

The carbon dioxide gas from the gas supplying apparatus 31 or air from the second light source device 22 which has been supplied into the water tank 32a passes through the water tank 32a, flows into the gas supply tube 36B, and is supplied into lumen from the distal end of the insertion portion 24 of the second endoscope 21. Note that the gas supplying/water supplying button 25a is operated by the surgeon.

Also, as shown in Fig. 48, the system controller 4 is electrically connected to the control unit 45 of the gas supplying apparatus 31 and the monitoring apparatus 200, as with the seventh embodiment. Accordingly, the system controller 4 supplies to the control unit 45 the detection signals of terminal-expiratory carbon dioxide partial pressure calculated by the monitoring apparatus 200, from the terminal-expiratory carbon dioxide gas concentration detected by the breathing sensor 301 of the respirator 300, that has been input. Further, the system controller 4 is electrically connected with the second light source device 22.

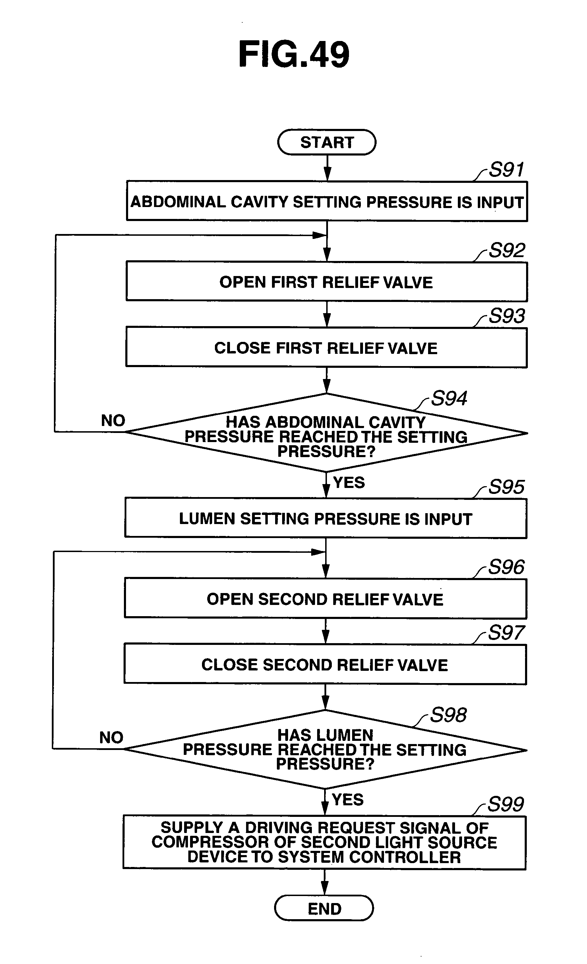

The operations of the gas supplying system 4a according to the present embodiment, configured as described above, will now be described with reference to the flowchart in Fig. 49.

Note that step S91 through S98 in Fig. 49 have been described in the flowchart in Fig. 43 according to the seventh embodiment, so description thereof will be omitted. With the present embodiment, after step S98, the control unit 45 supplies a compressor driving request signal for the second light source device 22, to the system controller 4 (S99).

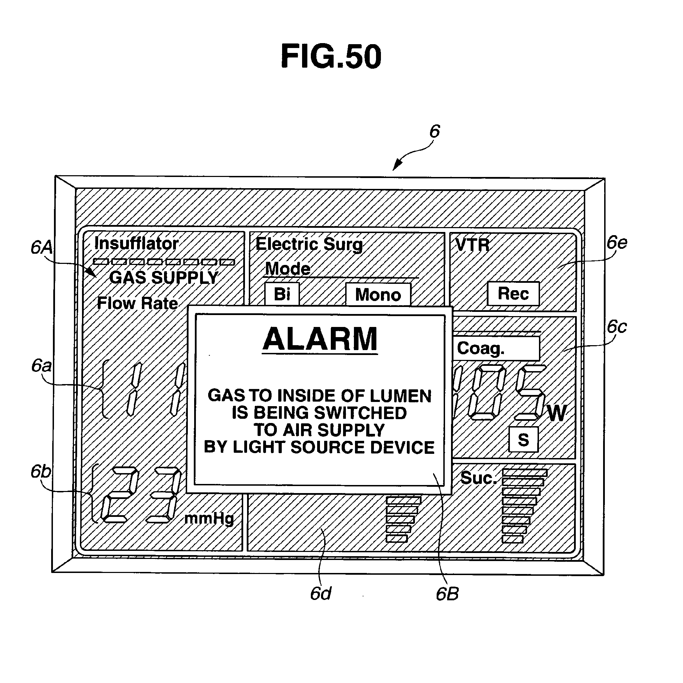

The system controller 4 which has received the control signal from the control unit 45 drives the compressor of the second light source device 22, and further displays an alarm screen 6B on the panel of the display panel 6, such as shown in Fig. 50. The alarm screen 6B displays an alarm message such as "Gas to inside of lumen is being switched to air supply by light source device ", as shown in Fig. 50.

Accordingly, the surgeon can supply air of a predetermined pressure from the compressor of the second light source device 22 into the lumen, based on predetermined operations of the gas supplying/water supplying button 25a of the second endoscope 21.

Consequently, in addition to the advantages of the above-described embodiments, the gas supplying system 4a according to the present embodiment can prevent interruption of surgery in the event that the terminal-expiratory carbon dioxide partial pressure of the patient 10 is at or below threshold value, by stopping supply of carbon dioxide gas to the lumen and supplying air.

Note that with the seventh and eighth embodiments, the gas supplying system 4a may depressurize or pressurize the inner pressure of the abdominal cavity or lumen, based on, for example, change of arterial oxygen saturation, blood flow, or the like, from the monitoring apparatus 200. Further, the gas supplying system 4a may depressurize or pressurize the inner pressure of the abdominal cavity or lumen based on change in combinations of such living body information.

According to the seventh and eighth embodiments described above, a gas supplying system 4a can be realized, wherein, in the event that there is an abnormality occurring in parameters notifying living body information of the patient, the burden on the doctor and nurses can be alleviated (the doctor and nurses can perform treatment smoothly).

Also, the present invention is not restricted to the above-described embodiments; rather, various modifications can be made without departing from the spirit and scope of the invention.

## Claims

1. A gas supplying apparatus, which is connected to a gas supplying channel of an endoscope, configured to supply gas to the body cavity of a patient via the gas supplying channel, comprising:
a switching unit configured to switch the gas supplying apparatus to a state of supplying the gas to the gas supplying channel, or a state of stopping supply of the gas;
a time measuring unit configured to measure the gas supply time of the gas; and
a control unit, which is electrically connected to the time measuring unit, configured to control the switching unit;
wherein the control unit controls the switching unit to supply the gas to the gas supplying channel, and then controls the switching unit so as to switch from a state of supplying the gas to the gas supplying channel to a state of stopping supply of the gas in the event that the gas supply time by the time measuring unit is inputted, and the gas supply time measured at the time measuring unit reaches the setting time set beforehand.

2. The gas supplying apparatus according to Claim 1, further comprising:
a flow-rate measuring unit configured to measure a flow rate of the gas supplied in the gas supplying channel; and
a comparison computing unit configured to calculate the start time of measuring of the gas supply time by the time measuring unit based on the measurement results of the flow-rate measuring unit;
wherein the control unit controls the switching unit to supply the gas to the gas supplying channel, and then controls the switching unit so as to switch from a state of supplying the gas to a state of stopping supply of gas in the event that the gas supply time by the time measuring unit is inputted at the start time of measuring based on the calculation result from the comparison computing unit, and the gas supply time measured by the time measuring unit reaches the setting time set beforehand.

3. The gas supplying apparatus according to Claim 2, wherein the comparison computing unit compares the measurement results by the flow-rate measuring unit and a threshold set beforehand, and calculates the start time of measuring of the gas supply time by the time measuring unit based on the comparison results.

4. The gas supplying apparatus according to Claim 2, wherein the comparison computing unit calculates the amount of change of flow rates in increments of time determined beforehand with measurement results by the flow-rate measuring unit, and also compares the calculation result and the threshold set beforehand, and calculates the start time of measuring of the gas supply time by the time measuring unit based on the comparison result.

5. The gas supplying apparatus according to any one of Claims 1 through 4, further comprising:
a timing setting unit configured to set the setting time arbitrarily.

6. The gas supplying apparatus according to any one of Claims 1 through 4, further comprising:
a notifying unit configured to notify that the gas supplying unit is switched from a state of supplying the gas to a state of stopping supply of the gas;
wherein the control unit controls the notifying unit to notify that the gas supply time measured by the time measuring unit has reached the setting time set beforehand, when the gas supply time measured by the time measuring unit reaches the setting time set beforehand.

7. An endoscope system comprising:
an endoscope having a gas supplying channel capable of supplying gas to the body cavity of a patient; and
a gas supplying apparatus including
a switching unit configured to switch the gas supplying apparatus to a state of supplying the gas to the gas supplying channel, or a state of stopping supply of the gas,
a time measuring unit configured to measure the gas supply time of the gas, and
a control unit, which is electrically connected to the time measuring unit, configured to control the switching unit;
wherein the control unit controls the switching unit to supply the gas to the gas supplying channel, and then controls the switching unit so as to switch from a state of supplying the gas to the gas supplying channel to a state of stopping supply of the gas in the event that the gas supply time by the time measuring unit is inputted, and the gas supply time measured at the time measuring unit reaches the setting time set beforehand.

8. The endoscope system according to Claim 7, wherein the gas supplying apparatus further comprises:
a flow-rate measuring unit configured to measure a flow rate of the gas supplied in the gas supplying channel; and
a comparison computing unit configured to calculate the start time of measuring of the gas supply time by the time measuring unit based on the measurement results of the flow-rate measuring unit;
and wherein the control unit controls the switching unit to supply the gas to the gas supplying channel, and then controls the switching unit so as to switch from a state of supplying the gas to a state of stopping supply of gas in the event that the gas supply time by the time measuring unit is inputted at the start time of measuring based on the calculation result from the comparison computing unit, and the gas supply time measured by the time measuring unit reaches the setting time set beforehand.

9. A control method of a gas supplying apparatus, comprising:
a gas supplying step of supplying gas via a gas supplying channel of an endoscope;
a measuring step of measuring a flow rate of the gas supplied in the gas supplying channel;
a detecting step of detecting whether or not a gas supply operation by the endoscope is being performed based on the measurement result in the measuring step; and
a control step of adjusting the flow rate of supply of the gas by controlling the flow rate of supply of the gas so as to be decreased in the gas supplying step in the event that a gas supply operation by the endoscope is not being performed is detected in the detecting step.

10. The control method of a gas supplying apparatus according to Claim 9, wherein in the control step, the flow rate of supply of the gas is decreased by controlling a state of not supplying the gas in the gas supplying step and a state of supplying the gas in the gas supplying step to be switched in increments of setting time set beforehand in the event of detecting in the detecting step that a gas supply operation by the endoscope is not being performed.

11. The control method of a gas supplying apparatus according to Claim 9, further comprising:
a suctioning step of aspirating the gas from the body cavity via a gas supplying channel of the endoscope;
wherein in the control step, the flow rate of supply of the gas is decreased by performing control so as to stop supply of the gas in the gas supplying step, and simultaneously aspirate the gas in the suctioning step in the event of detecting that a gas supply operation by the endoscope is not being performed in the detecting step.

12. A gas supplying apparatus, comprising:
a gas supplying unit configured to supply gas via a gas supplying channel of an endoscope;
a measuring unit configured to measure the flow rate of the gas supplied in the gas supplying channel;
a detecting unit configured to detect whether or not a gas supply operation by the endoscope is being performed based on the measurement result by the measuring unit; and
a control unit configured to regulate the flow rate of supply of the gas by controlling the gas supplying unit to decrease the flow rate of supply of the gas in the event of the detecting unit detecting that a gas supply operation by the endoscope is not being performed.

13. The gas supplying apparatus according to Claim 12, wherein the control unit decreases the flow rate of supply of the gas by controlling the gas supply unit to switch a state of not supplying the gas and a state of supplying the gas in increments of setting time set beforehand in the event of the detecting unit detecting that a gas supply operation by the endoscope is not being performed.

14. The gas supplying apparatus according to Claim 12, further comprising:
a suctioning unit configured to aspirate the gas from the body cavity via a gas supplying channel of the endoscope;
wherein the control unit decreases the flow rate of supply of the gas by controlling the gas supplying unit to stop supply of the gas, and simultaneously controlling the suctioning unit to aspirate the gas in the event of the detecting unit detecting that a gas supply operation by the endoscope is not being performed.

15. An endoscope system comprising:
an endoscope having a gas supplying channel; and
a gas supplying apparatus including
a gas supplying unit configured to supply gas via the gas supplying channel,
a measuring unit configured to measure the flow rate of the gas supplied in the gas supplying channel,
a detecting unit configured to detect whether or not a gas supply operation by the endoscope is being performed based on the measurement result by the measuring unit, and
a control unit configured to regulate the flow rate of supply of the gas by controlling the gas supplying unit to decrease the flow rate of supply of the gas in the event of the detecting unit detecting that a gas supply operation by the endoscope is not being performed.

16. The endoscope system according to Claim 15, wherein the control unit decreases the flow rate of supply of the gas by controlling the gas supply unit to switch a state of not supplying the gas and a state of supplying the gas in increments of setting time set beforehand in the event of the detecting unit detecting that a gas supply operation by the endoscope is not being performed.

17. The endoscope system according to Claim 15, wherein the gas supplying apparatus further includes
a suctioning unit configured to aspirate the gas from the body cavity via the gas supplying channel of the endoscope;
wherein the control unit decreases the flow rate of supply of the gas by controlling the gas supplying unit to stop supply of the gas, and simultaneously controlling the suctioning unit to aspirate the gas in the event of the detecting unit detecting that a gas supply operation by the endoscope is not being performed.

18. A gas supplying system, which is connected to a gas supplying channel of an endoscope, configured to supply gas to the abdominal cavity and lumen of a patient via the gas supplying channel, comprising:
a gas supplying unit configured to supply predetermined gas to an abdominal cavity and lumen;
a pressure regulating unit configured to regulate the internal pressure of each of the abdominal cavity and the lumen; and
a control unit electrically connected to an external apparatus configured to output living body information;
wherein the control unit regulates the internal pressure of each of the abdominal cavity and the lumen based on variations of the living body information inputted from the external apparatus.

19. The gas supplying system according to Claim 18, wherein the control unit drives and controls the gas supplying unit to apply pressure on the internal pressure of each of the abdominal cavity and the lumen up to each initial setting value when a detection value of the living body information inputted from the external apparatus reaches a predetermined second threshold or more.

20. The gas supplying system according to Claim 18, wherein the gas supplying unit includes
a first gas supplying unit configured to supply first gas to an abdominal cavity and lumen, and
a second gas supplying unit configured to supply second gas which is different from the first gas to the lumen;
and wherein the pressure regulating unit includes
a first pressure regulating unit configured to regulate the internal pressure of the abdominal cavity, and
a second pressure regulating unit configured to regulate the internal pressure of the lumen;
and wherein the control unit drives and controls the first pressure regulating unit to decrease the internal pressure of the abdominal cavity when a detection value of the living body information inputted from the external apparatus reaches a predetermined threshold or less, and subsequently drives and controls the second pressure regulating unit to decrease the internal pressure of the lumen, and subsequently drives and controls the second gas supplying unit to supply gas to the lumen.
